# EUROPÄISCHE PATENTSCHRIFT

(11) **EP 1 073 642 B1**
(45) Veröffentlichungstag und Bekanntmachung des Hinweises auf die Patenterteilung: **23.10.2002**
(21) Anmeldenummer: 99920604.8
(22) Anmeldetag: 07.04.1999
(51) Int. Cl.: C07D 239/28, C07D 401/04, C07D 409/14, A61K 31/505, A61P 31/20

(54) **VERWENDUNG VON DIHYDROPYRIMIDINEN ALS ARZNEIMITTEL UND NEUE STOFFE ZUR BEHANDLUNG VON HEPATITIS B**
THE USE OF DIHYDROPYRIMIDINES AS MEDICAMENTS, AND NOVEL SUBSTANCES FOR THE TREATMENT OF HEPATITIS B
UTILISATION DE DIHYDROPYRIMIDINES COMME MEDICAMENTS, ET NOUVELLES SUBSTANCES POUR LE TRAITEMENT DE L'HEPATITE B

(30) Priorität: 18.04.1998 DE 19817265
(43) Veröffentlichungstag der Anmeldung: 07.02.2001
(73) Patentinhaber: BAYER AG, 51368 Leverkusen (DE)
(72) Erfinder: STOLTEFUSS, Jürgen, D-42781 Haan (DE); GOLDMANN, Siegfried, D-42327 Wuppertal (DE); PAESSENS, Arnold, D-42781 Haan (DE); GRAEF, Erwin, D-42553 Velbert (DE); LOTTMANN, Stefan, D-42115 Wuppertal (DE)
(86) Internationale Anmeldenummer: EP9902346
(87) Internationale Veröffentlichungsnummer: WO99054312

(56) Entgegenhaltungen:
- EP-A- 0 103 796
- EP-A- 0 169 712
- WO-A-99/01438

## Beschreibung

Die vorliegende Erfindung betrifft die Verwendung von Dihydropyrimidinen zur Herstellung von Arzneimitteln zur Behandlung und Prophylaxe von Hepatitis B, neue Stoffe und verschiedene Verfahren zu ihrer Herstellung.

Aus der Publikation EP 103 796 A2 sind bereits Dihydropyrimidine mit einer kreislaufbeeinflussenden Wirkung bekannt.

Es wurde nun überraschenderweise gefunden, daß Dihydropyrimidine der allgemeinen Formel (I) 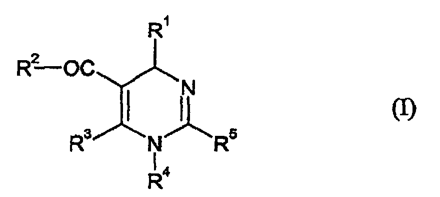 bzw. deren isomeren Form (Ia) 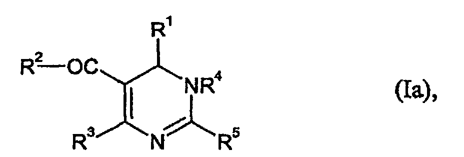 in welcher
- R¹: für Phenyl, Furyl, Thienyl, Triazolyl, Pyridyl, Cycloalkyl mit 3 bis 6 Kohlenstoffatomen oder fiir Reste der Formeln 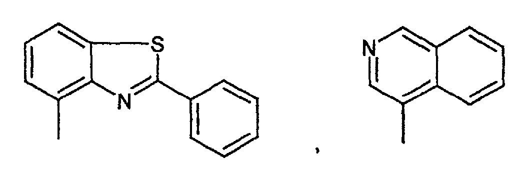 oder 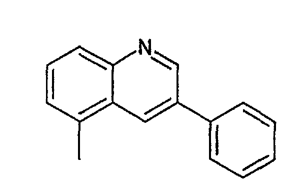 steht, wobei die oben aufgeführten Ringsysteme gegebenenfalls einfach oder mehrfach, gleich oder verschieden durch Substituenten, ausgewählt aus der Gruppe Halogen, Trifluormethyl, Nitro, Cyano, Trifluormethoxy, Carboxyl, Hydroxyl, (C₁-C₆)-Alkoxy, (C₁-C₆)-Alkoxycarbonyl und (C₁-C₆)-Alkyl substituiert sind, das seinerseits durch Aryl mit 6 bis 10 Kohlenstoffatomen oder Halogen substituiert sein kann,
und/oder die aufgeführten Ringsysteme gegebenenfalls durch Gruppen der Formel -S-R⁶, NR⁷R⁸, CO-NR⁹R¹⁰, SO₂-CF₃ und -A-CH₂-R¹¹ substituiert sind,
worin
R⁶ Phenyl bedeutet, das gegebenenfalls durch Halogen substituiert ist,
R⁷, R⁸, R⁹ und R¹⁰ gleich oder verschieden sind und Wasserstoff, Phenyl, Hydroxy-substituiertes Phenyl, Hydroxy, (C₁-C₆)-Acyl oder (C₁-C₆)-Alkyl bedeuten, das seinerseits durch Hydroxy, (C₁-C₆)-Alkoxycarbonyl, Phenyl oder Hydroxy substituiertes Phenyl substituiert sein kann,
A einen Rest O, S, SO oder SO₂ bedeutet,
R¹¹ Phenyl bedeutet, das gegebenenfalls ein- bis mehrfach, gleich oder verschieden durch Substituenten, ausgewählt aus der Gruppe Halogen, Nitro, Trifluormethyl, (C₁-C₆)-Alkyl und (C₁-C₆)-Alkoxy substituiert ist,
- R²: für einen Rest der Formel -XR¹² oder -NR¹³R¹⁴ steht,
worin
X eine Bindung oder Sauerstoff bedeutet,
R¹² Wasserstoff, geradkettiges oder verzweigtes (C₁-C₆)-Alkoxycarbonyl oder einen geradkettigen, verzweigten oder cyclischen, gesättigten oder ungesättigten (C₁-C₈)-Kohlenwasserstoffrest bedeutet, der gegebenenfalls eine oder zwei gleiche oder verschiedene Heterokettenglieder aus der Gruppe O, CO, NH, -NH-(C₁-C₄)-Alkyl, -N-((C₁-C₄)-Alkyl)₂, S oder SO₂ enthält und der gegebenenfalls substituiert ist durch Halogen, Nitro, Cyano, Hydroxy, Aryl mit 6 bis 10 Kohlenstoffatomen oder Aralkyl mit 6 bis 10 Kohlenstoffatomen, Heteroaryl oder einer Gruppe der Formel -NR¹⁵R¹⁶,
worin
R¹⁵ und R¹⁶ gleich oder verschieden sind und Wasserstoff, Benzyl oder (C₁-C₆)-Alkyl bedeuten,
R¹³ und R¹⁴ gleich oder verschieden sind und Wasserstoff, (C₁-C₆)-Alkyl oder Cycloalkyl mit 3 bis 6 Kohlenstoffatomen bedeuten,
- R³: für Wasserstoff, Amino oder
für einen Rest der Formel 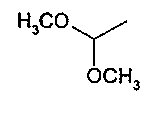 steht, oder für Formyl, Cyano, hydroxy-substituiertes (C₁-C₆)-Alkylthio, Trifluormethyl oder Pyridyl steht, oder
für einen geradkettigen, verzweigten oder cyclischen, gesättigten oder ungesättigten Kohlenwasserstoffrest mit bis zu 8 Kohlenstoffatomen steht, der gegebenenfalls ein- oder mehrfach, gleich oder verschieden durch Aryloxy mit 6 bis 10 Kohlenstoffatomen, Azido, Halogen, Cyano, Hydroxy, Carboxyl, (C₁-C₆)-Alkoxycarbonyl, einen 5- bis 7-gliedrigen heterocyclischen Ring, (C₁-C₆)-Alkylthio oder (C₁-C₆)-Alkoxy substituiert ist, das seinerseits durch Azido oder Amino substituiert sein kann,
und/oder durch Triazolyl substituiert ist, das seinerseits bis zu 3-fach durch (C₁-C₆)-Alkoxycarbonyl substituiert sein kann,
und/oder durch Gruppen der Formeln -OSO₂-CH₃ oder (CO)ₐ-NR¹⁷R¹⁸ substiutiert sein kann,
worin
a eine Zahl 0 oder 1 bedeutet,
R¹⁷ und R¹⁸ gleich oder verschieden sind und Wasserstoff oder Aryl, Aralkyl mit 6 bis 10 Kohlenstoffatomen bedeuten,
oder (C₁-C₆)-Alkyl bedeuten, das gegebenenfalls durch (C₁-C₆)-Alkoxycarbonyl, Amino, Hydroxyl, Phenyl oder Benzyl substituiert ist, wobei Phenyl oder Benzyl gegebenenfalls ein- oder mehrfach, gleich oder verschieden durch Hydroxy, Carboxyl, (C₁-C₆)-Alkyl oder (C₁-C₆)-Alkoxy substituiert sind,
oder (C₁-C₆)-Alkyl gegebenenfalls durch Gruppen der Formeln NH-CO-CH₃ oder NH-CO-CF₃ substituiert ist, oder
R¹⁷ und R¹⁸ gemeinsam mit dem Stickstoffatom einen Morpholin-, Piperidinyl- oder Pyrrolidinylring bilden, oder
- R³: für Phenyl steht, das gegebenenfalls durch Methoxy substituiert ist,
oder
- R² und R³: oder gemeinsam einen Rest der Formel 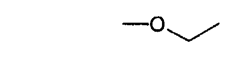 bilden,
- R⁴: für Wasserstoff, (C₁-C₄)-Alkyl, (C₂-C₄)-Alkenyl, Benzoyl oder für Acyl mit 2 bis 6 Kohlenstoffatomen steht,
- R⁴: für Wasserstoff, Methyl, Benzoyl oder für (C₂-C₆)-Acyl steht,
- R⁵: für Pyridyl, Pyrimidyl oder Pyrazinyl steht,
sowie deren Salze,
überraschenderweise eine antivirale Wirkung gegen Hepatitis B (HBV) besitzen und somit geeignet sind zur Prophylaxe und Bekämpfung von virusinduzierten Erkrankungen, insbesondere von akut und chronisch persistenten Virusinfektionen des HBV.

Cycloalkyl mit 3 bis 6 Kohlenstoffatomen steht im Rahmen der Erfindung für Cyclopropyl, Cyclopentyl, Cyclobutyl oder Cyclohexyl. Bevorzugt seien genannt: Cyclopentyl oder Cyclohexyl.

Aryl steht im allgemeinen für einen aromatischen Rest mit 6 bis 10 Kohlenstoffatomen. Bevorzugte Arylreste sind Phenyl und Naphthyl.

(C₁-C₆)-Acyl steht im Rahmen der Erfindung für einen geradkettigen oder verzweigten Acylrest mit 1 bis 6 Kohlenstoffatomen. Bevorzugt ist ein geradkettiger oder verzweigter Acylrest mit 1 bis 4 Kohlenstoffatomen. Besonders bevorzugte Acylreste sind Acetyl und Propionyl.

(C₁-C₆)-Alkyl steht im Rahmen der Erfindung für einen geradkettigen oder verzweigten Alkylrest mit 1 bis 6 Kohlenstoffatomen. Beispielsweise seien genannt: Methyl, Ethyl, Propyl, Isopropyl, tert.Butyl, n-Pentyl und n-Hexyl. Bevorzugt ist ein geradkettiger oder verzweigter Alkylrest mit 1 bis 4 Kohlenstoffatomen.

(C₂-C₆)-Alkenyl steht im Rahmen der Erfindung für einen geradkettigen oder verzweigten Alkenylrest mit 2 bis 6 Kohlenstoffatomen. Beispielsweise seien genannt: Ethenyl, Propenyl, Isopropenyl, tert.Butenyl, n-Pentenyl und n-Hexenyl. Bevorzugt ist ein geradkettiger oder verzweigter Niedrigalkenylrest mit 3 bis 5 Kohlenstoffatomen.

(C₁-C₆)-Alkoxy steht im Rahmen der Erfindung für einen geradkettigen oder verzweigten Alkoxyrest mit 1 bis 6 Kohlenstoffatomen. Beispielsweise seien genannt: Methoxy, Ethoxy, Propoxy, Isopropoxy, tert.Butoxy, n-Pentoxy und n-Hexoxy. Bevorzugt ist ein geradkettiger oder verzweigter Alkoxyrest mit 1 bis 4 Kohlenstoffatomen.

(C₁-C₆)-Alkoxycarbonyl steht im Rahmen der Erfindung für einen geradkettigen oder verzweigten Alkoxycarbonylrest mit 1 bis 6 Kohlenstoffatomen. Beispielsweise seien genannt: Methoxycarbonyl, Ethoxycarbonyl, Propoxycarbonyl, Isopropoxycarbonyl, tert.Butoxycarbonyl, n-Pentoxycarbonyl und n-Hexoxycarbonyl. Bevorzugt ist ein geradkettiger oder verzweigter Alkoxycarbonylrest mit 1 bis 4 Kohlenstoffatomen.

Bevorzugt verwendet werden Verbindungen der allgemeinen Formeln (I) bzw. (Ia) in welcher
- R¹: für Phenyl, Furyl, Thienyl, Triazolyl, Pyridyl, Cyclopentyl oder Cyclohexyl oder für Reste der Formeln 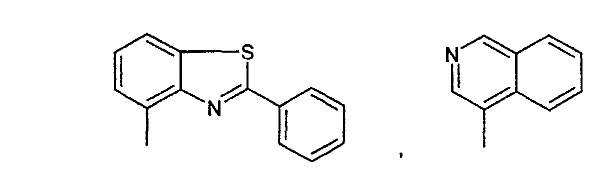 oder 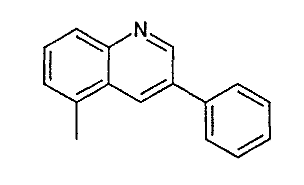 steht,
wobei die oben aufgeführten Ringsysteme gegebenenfalls ein- oder 2-fach gleich oder verschieden durch Substituenten, ausgewählt aus der Gruppe Halogen, Trifluormethyl, Nitro, SO₂-CF₃, Methyl, Cyano, Trifluormethoxy, Hydroxy, Carboxyl, Methoxycarbonyl oder Resten der Formel -CO-NH-CH₂-C(CH₃)₃, -CO-NH(CH₂)₂OH, -CO-NH-CH₂-C₆H₅, -CO-NH-C₆H₅, -CO-NH-(pOH)-C₆H₄, -O-CH₂-C₆H₅ oder -S-pCl-C₆H₄ substituiert sind,
- R²: für einen Rest der Formel -XR¹² oder -NR¹³R¹⁴ steht,
worin
X eine Bindung oder ein Sauerstoffatom bedeutet,
R¹² Wasserstoff, (C₁-C₄)-Alkenyl, (C₁-C₄)-Alkoxycarbonyl oder (C₁-C₄)-Alkyl bedeutet, das gegebenenfalls durch Pyridyl, Cyano, Phenoxy, Benzyl oder durch einen Reste der Formel -NR¹⁵R¹⁶ substituiert sind,
worin
R¹⁵ und R¹⁶ gleich oder verschieden sind und Wasserstoff, Benzyl oder (C₁-C₄)-Alkyl bedeuten,
R¹³ und R¹⁴ gleich oder verschieden sind und Wasserstoff, (C₁-C₄)-Alkyl oder Cyclopropyl bedeuten,
- R³: für Wasserstoff, Amino oder einen Rest der Formel 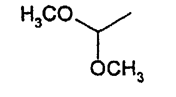 steht,
oder
für Formyl, Cyano, hydroxy-substituiertes (C₁-C₄)-Alkylthio, Trifluormethyl, Cyclopropyl oder Pyridyl steht, oder
oder für (C₁-C₄)-Alkyl steht, das gegebenenfalls durch Halogen, (C₁-C₄)-Alkoxycarbonyl, Hydroxy oder durch Triazolyl substituiert ist, das seinerseits bis zu 3-fach durch (C₁-C₄)-Alkoxycarbonyl substituiert sein kann,
und/oder Alkyl gegebenenfalls durch Gruppen der Formeln -OSO₂-CH₃ oder (CO)ₐ-NR¹⁷R¹⁸ substituiert ist,
worin
a eine Zahl 0 oder 1 bedeutet,
R¹⁷ und R¹⁸ gleich oder verschieden sind und Wasserstoff, Phenyl oder Benzyl bedeuten, oder
C₁-C₄-Alkyl bedeuten, das gegebenenfalls durch (C₁-C₄)-Alkoxycarbonyl, Amino, Hydroxyl, Phenyl oder Benzyl substituiert ist, wobei Phenyl oder Benzyl gegebenenfalls ein- oder mehrfach gleich oder verschieden durch Hydroxy, Carboxy, (C₁-C₄)-Alkyl oder (C₁-C₄)-Alkoxy substituiert sind,
und/oder (C₁-C₄)-Alkyl gegebenenfalls durch Reste der Formeln -NH-CO-CH₃ oder -NH-CO-CF₃ substituiert ist, oder
R¹⁷ und R¹⁸ gemeinsam mit dem Stickstoffatom einen Morpholin-, Piperidinyl- oder Pyrrolidinylring bilden, oder
- R³: für Phenyl steht, das gegebenenfalls durch Methoxy substiuiert ist,
oder
- R² und R³: gemeinsam einen Rest der Formel 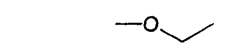 bilden,
- R⁴: für Wasserstoff, Methyl, Vinyl oder Acetyl steht,
- R⁵: für Pyridyl, Pyrimidyl oder Pyrazinyl steht,
und deren Salze
bei der Bekämpfung und Prophylaxe von Hepatitis B.

Besonders bevorzugt verwendet werden Verbindungen der allgemeinen Formeln (I) und (Ia),
in welcher
- R¹: für Phenyl, Furyl, Thienyl, Triazolyl, Pyridyl, Cyclopentyl, Cyclohexyl oder für Reste der Formeln 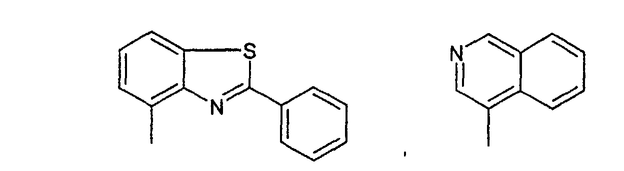 oder 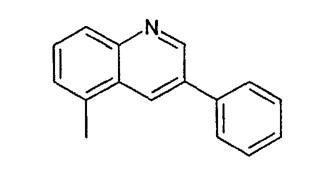 steht,
wobei die oben aufgeführten Ringsysteme gegebenenfalls bis zu 2-fach, gleich oder verschieden durch Substituenten, ausgewählt aus der Gruppe Fluor, Chlor, Brom, Jod, Hydroxy, Trifluormethyl, Nitro, SO₂-CF₃, Methyl, Cyano, Trifluormethoxy, Carboxyl, Methoxycarbonyl oder Resten der Formeln -CO-NH-CH₂-C(CH₃)₃, -CO-NH(CH₂)₂OH, -CO-NH-CH₂-C₆H₅, -CO-NH-C₆H₅, -CO-NH-(pOH)-C₆H₄, -O-CH₂-C₆H₅ oder -S-pCl-C₆H₄ substituiert sind,
- R²: für einen Rest der Formel -XR¹² oder -NR¹³R¹⁴ steht,
worin
X eine Bindung oder ein Sauerstoffatom bedeutet,
R¹² Wasserstoff, (C₁-C₃)-Alkenyl, (C₁-C₄)-Alkoxycarbonyl oder (C₁-C₄)-Alkyl bedeutet, das gegebenenfalls durch Pyridyl, Cyano, Phenoxy, Benzyl oder durch einen Rest der Formel -NR¹⁵R¹⁶ substituiert sind,
worin
R¹⁵ und R¹⁶ gleich oder verschieden sind und Wasserstoff oder Methyl bedeuten,
R¹³ und R¹⁴ gleich oder verschieden sind und Wasserstoff, (C₁-C₃)-Alkyl oder Cyclopropyl bedeuten,
- R³: für Wasserstoff, Amino oder für einen Rest der Formel 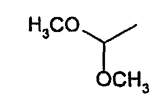 steht,
oder
für Formyl, Cyano, hydroxy-substituiertes (C₁-C₄)-Alkylthio, Trifluormethyl, Cyclopropyl oder Pyridyl steht, oder
oder für (C₁-C₄)-Alkyl steht, das gegebenenfalls durch Fluor, Chlor, (C₁-C₃)-Alkoxycarbonyl, Hydroxy oder durch Triazolyl substituiert ist, das seinerseits bis zu 3-fach durch (C₁-C₃)-Alkoxycarbonyl substituiert sein kann,
und/oder Alkyl gegebenenfalls durch Gruppen der Formeln -OSO₂-CH₃ oder (CO)ₐ-NR¹⁷R¹⁸ substituiert ist,
worin
a eine Zahl 0 oder 1 bedeutet,
R¹⁷ und R¹⁸ gleich oder verschieden sind und Wasserstoff, Phenyl oder Benzyl bedeuten, oder
(C₁-C₃)-Alkyl bedeuten, das gegebenenfalls durch (C₁-C₃)-Alkoxycarbonyl, Amino, Hydroxyl, Phenyl oder Benzyl substituiert ist, wobei Phenyl oder Benzyl gegebenenfalls ein- oder zweifach, gleich oder verschieden durch Hydroxy, Carboxy, (C₁-C₃)-Alkyl oder (C₁-C₃)-Alkoxy substituiert sind,
und/oder (C₁-C₄)-Alkyl gegebenenfalls durch Reste der Formeln -NH-CO-CH₃ oder -NH-CO-CF₃ substituiert ist, oder
R¹⁷ und R¹⁸ gemeinsam mit dem Stickstoffatom einen Morpholin-, Piperidinyl- oder Pyrrolidinylring bilden, oder
- R³: für Phenyl steht, das gegebenenfalls durch Methoxy substiuiert ist,
oder
- R² und R³: gemeinsam einen Rest der Formel 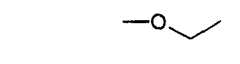 bilden,
- R⁴: für Wasserstoff, Methyl, Vinyl oder Acetyl steht,
- R⁵: für Pyridyl, Pyrimidyl oder Pyrazinyl steht,
und deren Salze
bei der Bekämpfung und Prophylaxe von Hepatitis B.

Ganz besonders bevorzugt verwendet werden erfindungsgemäße Verbindungen der allgemeinen Formeln (I) und (Ia)
in welcher
- R¹: für Phenyl oder Triazolyl steht, die gegebenenfalls bis zu 2-fach gleich oder verschieden durch Fluor, Chlor, Brom der Jod substituiert sind,
- R²: für geradkettiges oder verzweigtes Alkoxycarbonyl mit bis zu 4 Kohlenstoffatomen steht,
- R³: für Methyl, Ethyl oder Cyclopropyl steht,
oder
- R² und R³: gemeinsam einen Rest der Formel 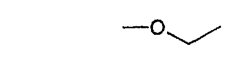 bilden,
- R⁴: für Wasserstoff, Vinyl oder Acetyl steht,
und
- R⁵: für Pyridyl steht,
bei der Prophylaxe und Bekämpfung von Hepatitis B.

Die vorliegenden Erfindung betrifft außerdem neue Stoffe, die in der Tabelle A aufgeführt sind:

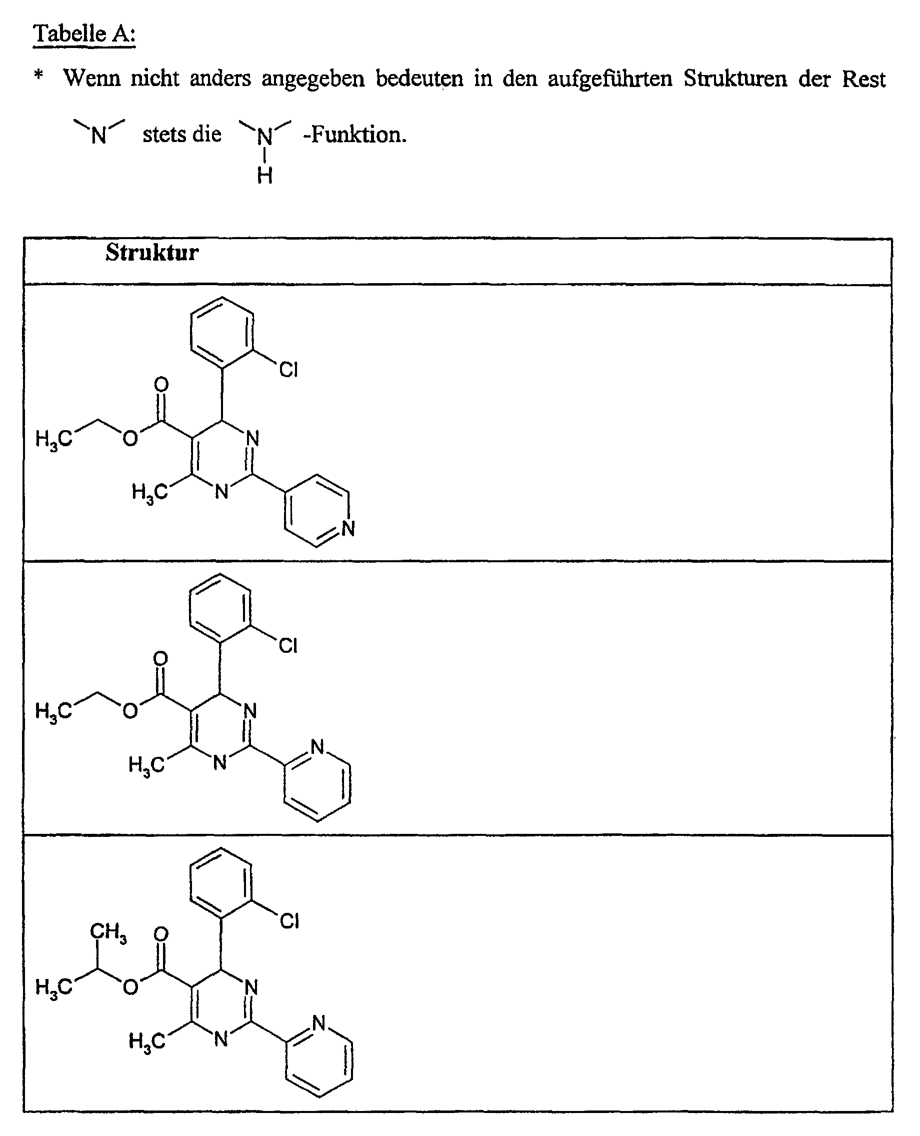

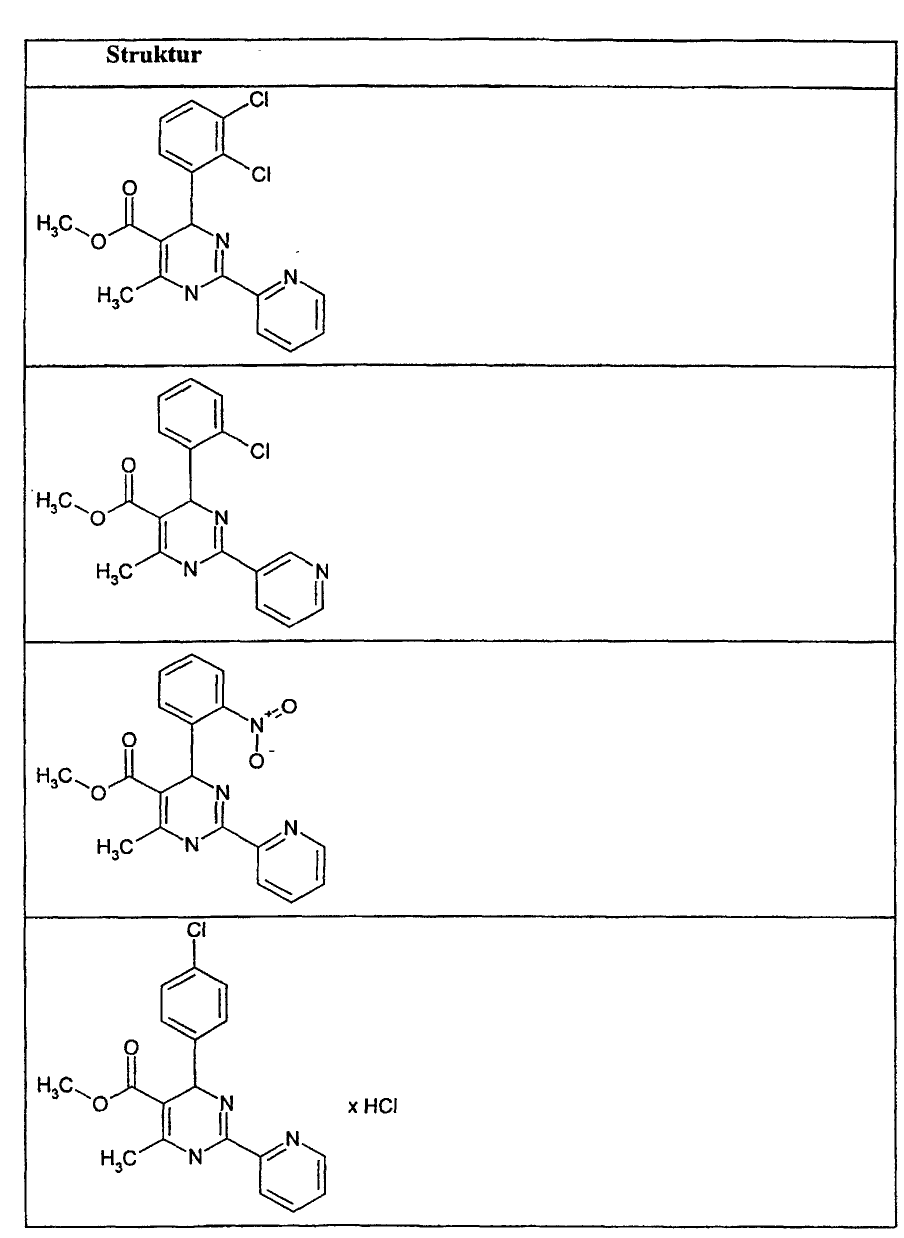

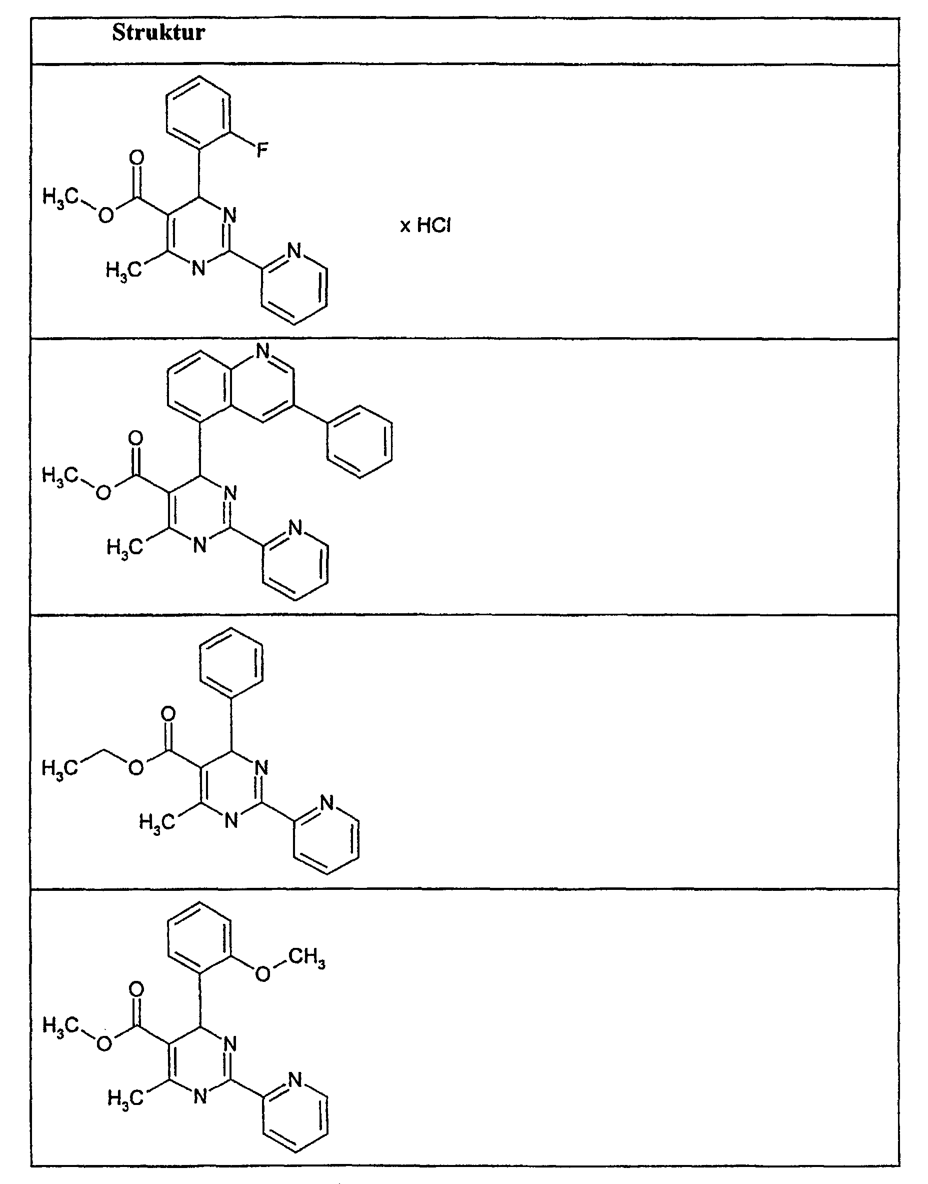

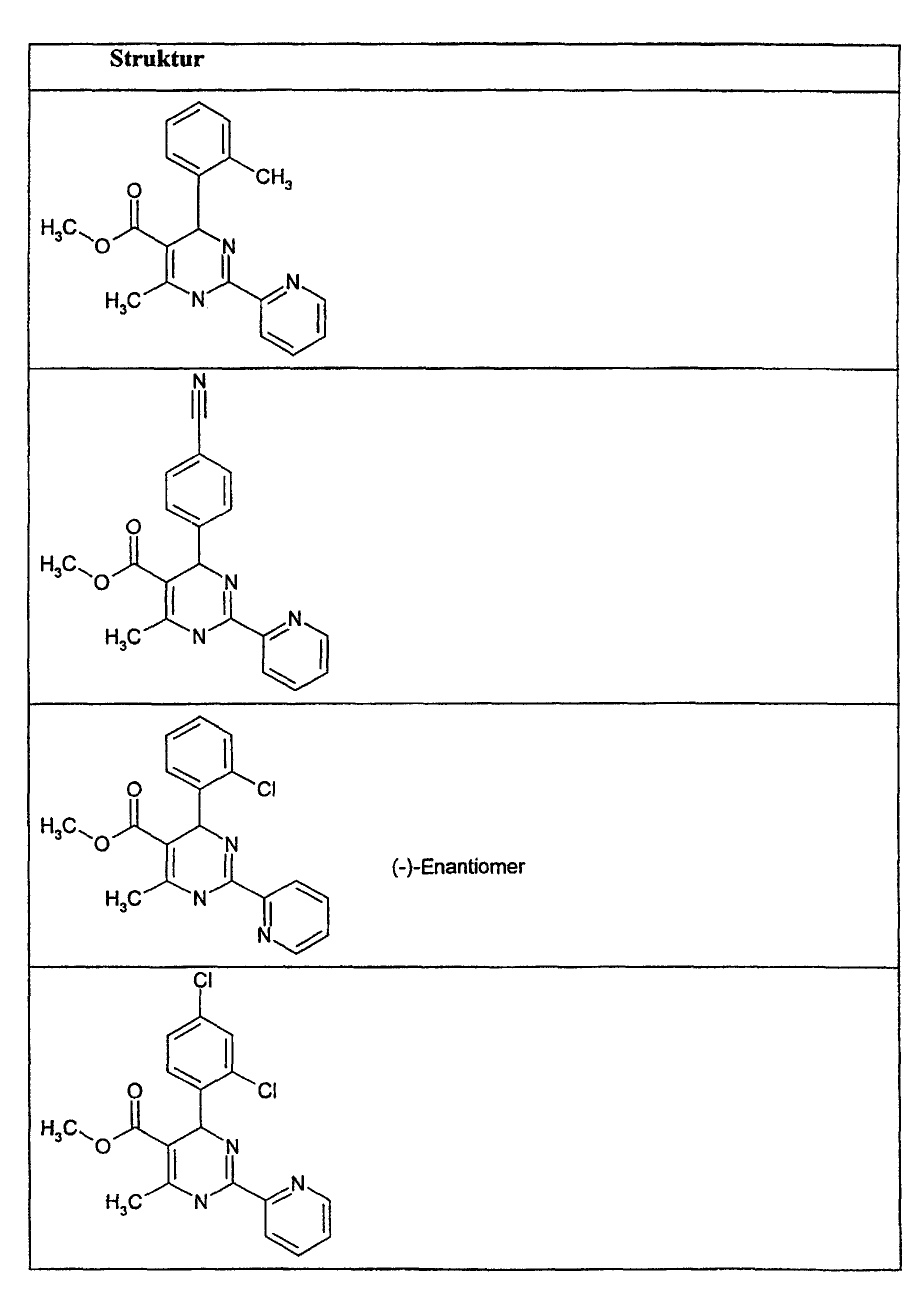

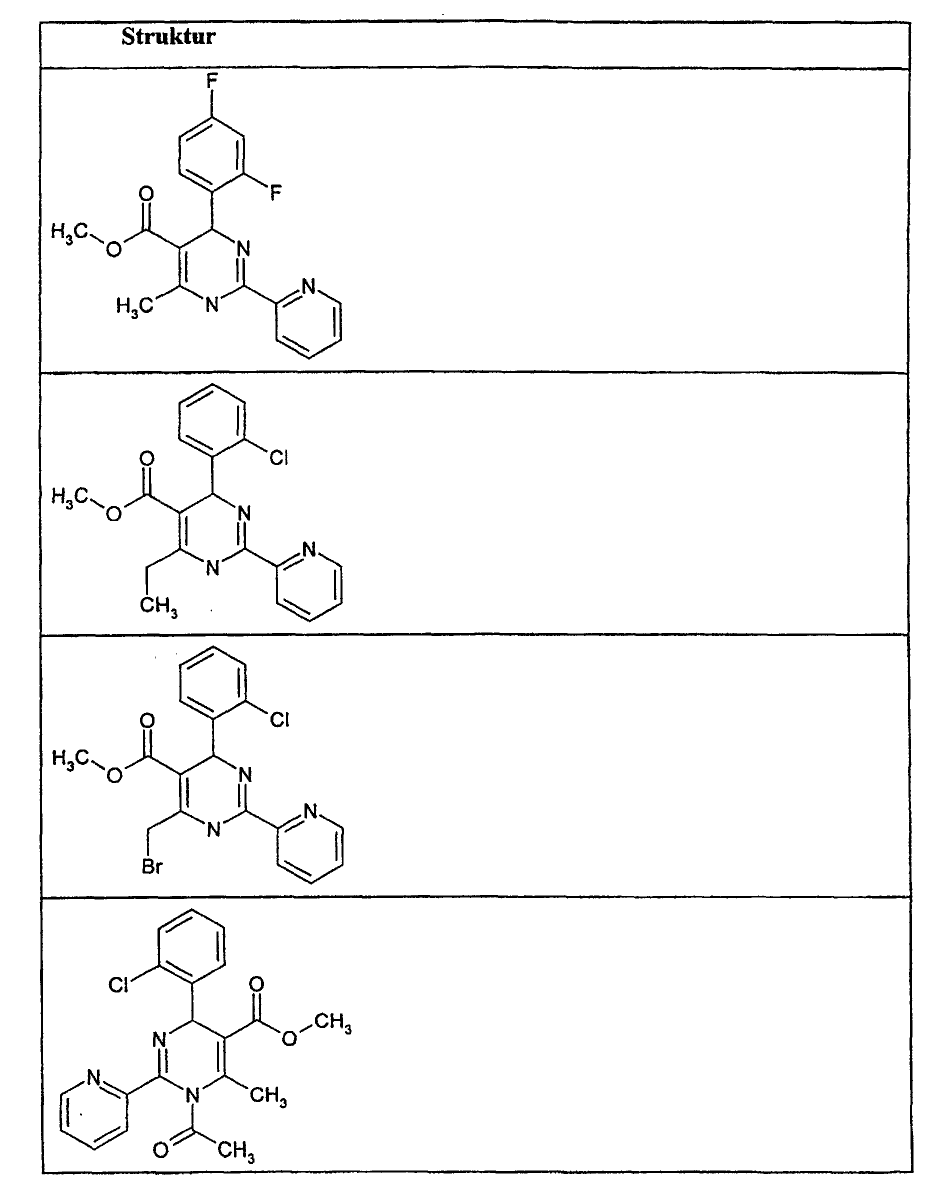

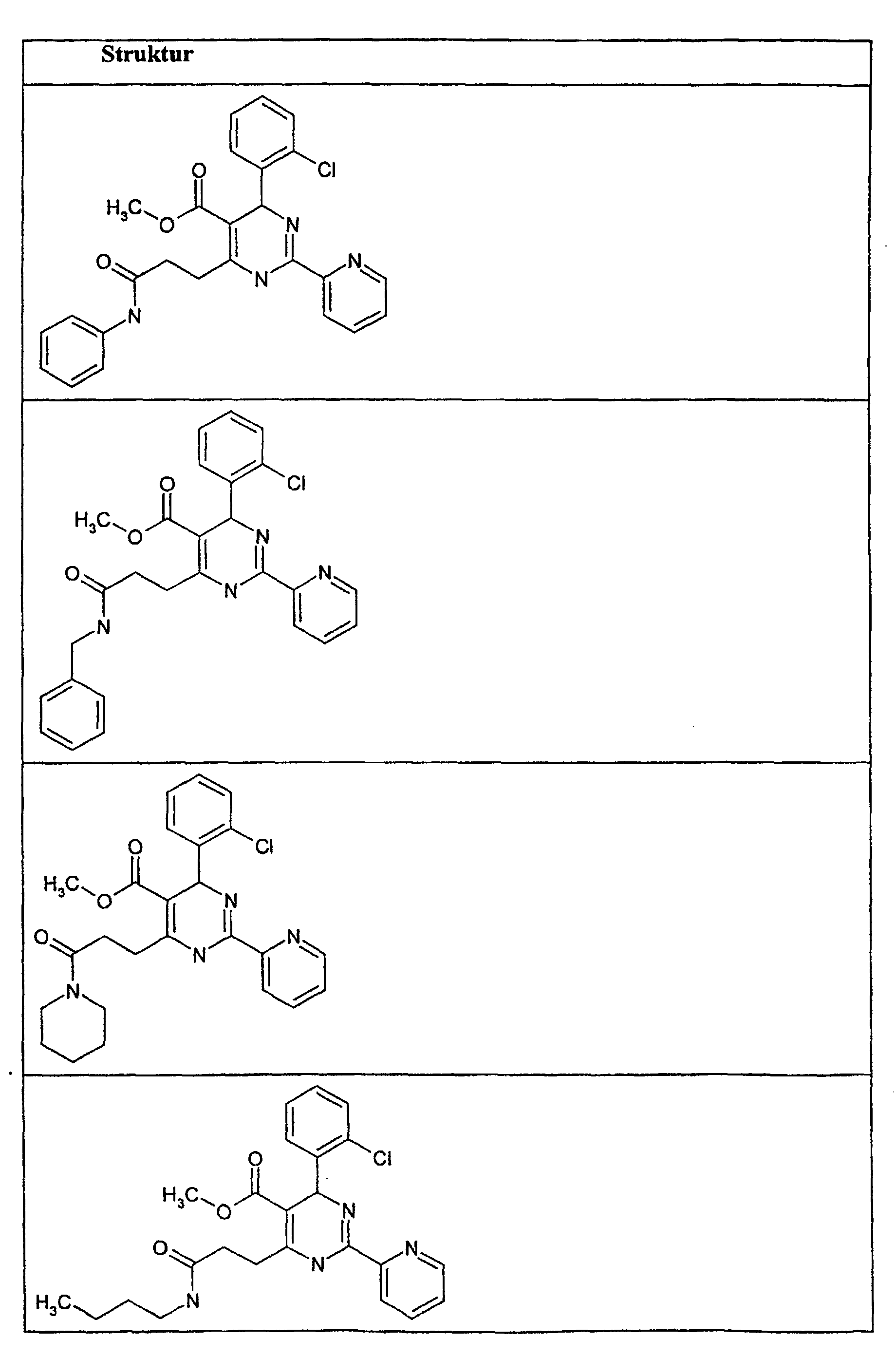

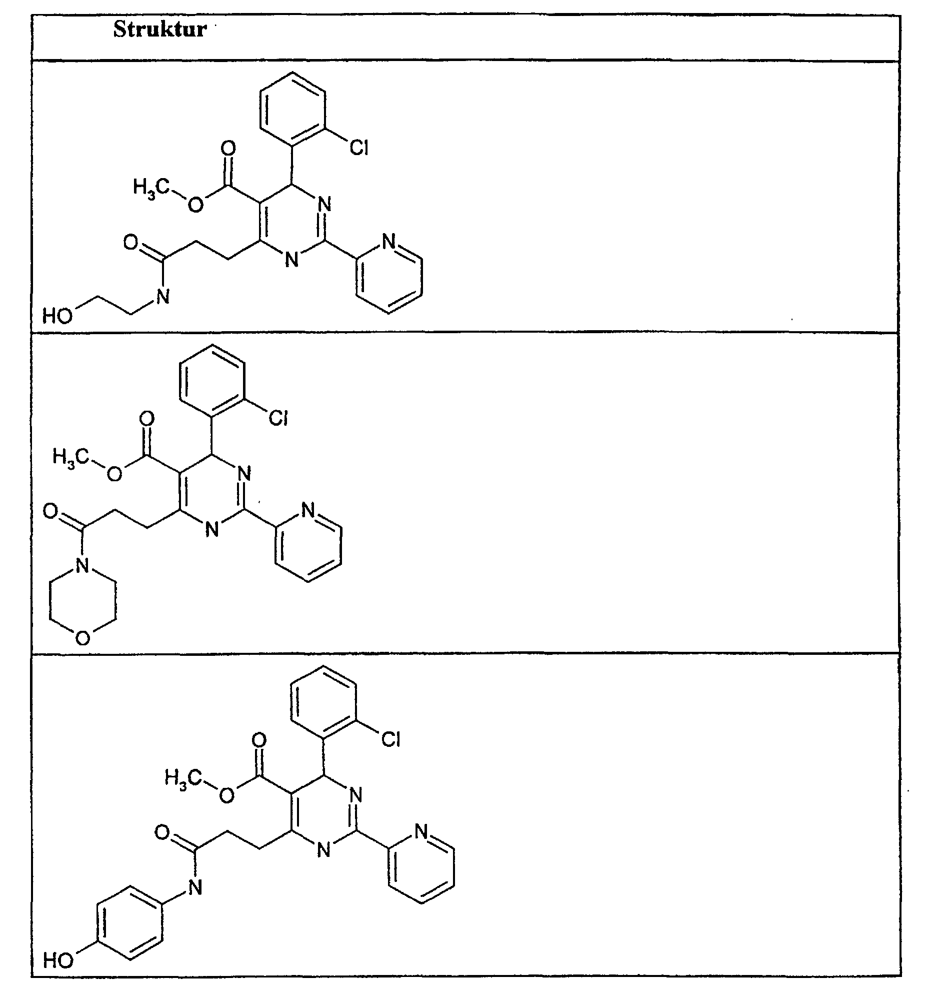

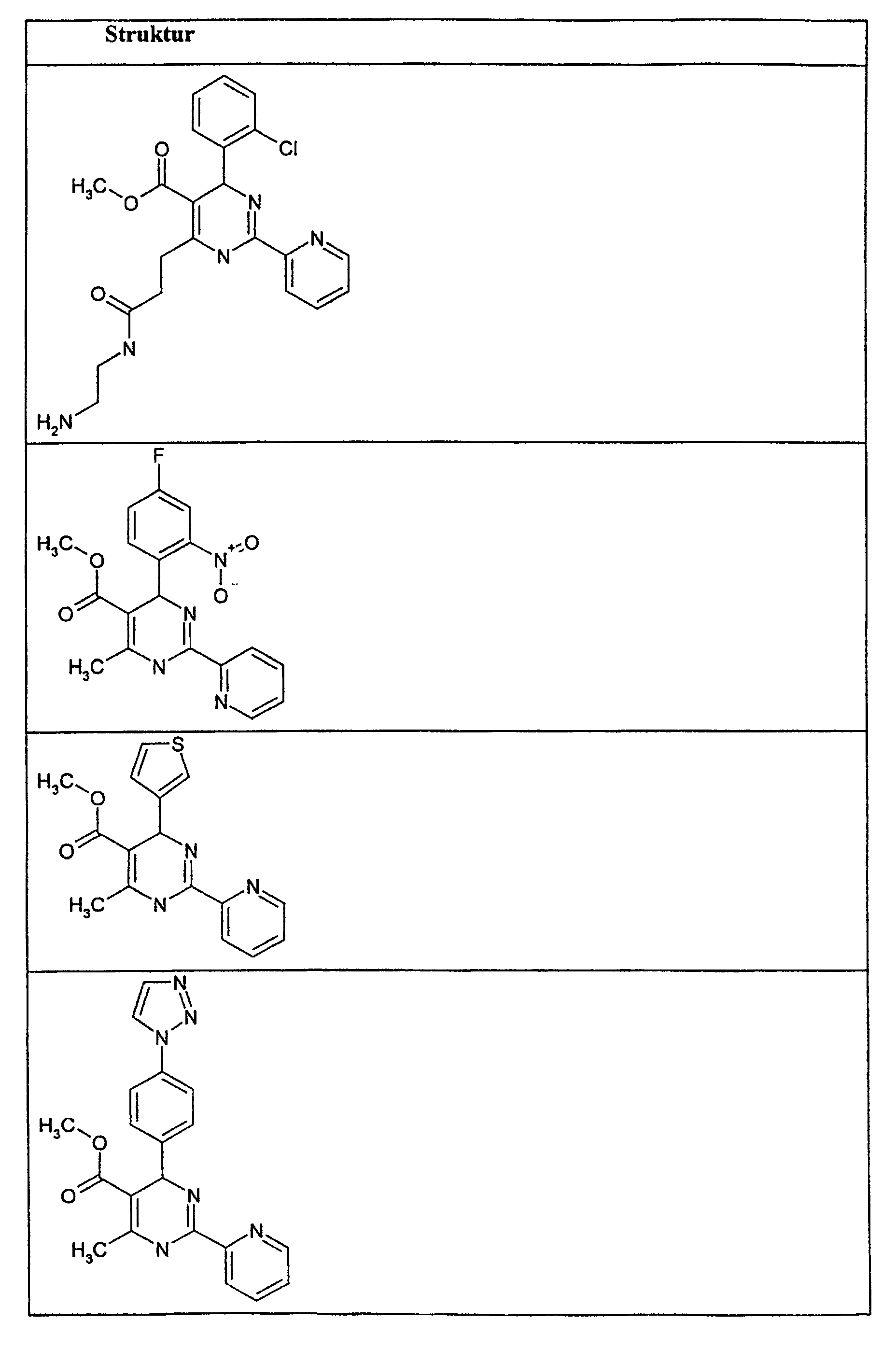

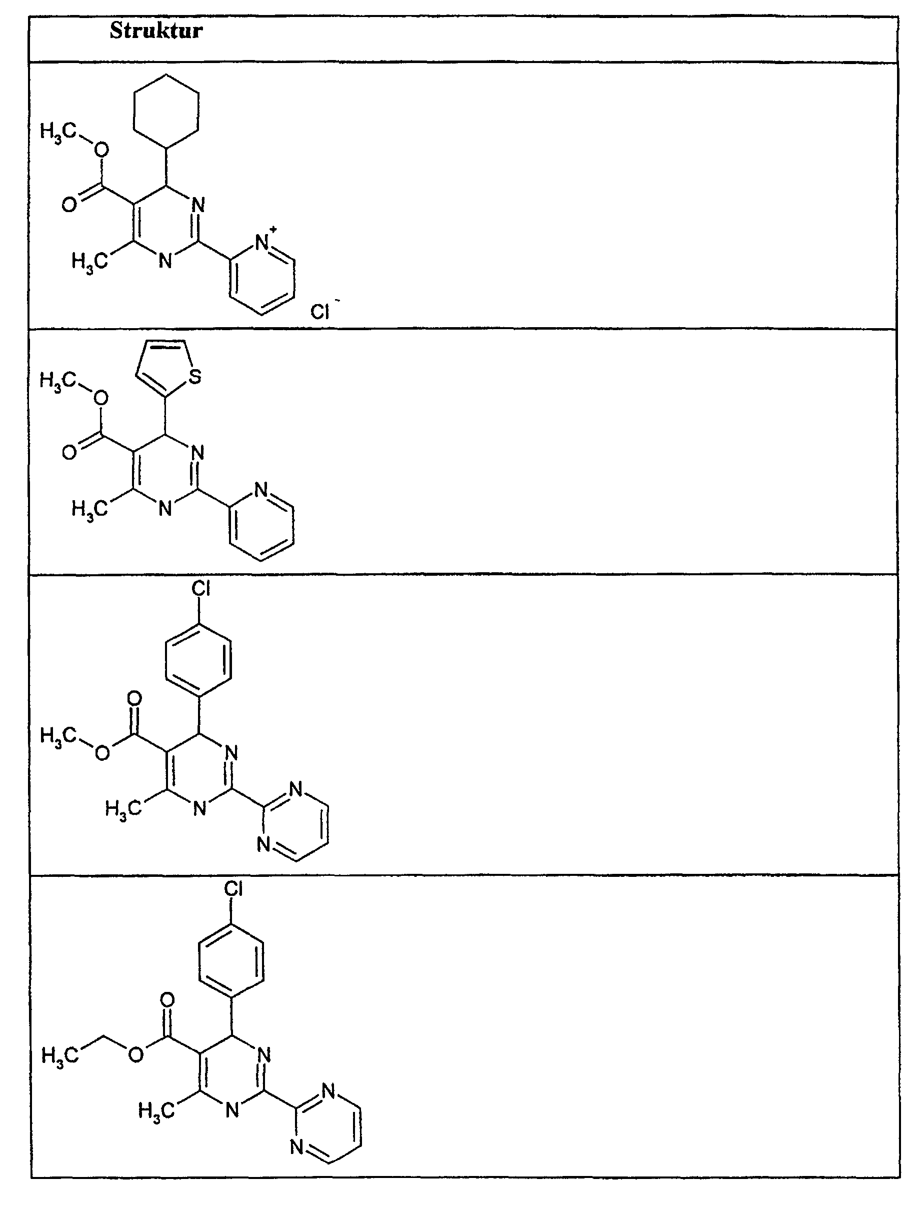

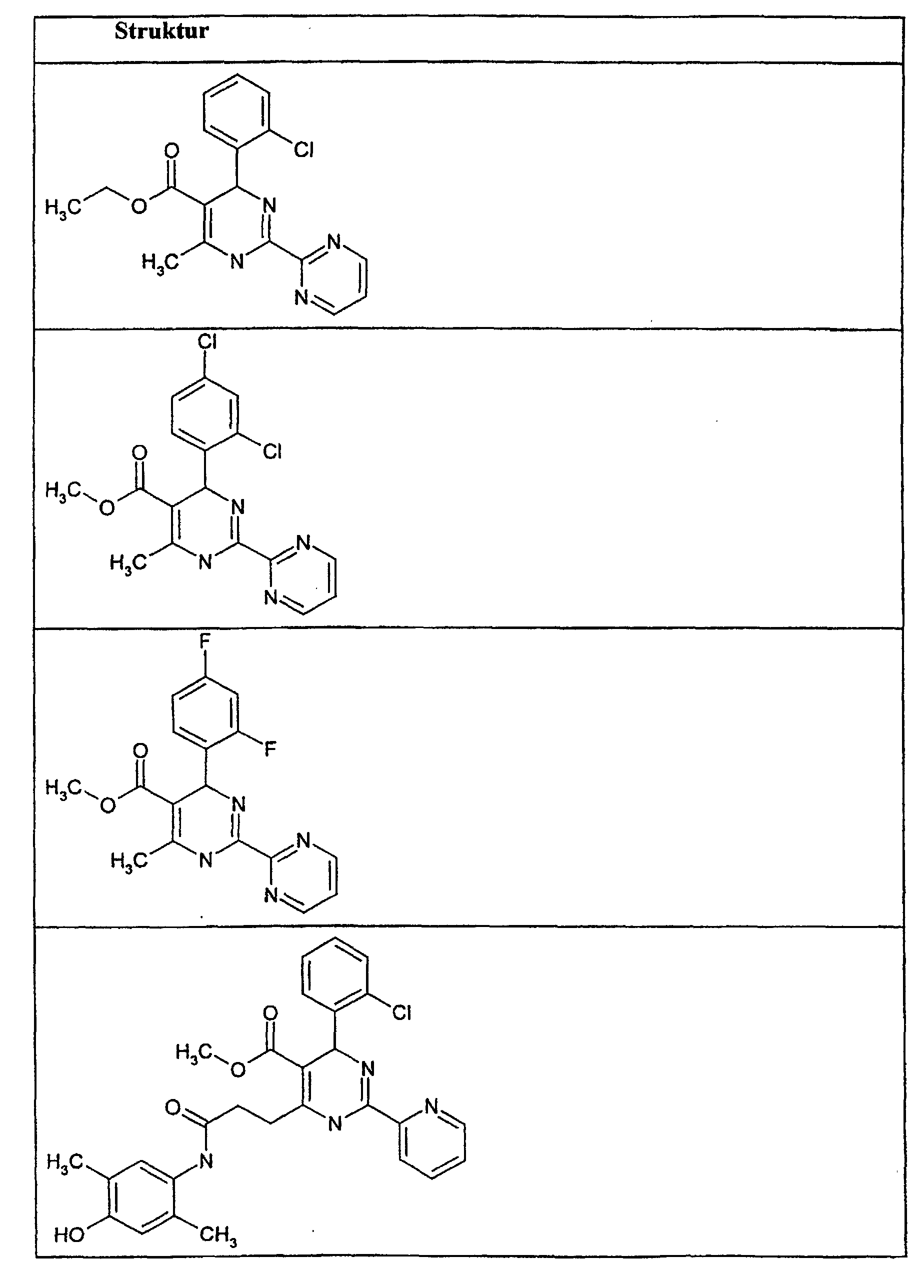

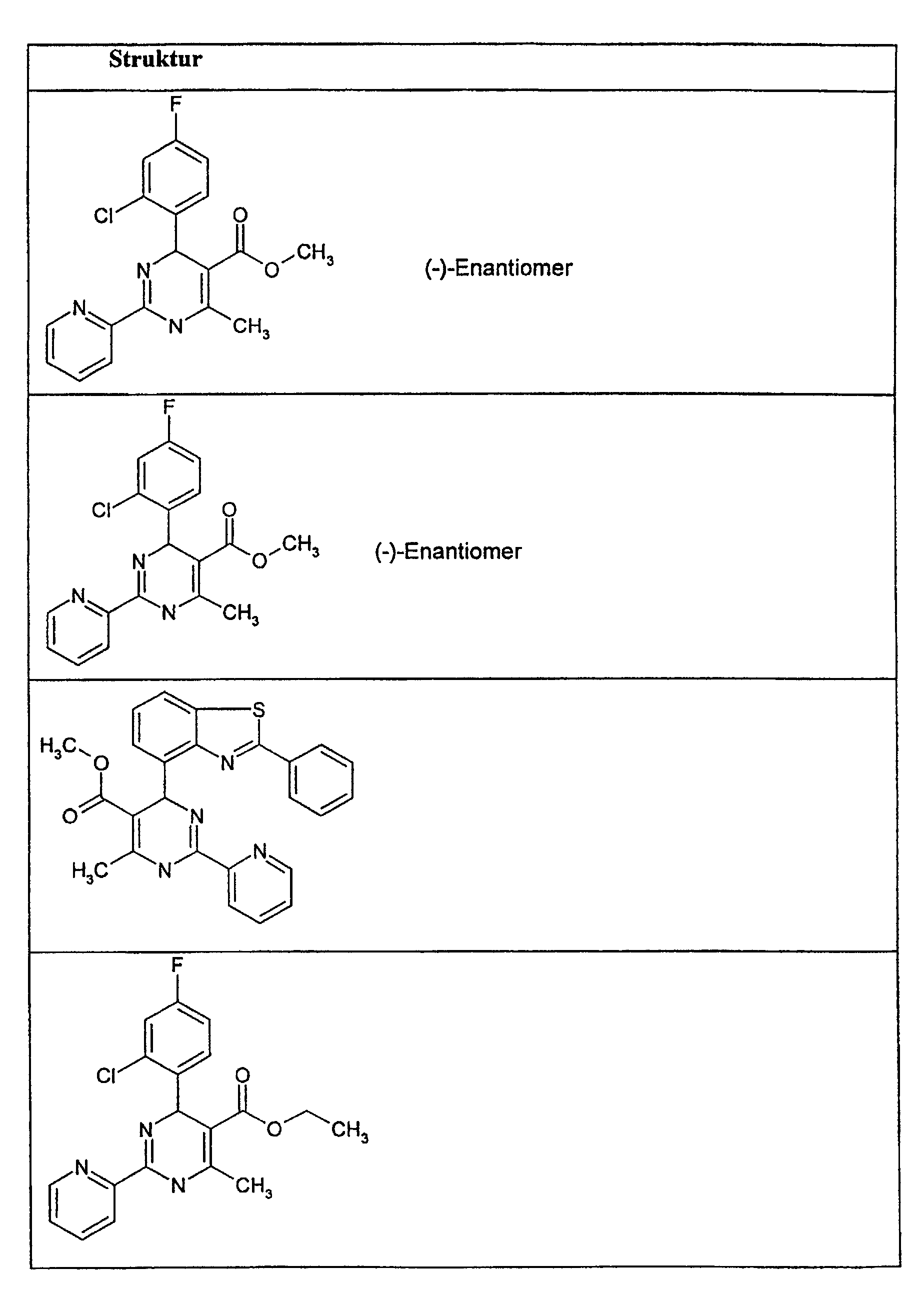

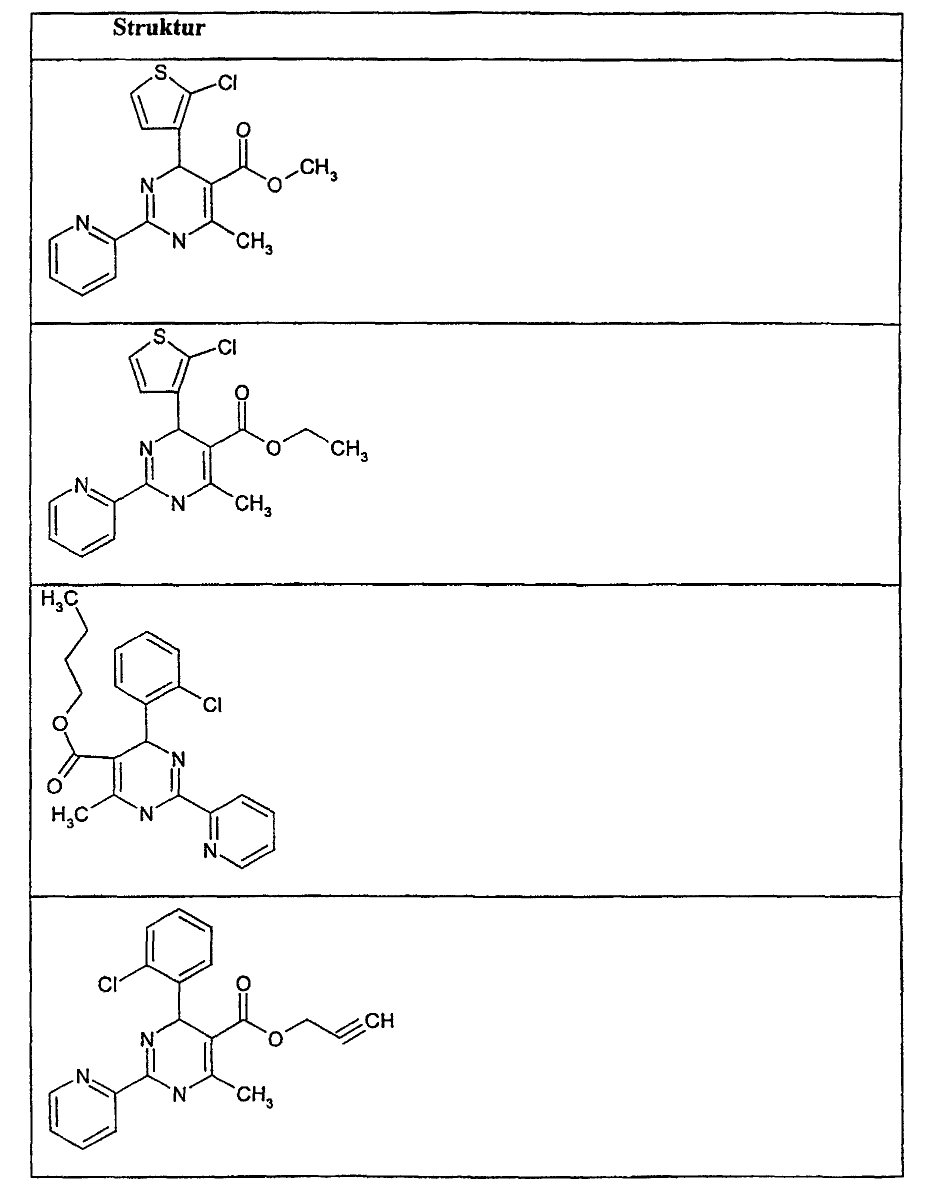

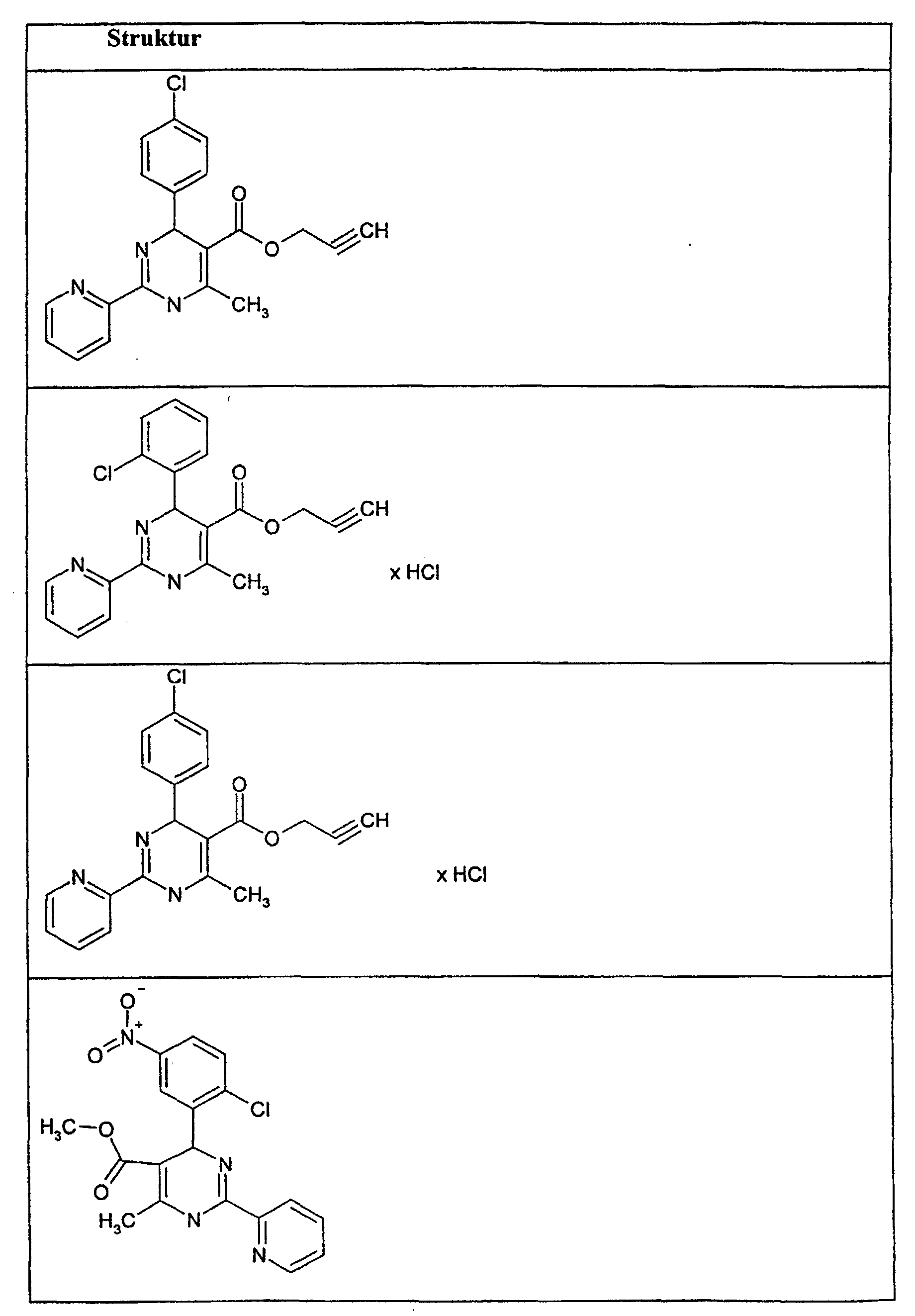

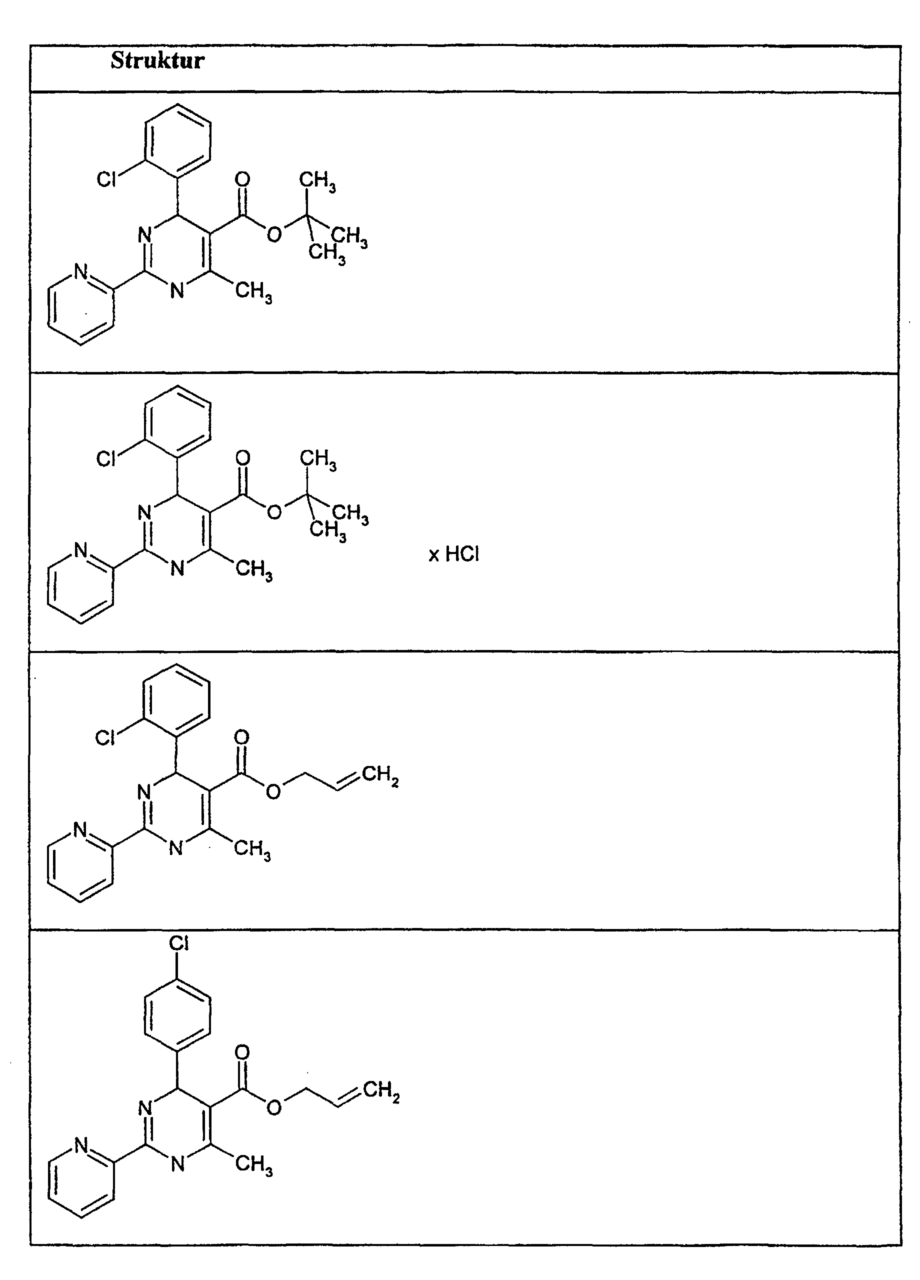

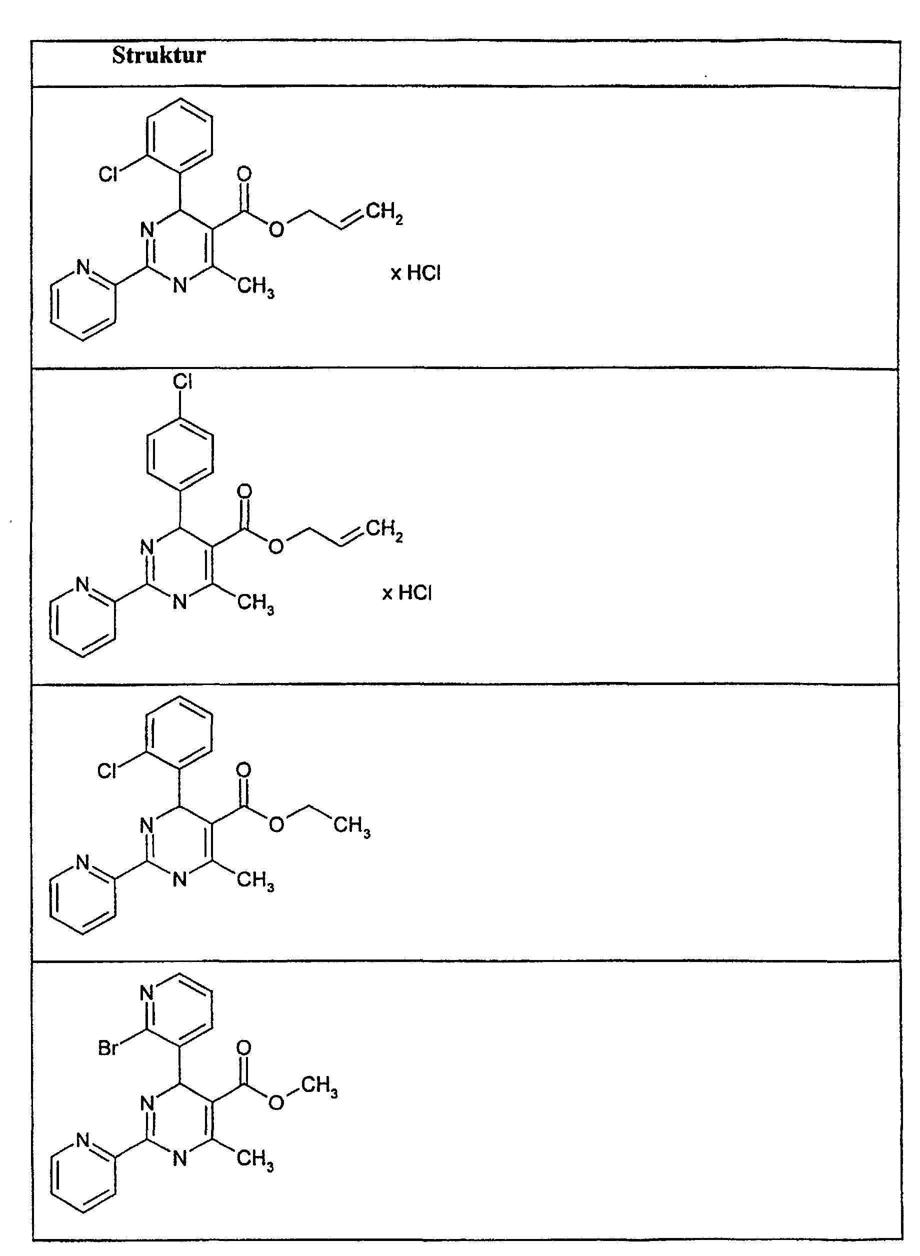

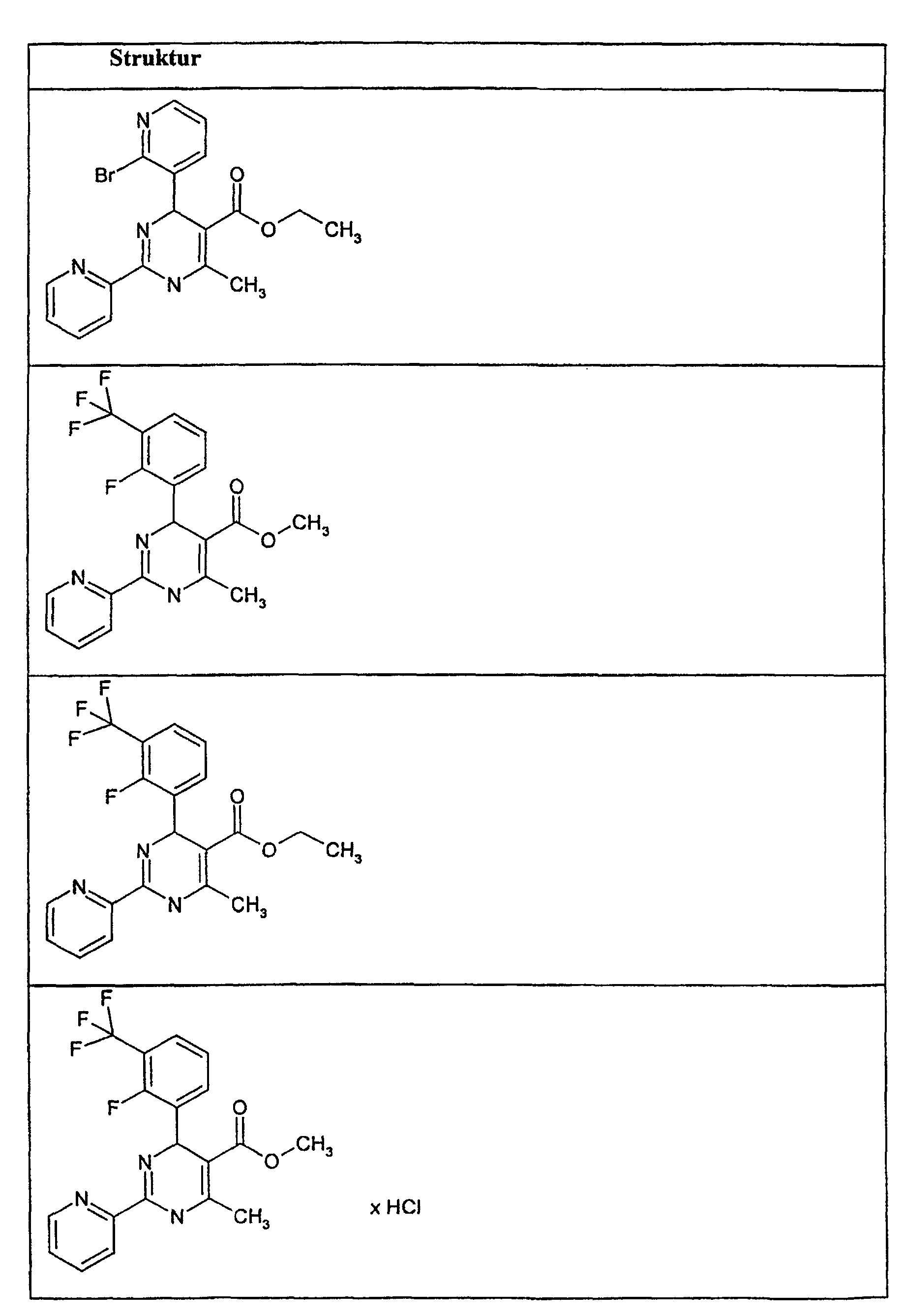

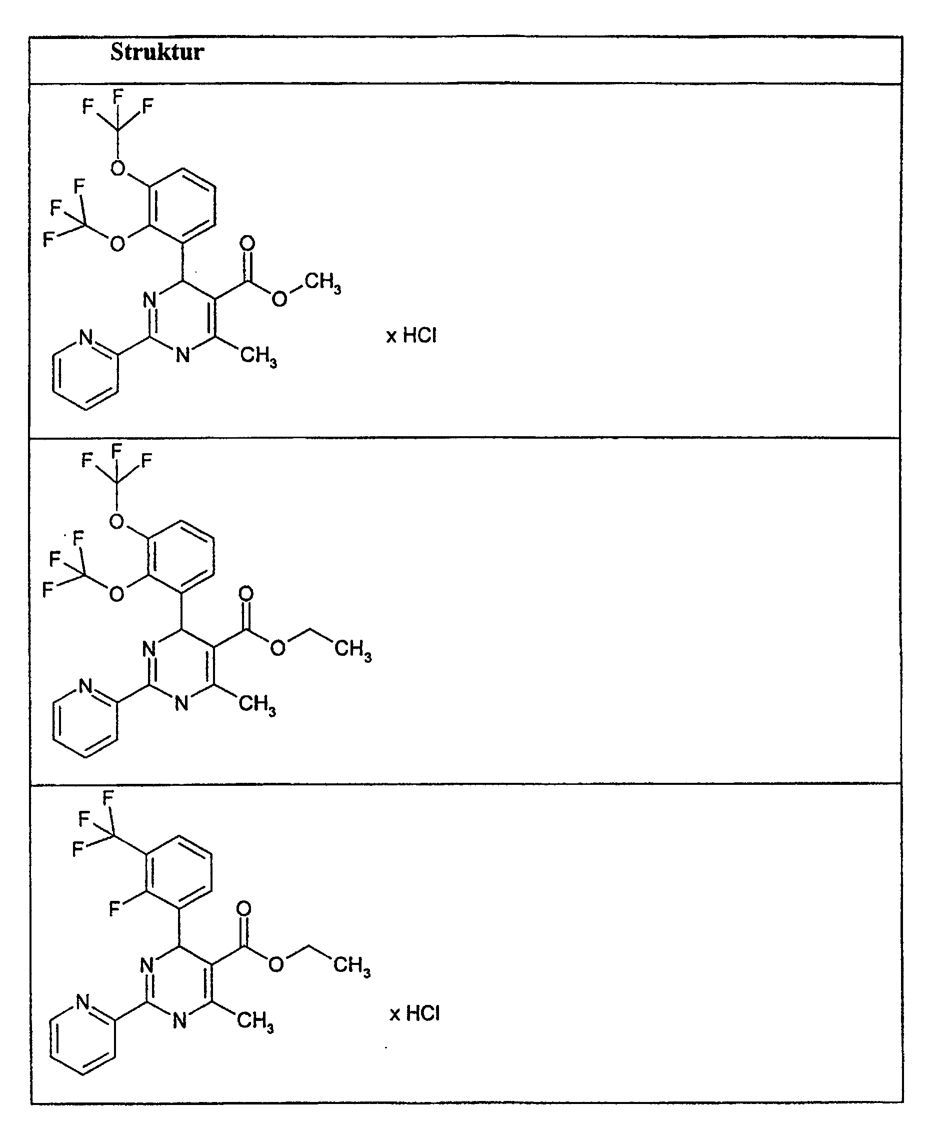

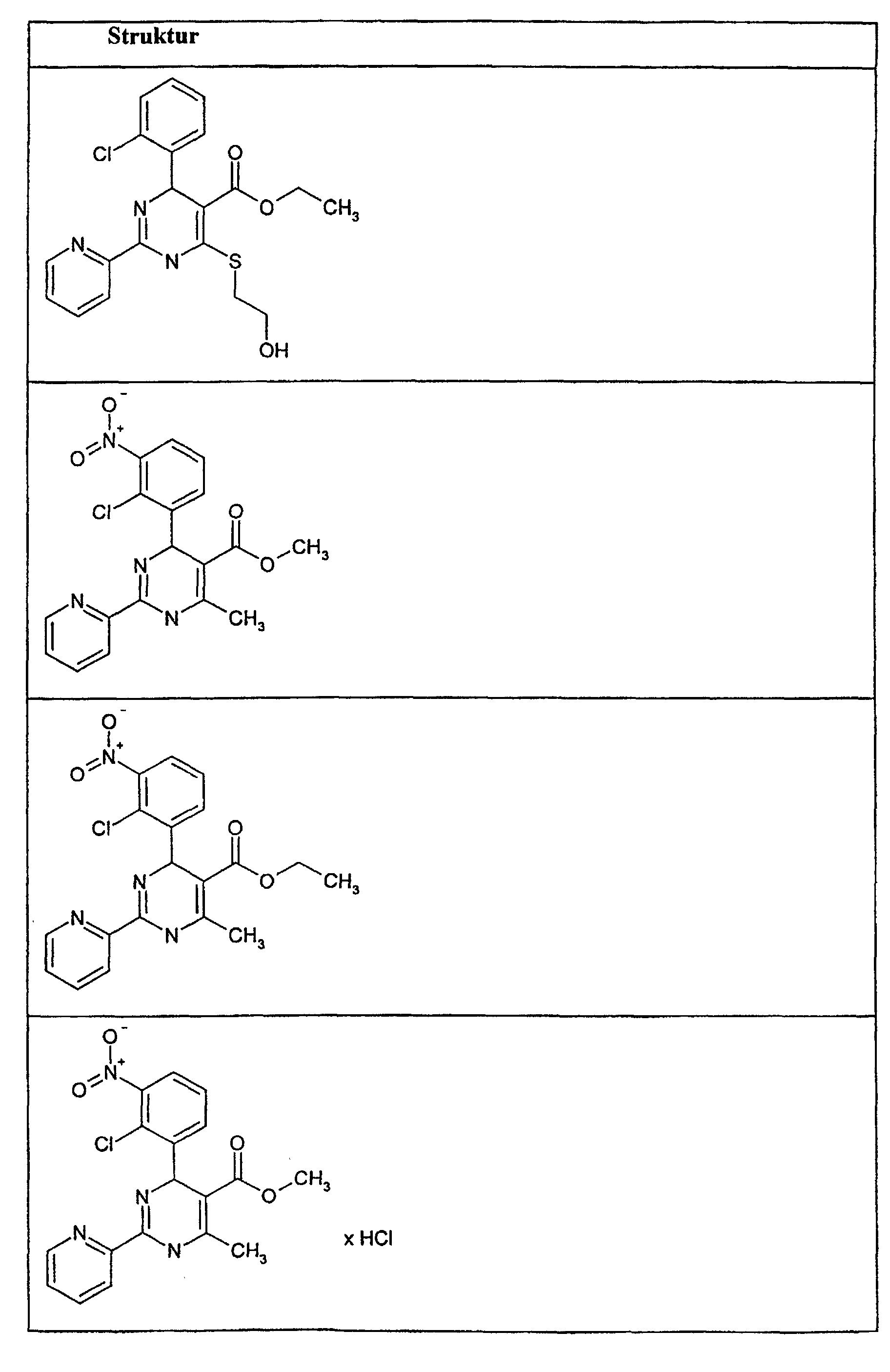

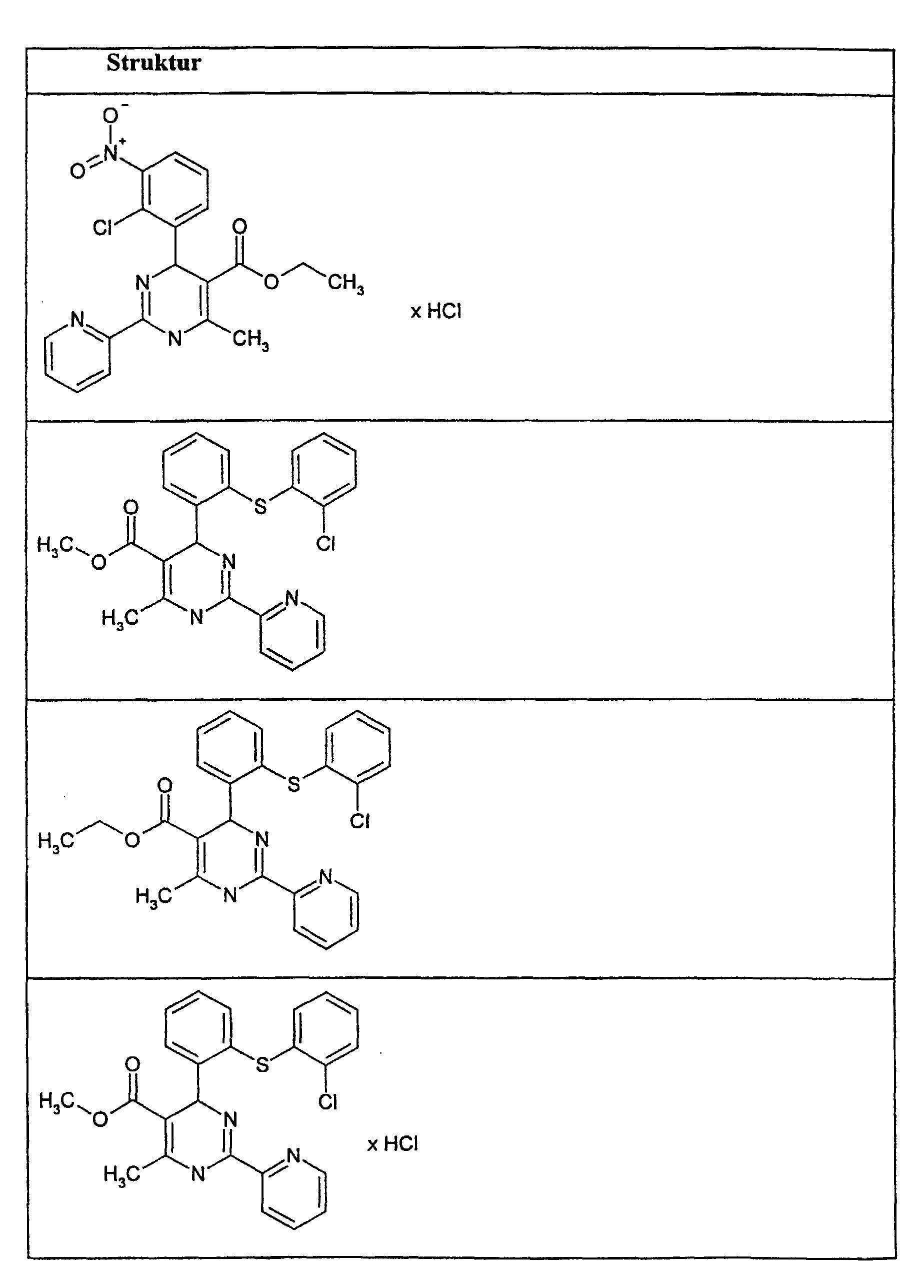

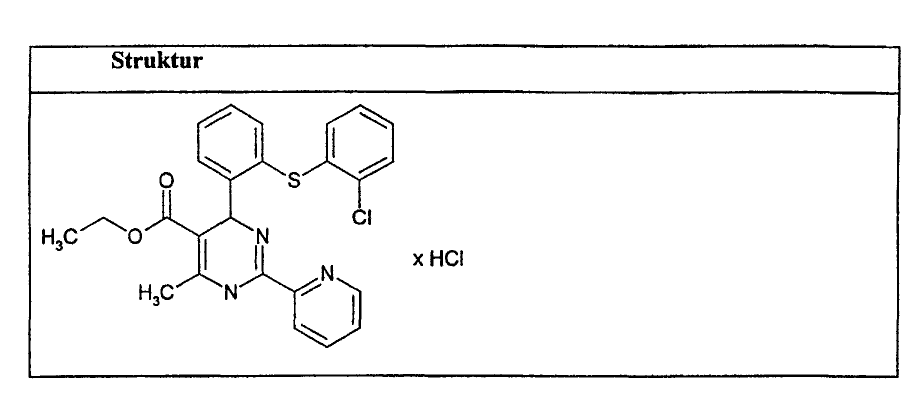

Bevorzugt verwendet werden die neuen Stoffe, die in der Tabelle B aufgeführt sind.

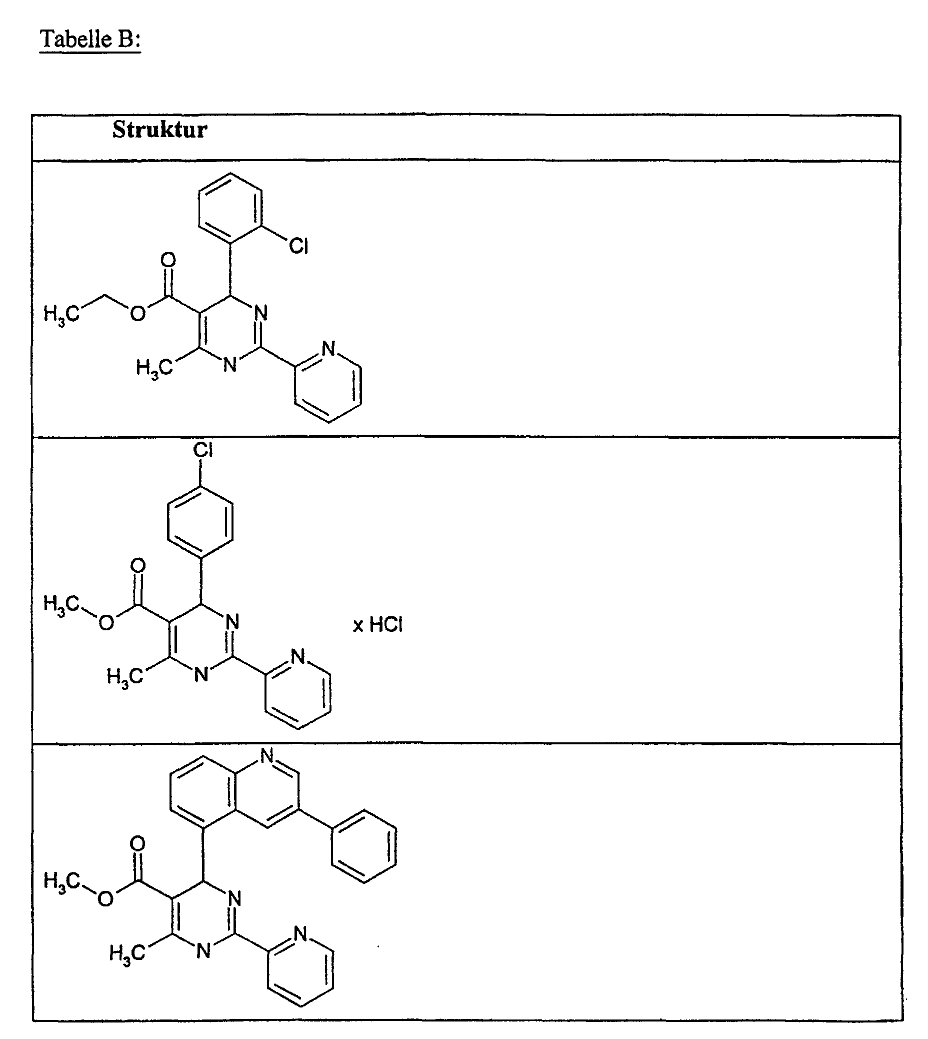

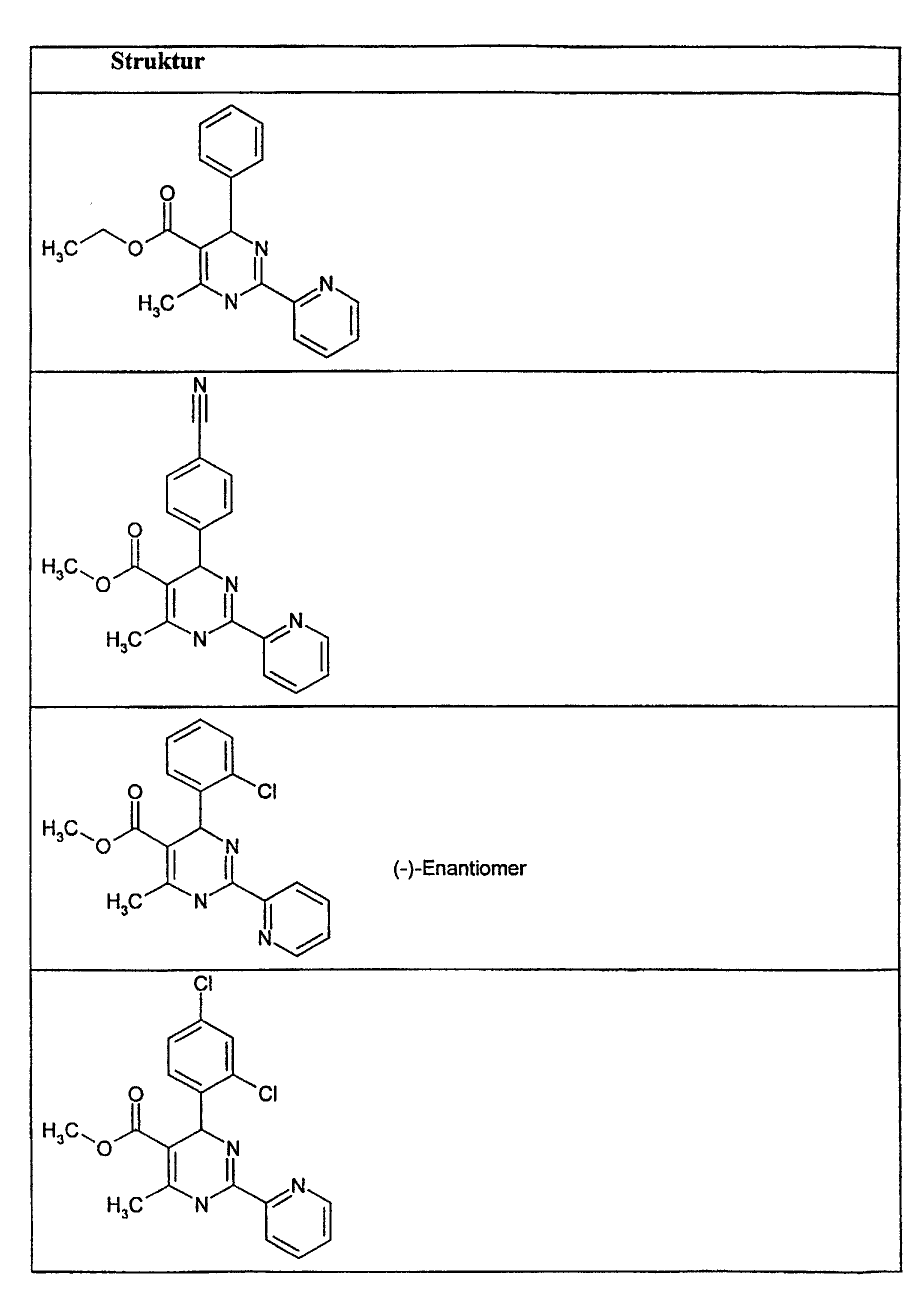

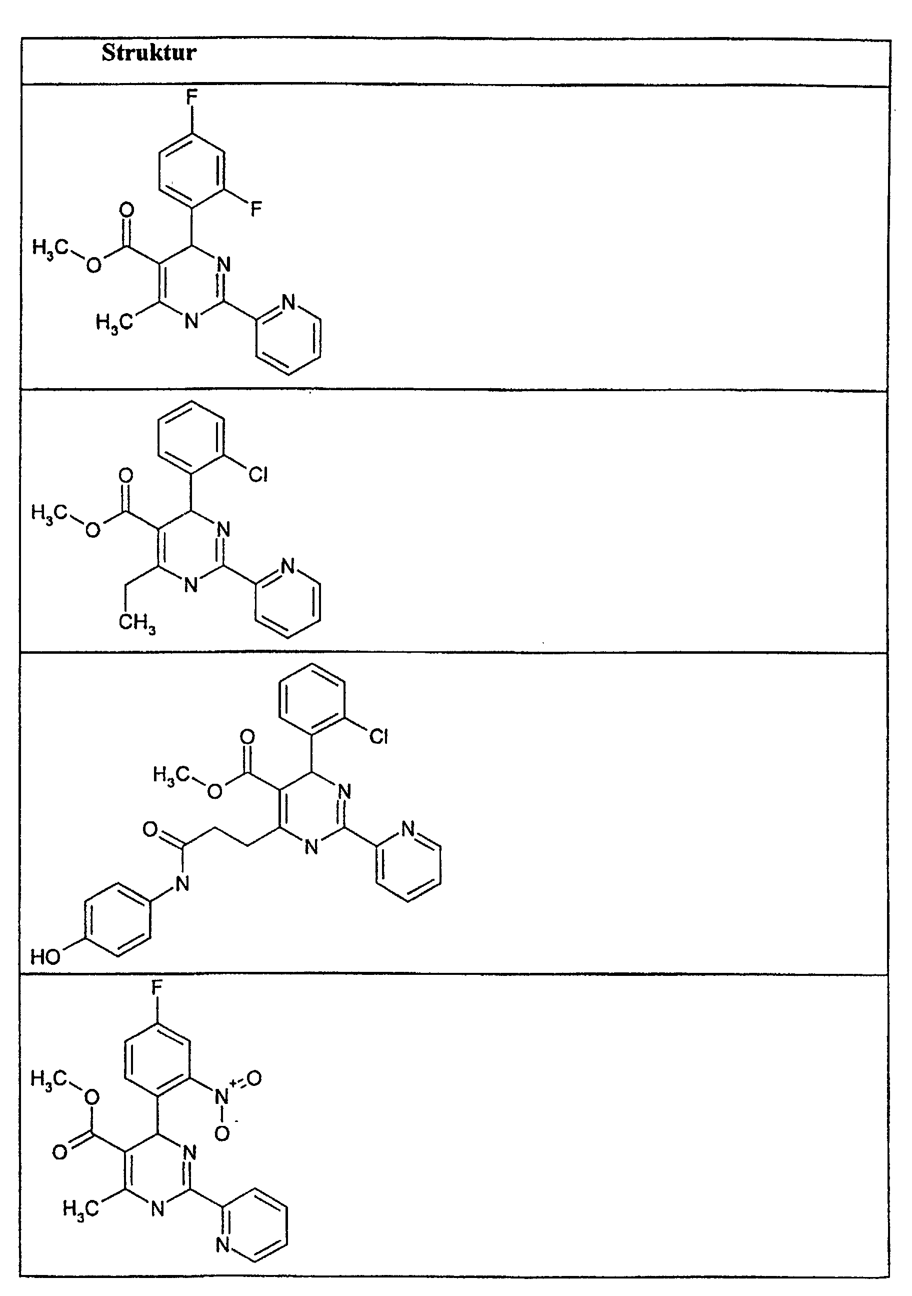

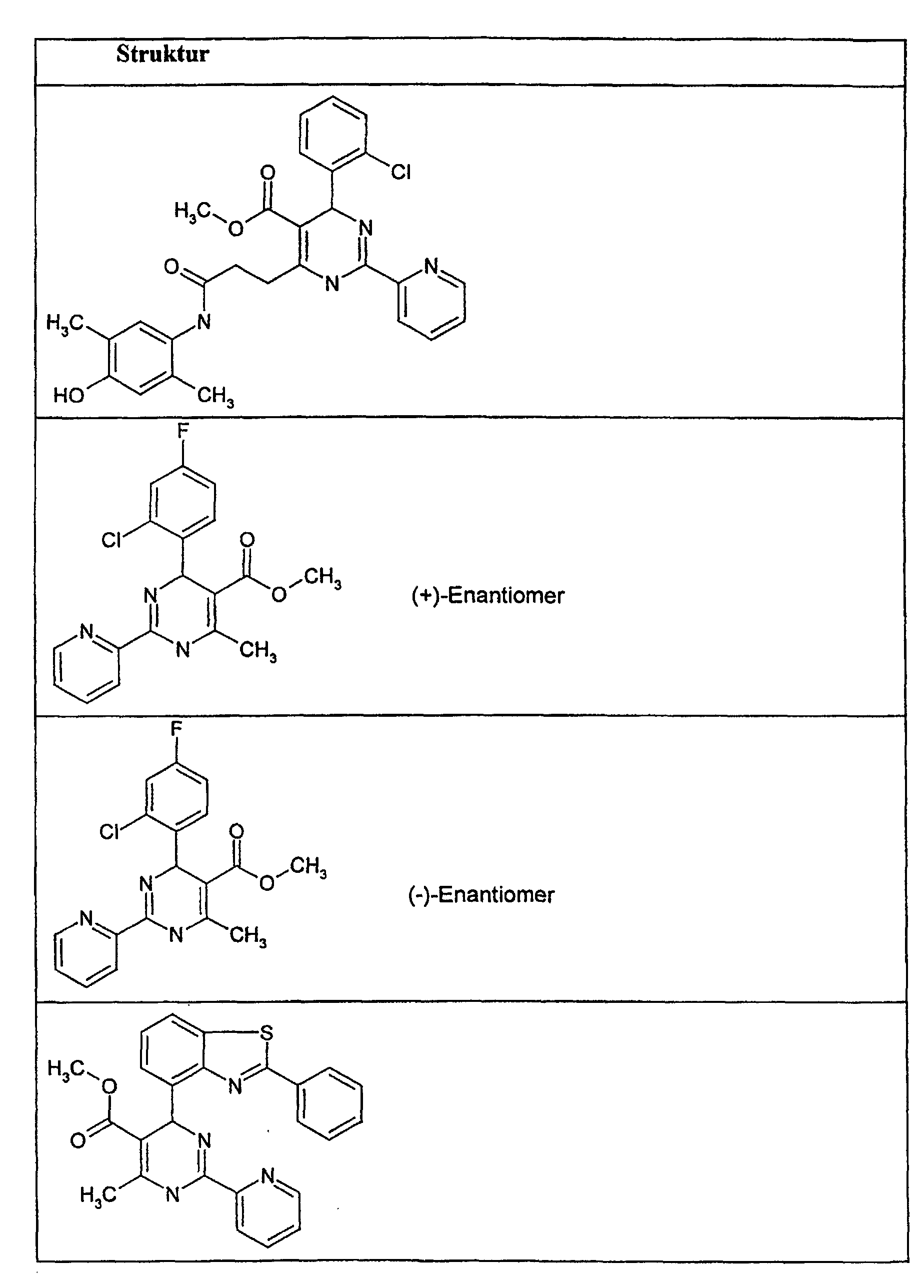

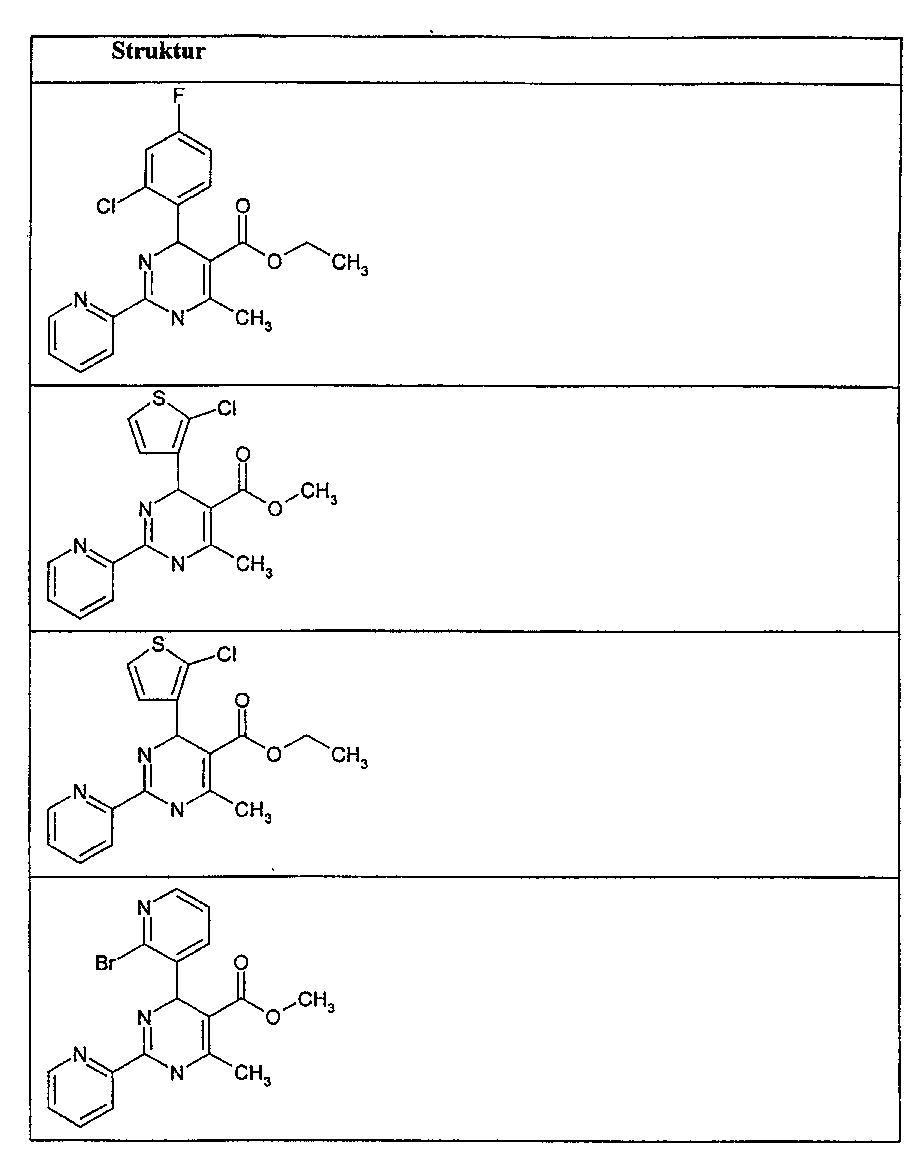

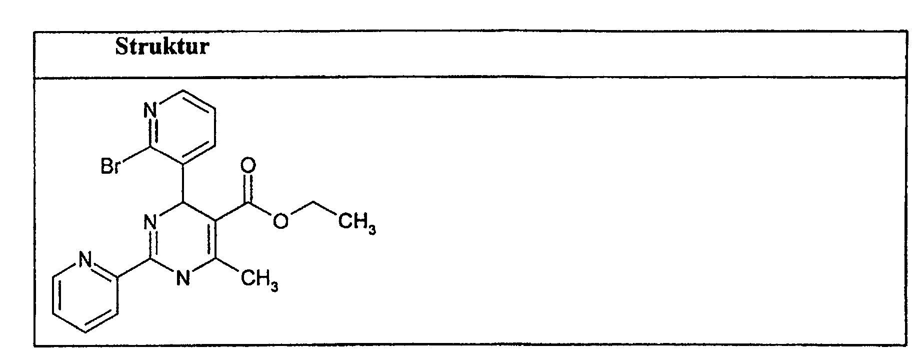

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen.

Die erfindungsgemäßen Stoffe können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Physiologisch unbedenkliche Salze können ebenso Metall- oder Ammoniumsalze der erfindungsgemäßen Verbindungen sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen der allgemeinen Formeln (I) und die neuen Stoffe (Tabelle A) können hergestellt werden,
indem man
[A] Aldehyde der allgemeinen Formel (II)

   R¹-CHO (II)

   in welcher
   - R¹: die oben angegebene Bedeutung hat,
   mit Amidinen der Formel (III) 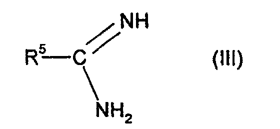 in welcher
   - R⁵: die oben angegebene Bedeutung hat,
   und Verbindungen der allgemeinen Formel (IV)

   R³-CO-CH₂-CO-R² (IV)

   in welcher
   - R² und R³: die oben angegebene Bedeutung haben,
   gegebenenfalls in Gegenwart inerter organischer Lösemittel mit oder ohne Basen- bzw. Säurezusatz umsetzt,
   oder
[B] Verbindungen der allgemeinen Formel (V) 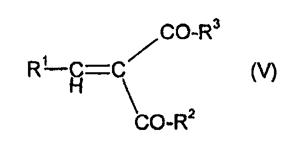 in welcher
   - R¹, R² und R³: die oben angegebene Bedeutung haben,
   mit Amidinen oder deren Hydrochloriden der allgemeinen Formel (III) 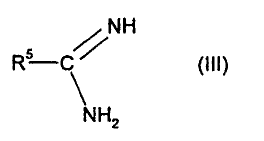 in welcher
   - R⁵: die oben angegebene Bedeutung hat,
   gegebenenfalls in Gegenwart inerter organischer Lösemittel bei Temperaturen zwischen 20°C und 150°C mit oder ohne Basen- oder Säurezusatz umsetzt,
   oder
[C] Aldehyde der allgemeinen Formel (II)

   R¹-CHO (II)

   in welcher
   - R¹: die oben angegebene Bedeutung hat,
   mit Verbindungen der allgemeinen Formel (VI) 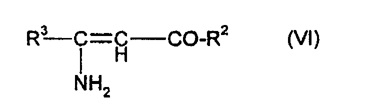 in welcher
   - R² und R³: die oben angegebene Bedeutung haben,
   und Amidinen der allgemeinen Formel (III) wie oben beschrieben umsetzt,
   oder
[D] Aldehyde der allgemeinen Formel (II) mit Verbindungen der allgemeinen Formel (IV) und Iminoethern der allgemeinen Formel (VII) 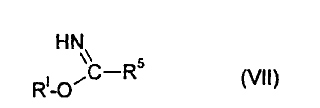 in welcher
   - R⁵: die oben angegebene Bedeutung hat,
   und
   - R¹: für (C₁-C₄)-Alkyl steht,
in Gegenwart von Ammoniumsalzen umsetzt.

Das bevorzugte erfindungsgemäße Verfahren [A] kann durch folgendes Formelschema beispielhaft erläutert werden:

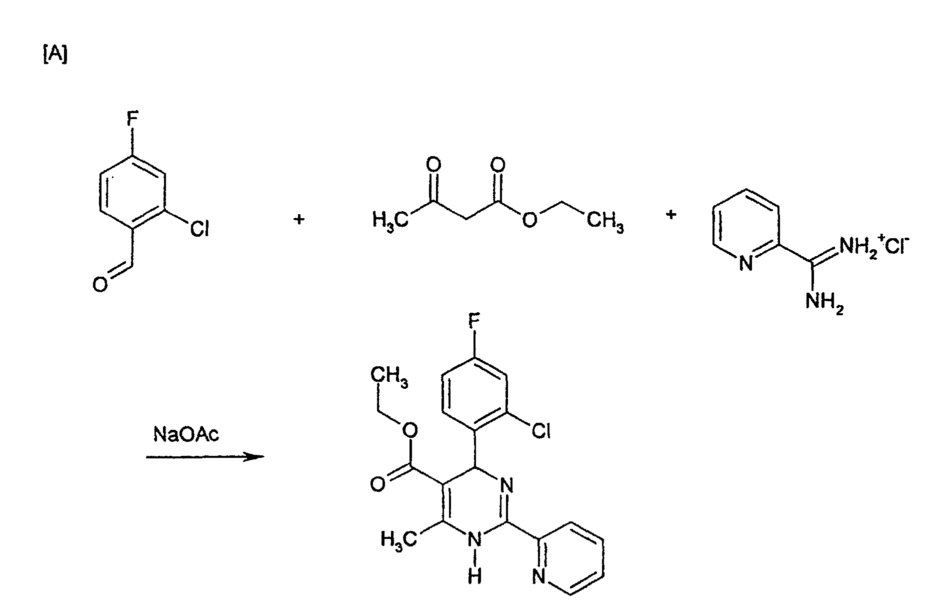

Für alle Verfahrensvarianten A, B, C und D kommen als Lösemittel alle inerten organischen Lösemittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150°C, vorzugsweise jedoch bei der Siedetemperatur des jeweiligen Lösemittels.

Die Umsetzung kann bei Normaldruck, aber auch bei erhöhtem Druck durchgeführt werden. Im allgemeinen arbeitet man unter Normaldruck.

Die Umsetzung kann mit oder ohne Basen- bzw. Säurezusatz durchgeführt werden, es hat sich jedoch gezeigt, daß eine Umsetzung im Sinne der Erfindung vorzugsweise in Gegenwart von schwächeren Säuren wie z.B. Essigsäure oder Ameisensäure stattfindet.

Die als Ausgangsstoffe verwendeten Aldehyde der allgemeinen Formel (II) sind bekannt oder können nach literaturbekannten Methoden darstellt werden [vgl. T.D. Harris und G.P. Roth, J. Org. Chem. 44, 146 (1979), Deutsche Offenlegungsschrift 2 165 260, Juli 1972, Deutsche Offenlegungsschrift 2 401 665, Juli 1974, Mijano et al., Chem. Abstr. 59, (1963), 13 929 c, E. Adler und H.-D. Becker, Chem. Scand. 15, 849 (1961), E.P. Papadopoulos, M. Mardin und Ch. Issidoridis, J. Org. Chem. Soc. 78, 2543 (1956)).

Die als Ausgangsstoffe verwendeten Yliden-β-ketoester der Formel (V) können nach literaturbekannten Methoden dargestellt werden [vgl. G. Jones, "The Knoevenagel Condensation", in Organic Reactions, Vol. XV, 204 ff. (1967)].

Die als Ausgangsstoffe verwendeten Enaminocarbonsäureester der Formel (VI) und die Iminoether der allgemeinen Formel (VII) sind bekannt oder können nach literaturbekannten Methoden dargestellt werden [vgl. S.A. Glickman and A.C. Cope, J. Am. Chem. Soc. 67, 1017 (1945)].

Die als Ausgangsstoffe verwendeten β-Ketocarbonsäureester der allgemeinen Formel (IV) sind bekannt oder können nach literaturbekannten Methoden dargestellt werden [z.B. D. Borrmann, "Umsetzung von Diketen mit Alkoholen, Phenolen und Mercaptanen", in Houben-Weyl, Methoden der organischen Chemie, Vol. VII/4, 230 ff (1968); Y. Oikawa, K. Sugano und O. Yonemitsu, J. Org. Chem. 43, 2087 (1978)].

Die Verbindungen der allgemeinen Formel (III) können hergestellt werden, indem man Verbindungen der Formel (VIII)

R⁵-CN (VIII)

in welcher
- R⁵: die oben angegebene Bedeutung hat,
wie üblich über die Iminoether und abschließend mit Ammoniumchlorid in Methanol umsetzt [vgl. hierzu in Analogie zu W.K. Fife, Heterocycles 22, 93-96 (1984); T. Sakamoto, S. Kaneda, S. Nishimura, H. Yamanaka, Chem. Pharm. Bull. 33, 565, 571 (1986)].

Alle Verfahrensschritte erfolgen bei Normaldruck und in einem Temperaturbereich von 0°C bis 130°C, bevorzugt von 20°C bis 100°C.

Die Verbindungen der allgemeinen Formel (VIII) sind an sich bekannt.

Die antivirale Wirkung der erfindungsgemäßen Verbindungen wurde in Anlehnung an die von Sells et al. ( M.A. Sells, M.-L. Chen, and G. Acs (1987) Proc. Natl. Acad. Sci. 84, 1005 - 1009) und Korba et al. (B.E. Korba and J.L. Gerin (1992) Antiviral Research 19, 55 - 70) beschriebenen Methoden bestimmt.

Die antiviralen Tests wurden in 96-well-Mikrotiterplatten durchgeführt. Die erste vertikale Reihe der Platte erhielt nur Wachstumsmedium und HepG2.2.15-Zellen. Sie diente als Viruskontrolle.
Stammlösungen der Testverbindungen (50 mM) wurden zunächst in DMSO gelöst, weitere Verdünnungen wurden in Wachstumsmedium der HepG2.2.15 hergestellt. Die erfmdungsgemäßen Verbindungen wurden in der Regel in einer Testkonzentration von 100 µM (1. Testkonzentration) jeweils in die zweite vertikale Testreihe der Mikrotiterplatte pipettiert und anschließend in 2-er Schritten 2¹⁰ fach in Wachstumsmedium plus 2% foetales Kälberserum, verdünnt (Volumen 25 µl).
Jeder Napf der Mirotiterplatte erhielt dann 225 µl einer HepG2.2.15 Zellsuspension (5 x 10⁴ Zellen/ml) in Wachstumsmedium plus 2% fötales Kälberserum.
Der Testansatz wurde 4 Tage, 37° Celsius, 5 % CO₂, inkubiert.
Anschließend wurde der Überstand abgesaugt und verworfen, und die Näpfe erhielten 225 µl frisch zubereitetes Wachtumsmedium. Die erfindungsgemäßen Verbindungen wurden jeweils erneut als 10-fach konzentrierte Lösung in einem Volumen von 25 µl zugefügt. Die Ansätze wurden weitere 4 Tage inkubiert.
Vor der Ernte der Überstände zur Bestimmung des antiviralen Effektes wurden die HepG2.2.15-Zellen lichtmikroskopisch oder mittels biochemischer Nachweisverfahren (z.B. Alamar Blue Färbung oder Trypanblau-Färbung) auf zytotoxische Veränderungen untersucht.
Anschließend wurden die Überstände geerntet und mittels Vakuum auf mit Nylonmembran bespannten 96 Napf Dot Blot Kammern (entsprechend den Angaben der Hersteller) gesogen.

### Zytotoxizitätsbestimmung

Substanzinduzierte zytotoxische oder zytostatische Veränderungen der HepG2.2.15-Zellen wurden z.B. lichtmikroskopisch als Änderungen der Zellmorphologie ermittelt. Derartige Substanz-induzierte Veränderungen der HepG2.2.15 Zellen im Vergleich zu unbehandelten Zellen wurden z.B. als Zellyse, Vakuolisierung oder veränderter Zellmorphologie sichtbar. 50 % Zytotoxizität (Tox.-50) bedeuten, daß 50 % der Zellen eine der entsprechenden Zellkontrolle vergleichbare Morphologie aufweisen.
Die Verträglichkeit einiger der erfindungsgemäßen Verbindungen wurde zusätzlich auf anderen Wirtszellen wie z.B. HeLa-Zellen, primäre periphere Blutzellen des Menschen oder transformierte Zellinien wie H-9-Zellen, getestet.
Es konnten keine Zell-zytotoxischen Veränderungen bei Konzentrationen der erfindungsgemäßen Verbindungen von >10µM festgestellt werden.

### Bestimmung der antiviralen Wirkung

Nach Transfer der Überstände auf die Nylon Membran der Blot Apparatur (s.o.) wurden die Überstände der HepG2.2.15-Zellen denaturiert (1.5 M NaCl/ 0.5 N NaOH), neutralisiert (3 M NaCl/ 0.5 M Tris HCl, pH 7.5) und gewaschen (2 x SSC).

Anschließend wurde die DNA durch Inkubation der Filter bei 120 °C, 2 - 4 Stunden, an die Membran gebacken.

### Hybridisierung der DNA

Der Nachweis der viralen DNA von den behandelten HepG2.2.15-Zellen auf den Nylonfiltern wurde in der Regel mit nichtradioaktiven, Digoxigenin-markierten Hepatitis B spezifischen DNA-Sonden durchgeführt, die jeweils nach Angabe des Herstellers mit Digoxigenin markiert, gereinigt und zur Hybridisierung eingesetzt wurden.
Die Prähybridisierung und Hybridisierung erfolgte in 5 x SSC. 1 x Blockierungsreagenz, 0,1 % N-Lauroylsarcosin, 0,02 % SDS und 100 µg Sperma-DNA des Herings. Die Prähybridisierung erfolgte 30 Minuten bei 60°C, die spezifische Hybridisierung mit 20 - 40 ng/ml der digoxigenierten, denaturierten HBV-spezifischen DNA (14 Stunden, 60°C). Anschließend wurden die Filter gewaschen.

### Nachweis der HBV DNA durch Digoxigenin Antikörper

Der immunologische Nachweis der Digoxigenin markierten DNA erfolgte nach Angaben des Herstellers.
Die Filter wurden gewaschen und in einem Blockierungsreagenz (nach Angabe des Herstellers) prähybridisiert. Anschließend wurde mit einem Anti-DIG-Antikörper, der mit alkalischer Phosphatase gekoppelt war, 30 Minuten hybridisiert. Nach einem Waschschritt wurde das Substrat der alkalischen Phosphatase, CSPD, zugefügt, 5 Minuten mit den Filtern inkubiert, anschließend in Plastikfolie eingepackt und weitere 15 Minuten bei 37°C inkubiert. Die Chemilumineszenz der Hepatitis B-spezifischen DNA-Signale wurde über eine Exposition der Filter auf einem Röntgenfilm sichtbar gemacht (Inkubation je nach Signalstärke: 10 Minuten bis 2 Stunden).
Die halbmaximale Hemmkonzentration (IC-50, inhibitorische Konzentration 50 %) wurde als die Konzentration bestimmt, bei der gegenüber einer unbehandelten Probe die Hepatitis B-spezifische Bande durch die erfindungsgemäße Verbindung um 50 % reduziert wurde.

Die Behandlung der Hepatits B Virus-produzierenden HepG2.2.15-Zellen mit den erfindungsgemäßen Verbindungen führte überraschenderweise zu einer Reduktion viraler DNA im Zellkulturüberstand, die von den Zellen in Form von Virionen in den Zellkulturüberstand ausgeschleust wird.

Die erfindungsgemäßen Verbindungen zeigen ein neue, nicht vorhersehbare und wertvolle Wirkung gegen Viren. Sie sind überraschenderweise antiviral gegen Hepatitis B (HBV) wirksam und sind somit zur Behandlung von virusinduzierten Erkrankungen, insbesondere von akuten und chronisch persistenten Virusinfektionen des HBV geeignet. Ein chronische Viruserkrankung, hervorgerufen durch das HBV, kann zu unterschiedlich schweren Krankheitsbildern führen; bekanntermaßen führt die chronische Hepatitis B-Virusinfektion in vielen Fällen zur Leberzirrhose und/oder zum hepatozellulären Karzinom.

Als Indikationsgebiete für die erfindungsgemäß verwertbaren Verbindungen können beispielsweise genannt werden:

Die Behandlung von akuten und chronischen Virusinfektionen, die zu einer infektiösen Hepatitis führen können, beispielsweise die Infektionen mit Hepatitis B-Viren. Besonders bevorzugt ist die Behandlung von chronischen Hepatitis B-Infektionen und die Behandlung von akuter Hepatitis B-Virusinfektion.

Zur vorliegenden Erfindung gehören pharmazeutische Zubereitungen, die neben nicht-toxischen, inerten pharmazeutisch geeigneten Trägerstoffen eine oder mehrere Verbindungen der Tabelle A enthalten oder die aus einem oder mehreren Wirkstoffen der Tabelle A bestehen, sowie Verfahren zur Herstellung dieser Zubereitungen.

Die Wirkstoffe der Formeln (I), (Ia) und Tabelle A sollen in den oben aufgeführten pharmazeutischen Zubereitungen, vorzugsweise in einer Konzentration von etwa 0,1 bis 99,5 Gew.-%, vorzugsweise von etwa 0,5 bis 95 Gew.-% der Gesamtmischung, vorhanden sein.

Die oben aufgeführten pharmazeutischen Zubereitungen können außer den Verbindungen der Formeln (I), (Ia) und Tabelle A auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels, sowie dem Zeitraum bzw. Intervall, innerhalb welchem die Verabreichung erfolgt.

### Herstellungsbeispiele

### Beispiel 1

### 4-(2-Chlorphenyl)-2-(pyridin-2-yl)-6-methyl-1,4-dihydro-pyrimidin-5-carbonsäureethylester

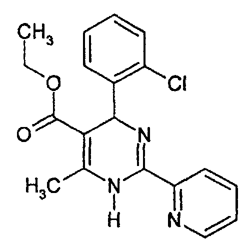

700 mg (5 mmol) 2-Chlorbenzaldehyd werden in 15 ml Isopropanol nacheinander mit 650 mg Acetessigsäureethylester, 790 mg (5 mmol) 2-Amidinopyridiniumhydrochlorid (Avocado z.B. als Anbieter) und 43,06 mg Natriumacetat versetzt und 6 Stunden gekocht. Es wird abgekühlt, eingeengt, in 40 ml 0,5 N HCl und Essigester gelöst, abgetrennt, die organische Phase mit 10 ml 1N HCl extrahiert und die vereinigten wäßrigen Phasen mit Ether gewaschen. Die wäßrige Phase wird mit verdünnter Ammoniaklösung basisch gestellt, mit Essigester extrahiert, es wird mit H₂O gewaschen, getrocknet und eingeengt. Es wird in wenig Acetonitril gelöst und auskristallisiert. Es wird abgesaugt, mit Acetonitril gewaschen, bei 60°C im Vakuum getrocknet.
DC: rein (Toluol/Essigester = 4:1)
Ausbeute: 750 mg (42 %)
Schmp.: 137-138°C

Analog der Vorschrift des Beispiels 1 werden die in Tabelle 1 aufgeführten Verbindungen hergestellt:

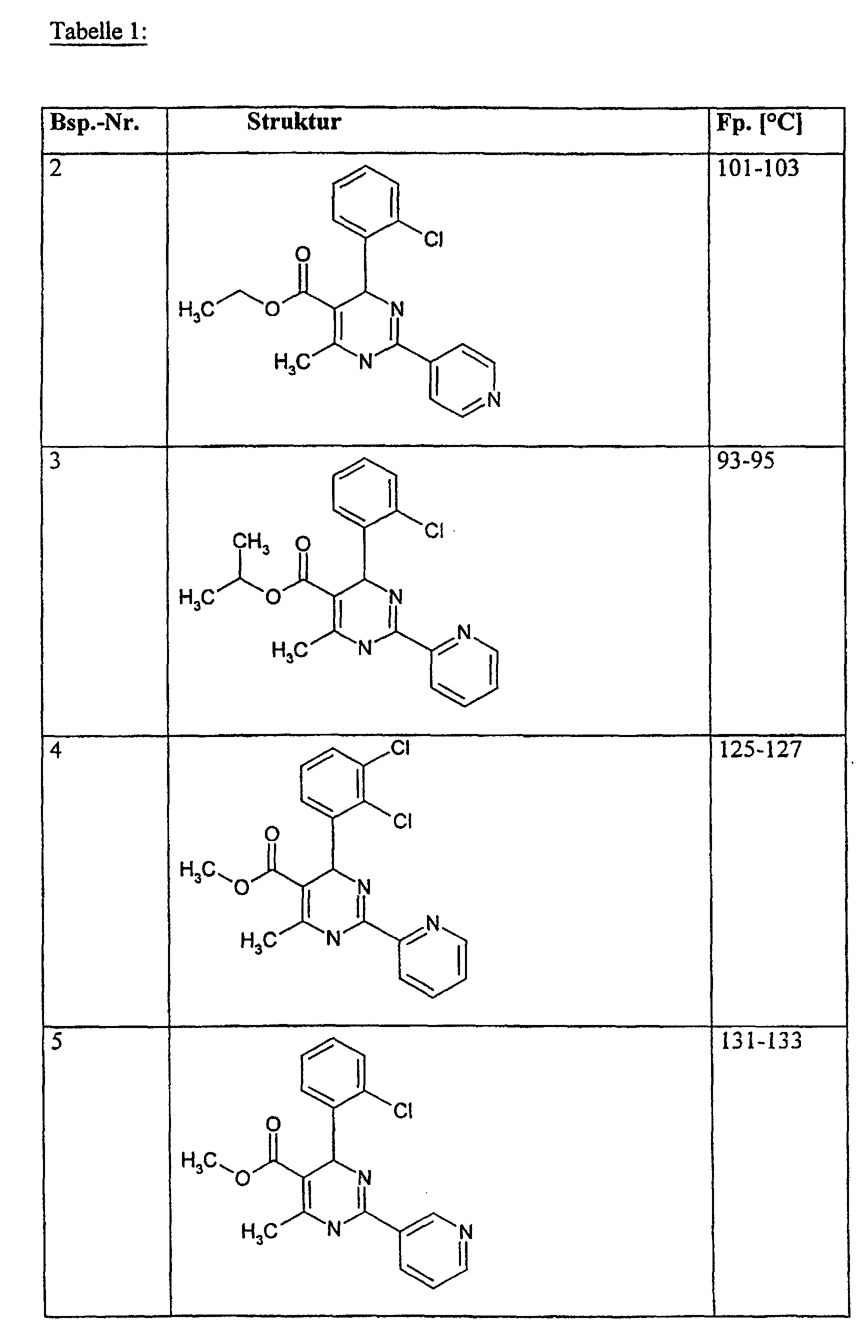

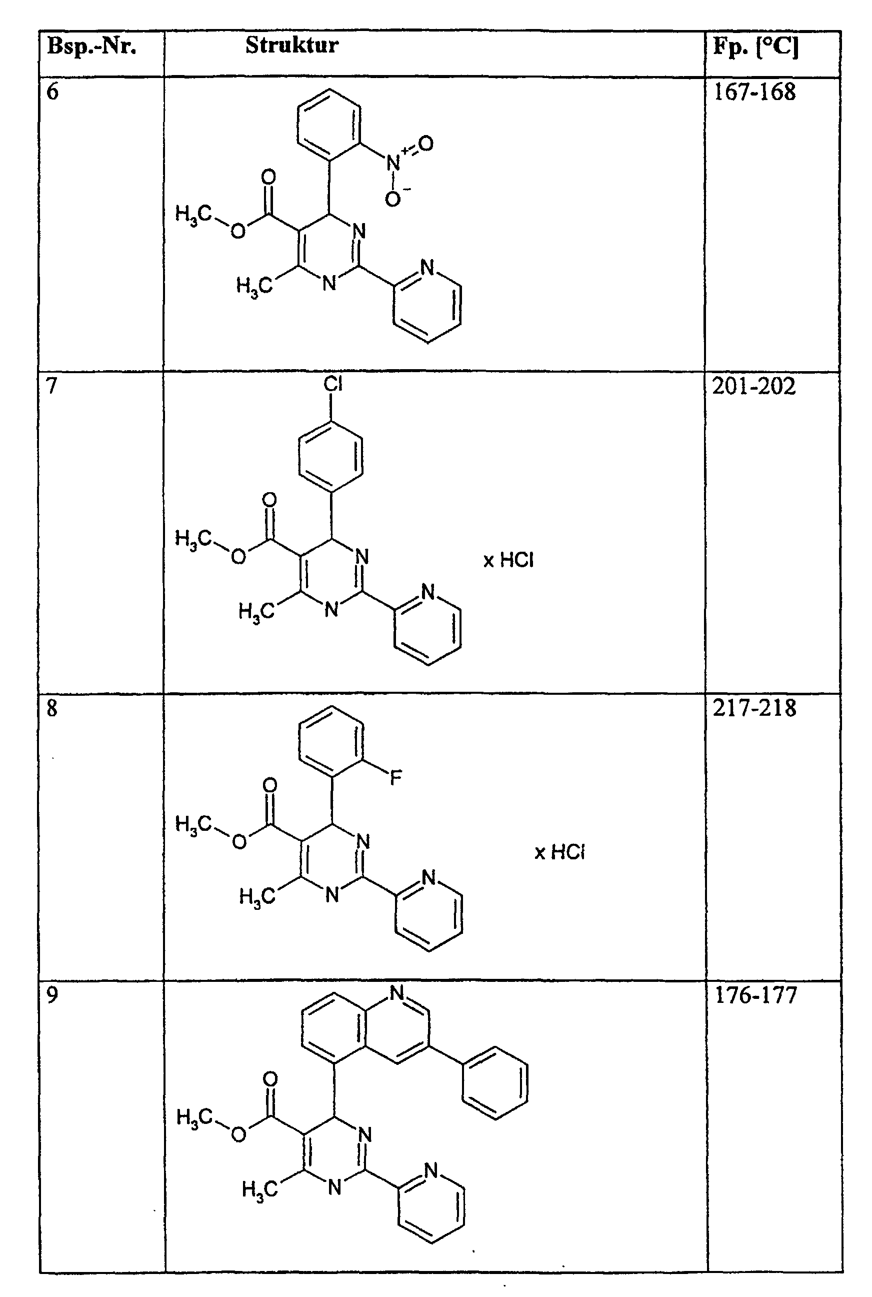

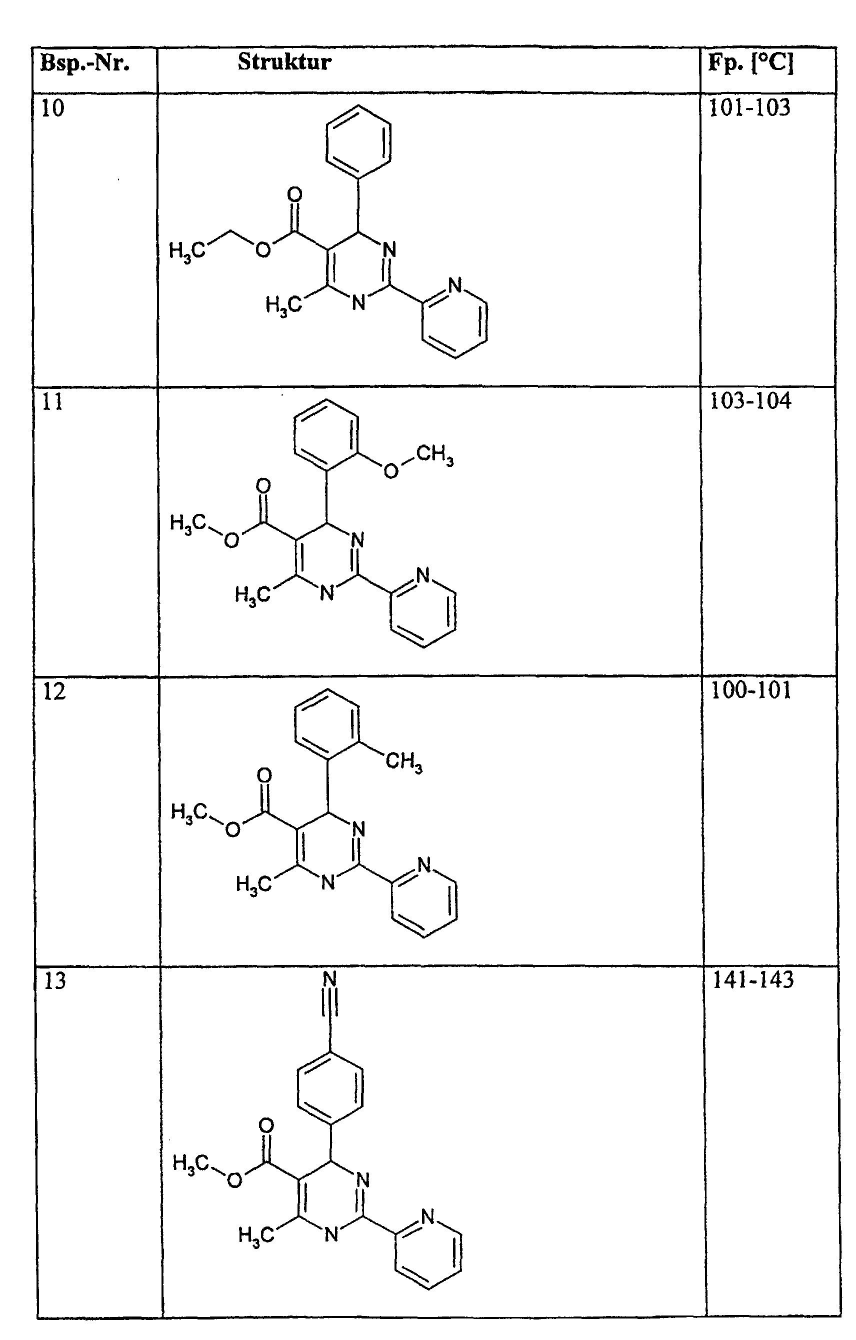

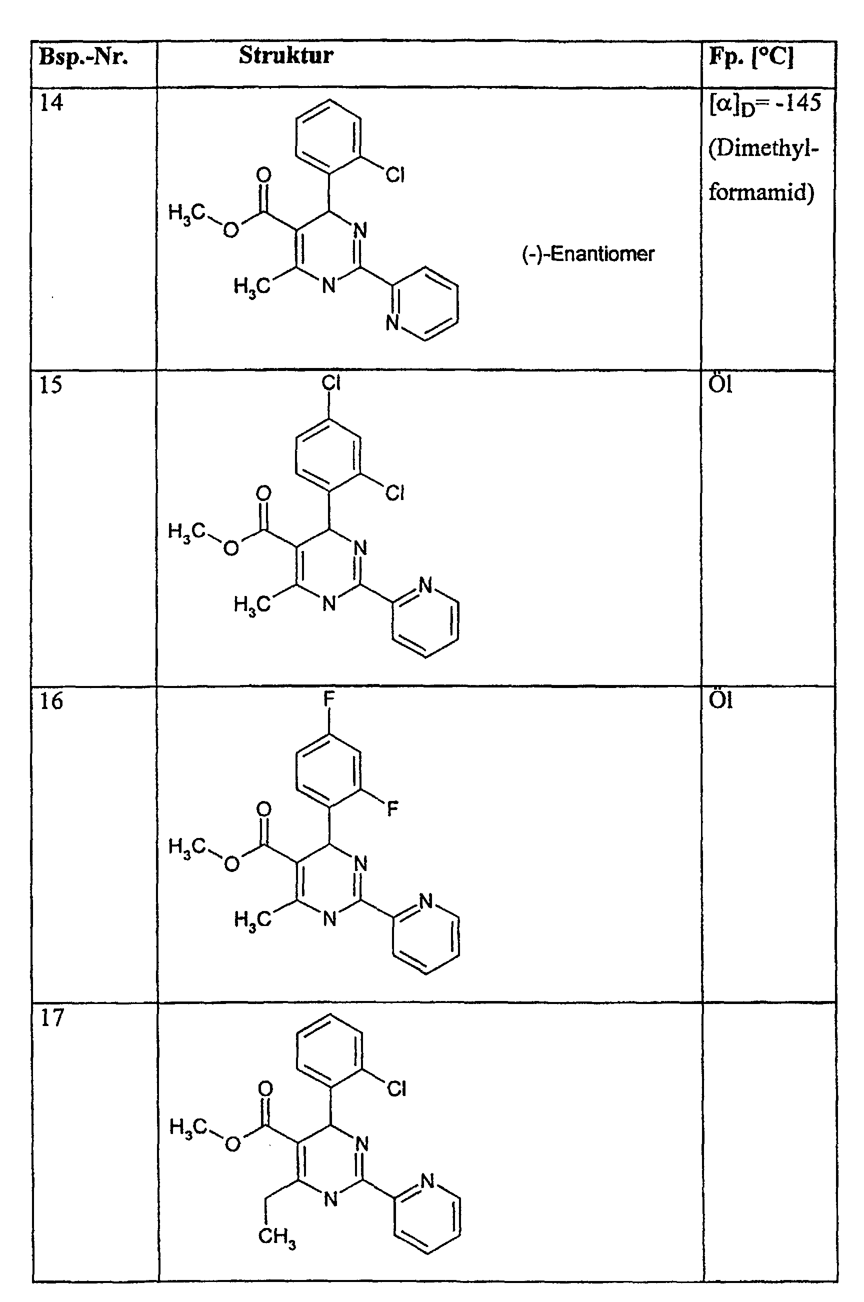

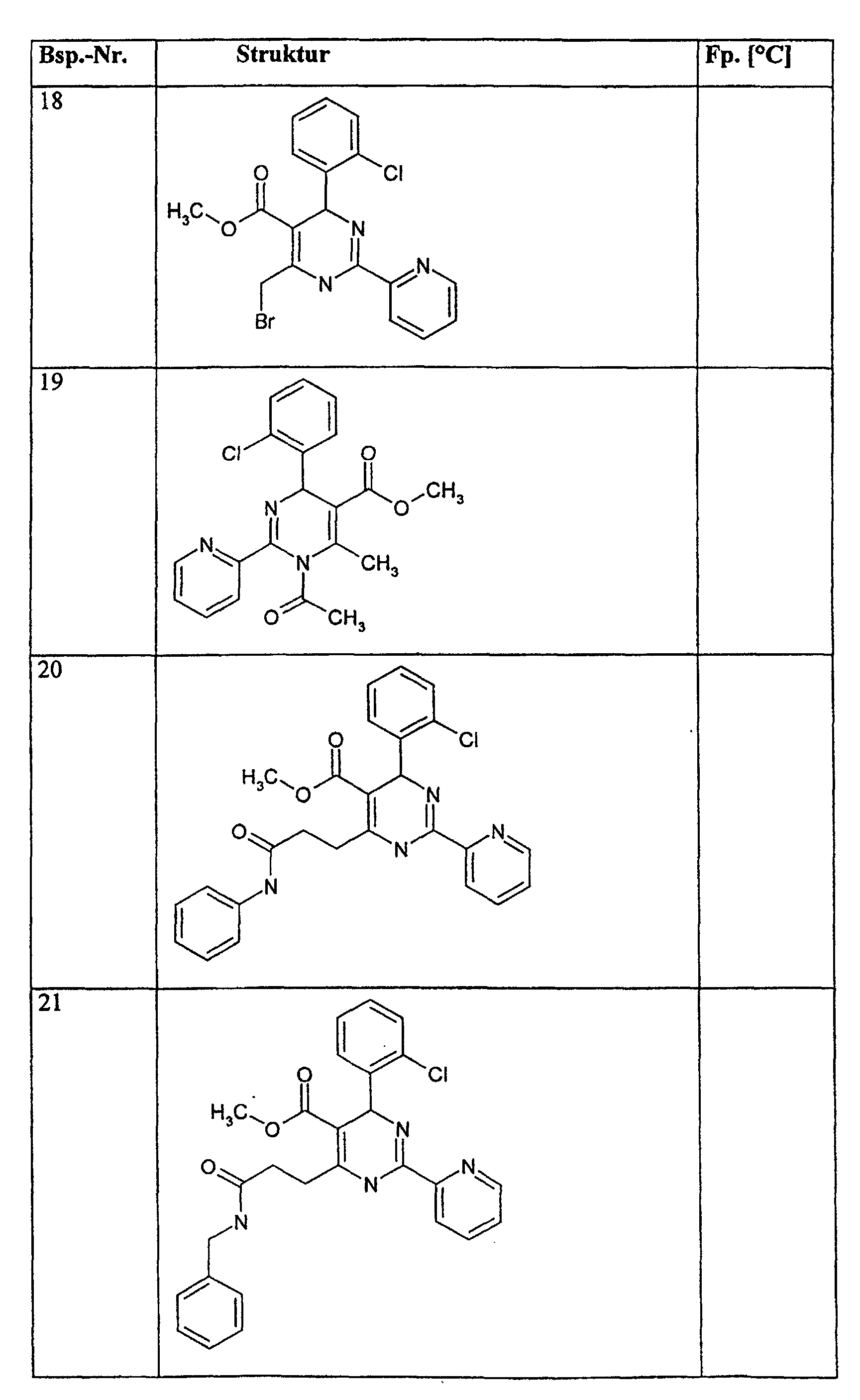

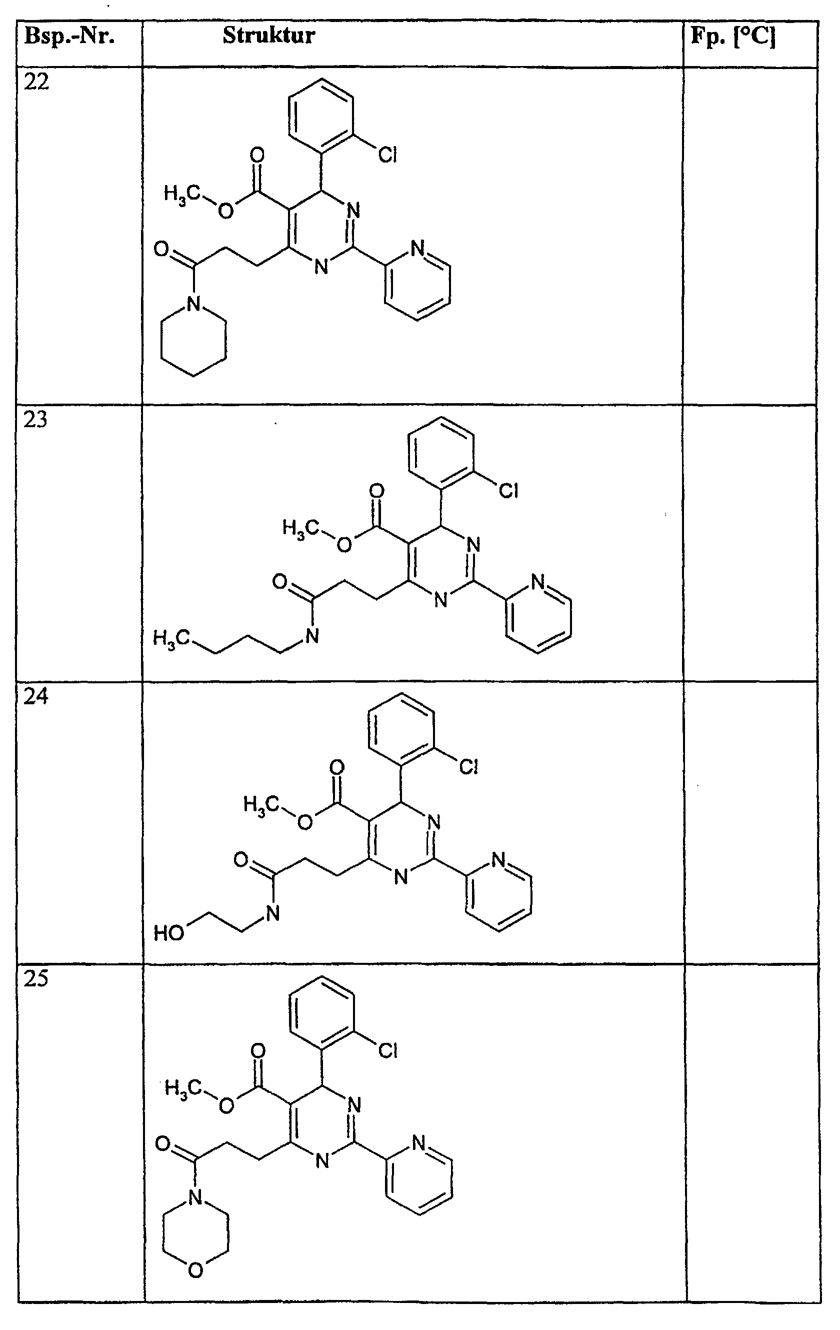

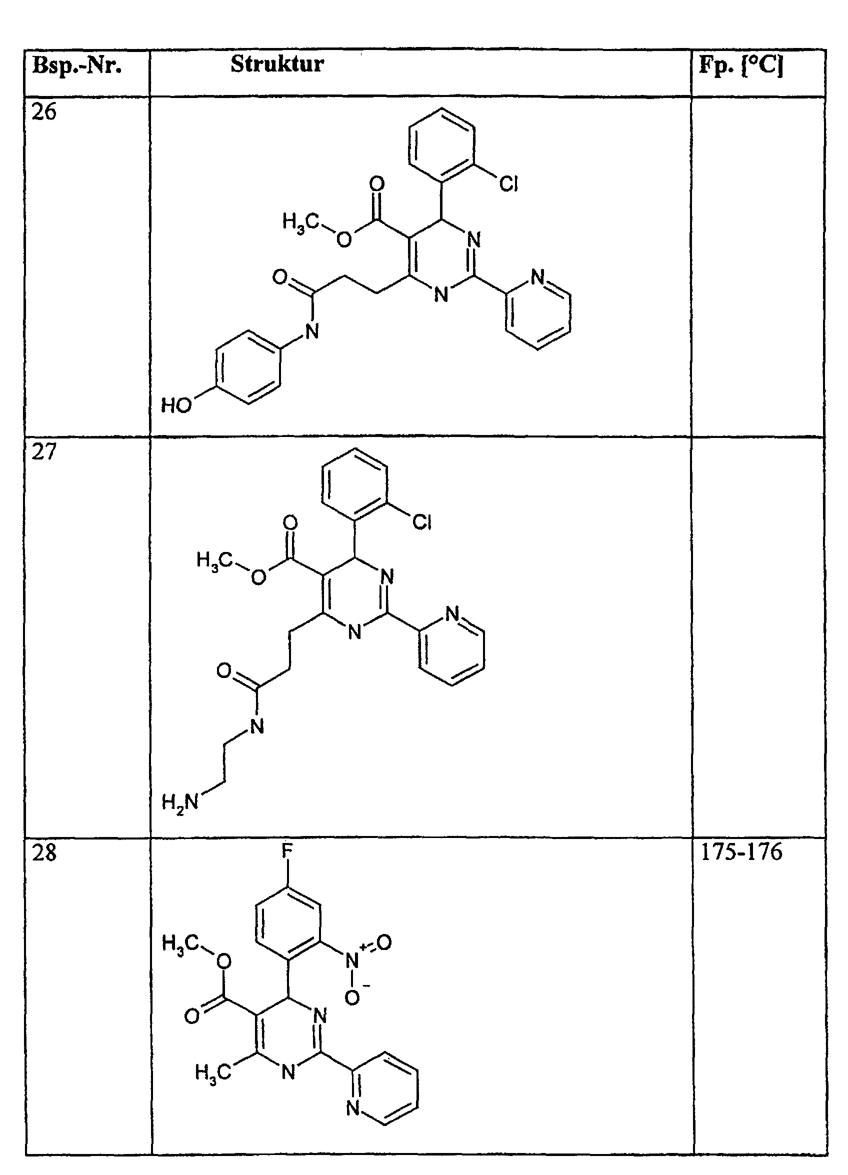

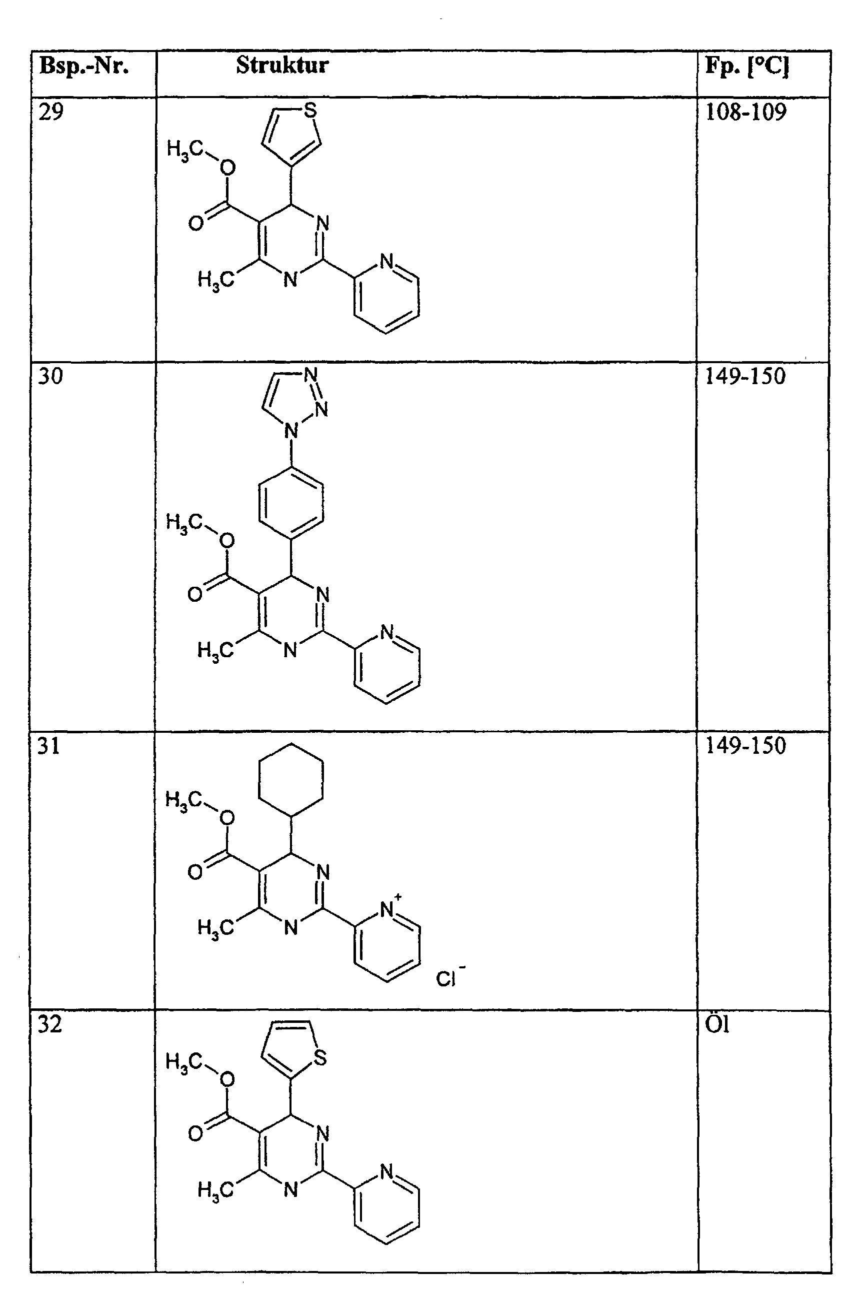

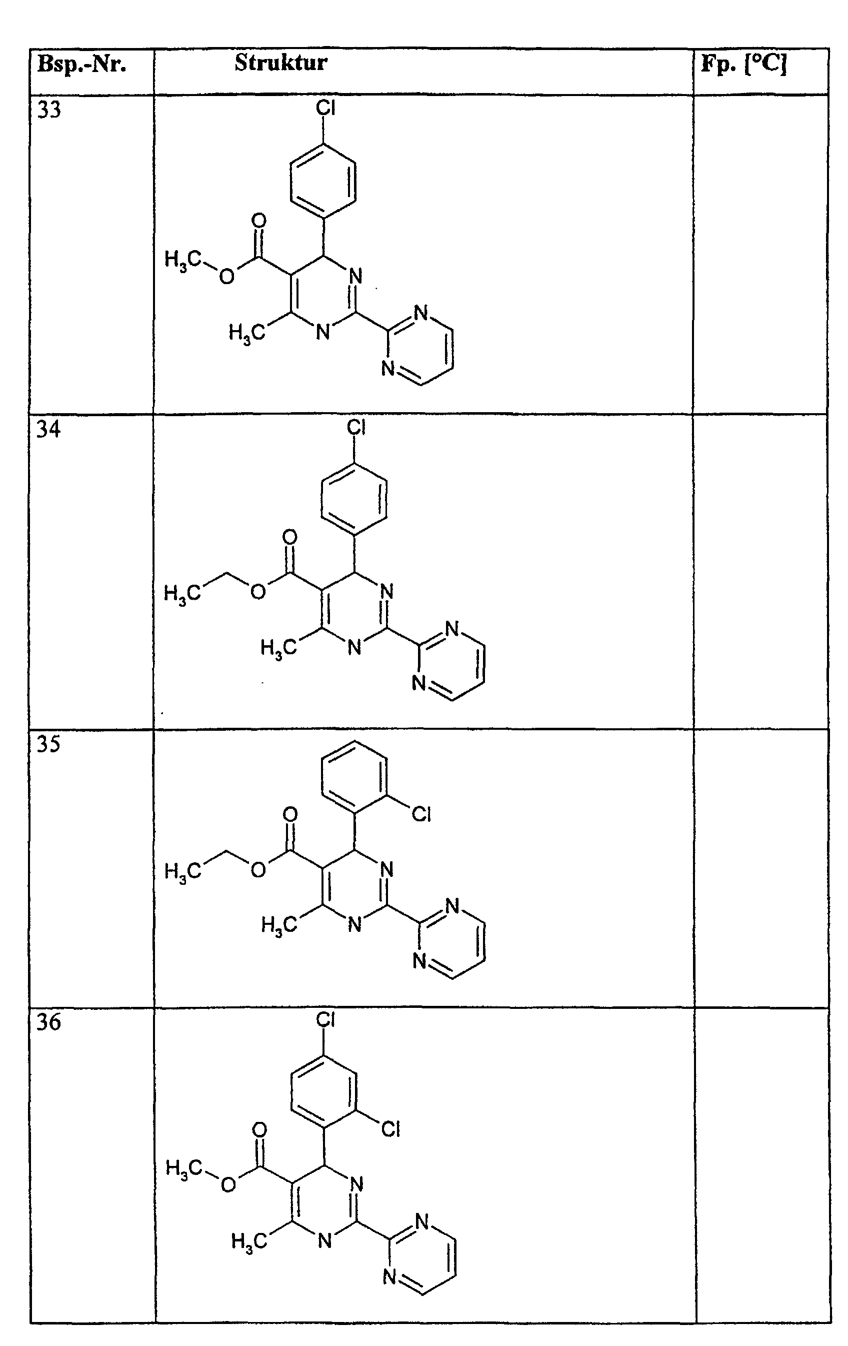

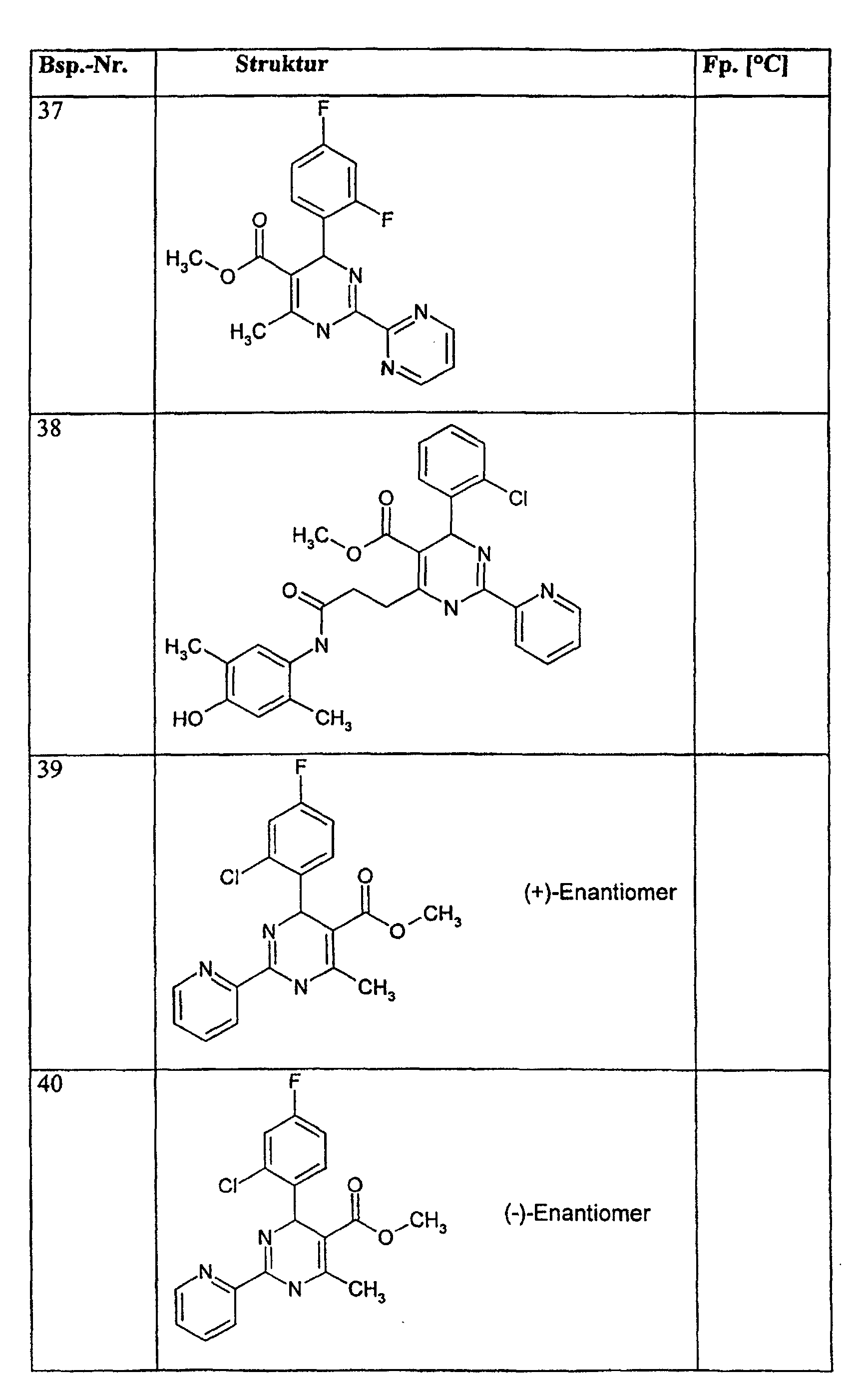

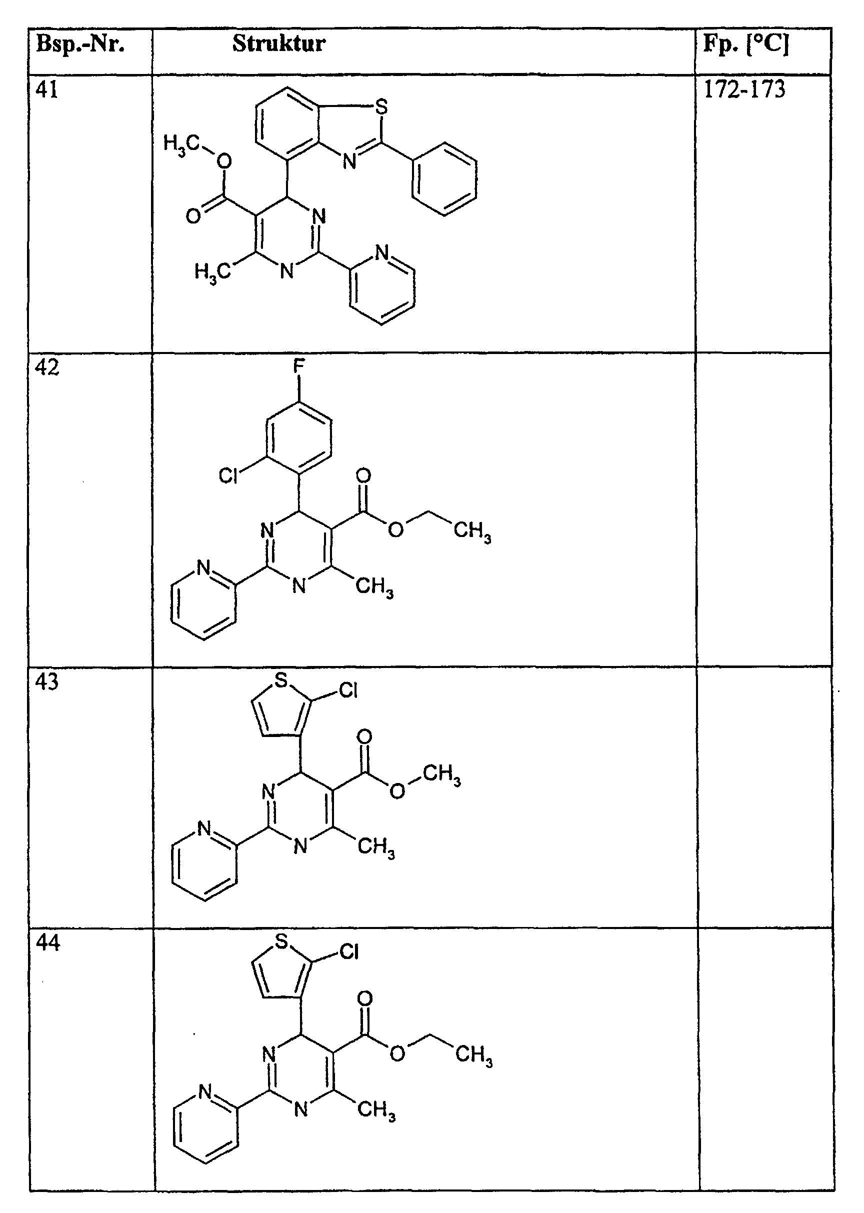

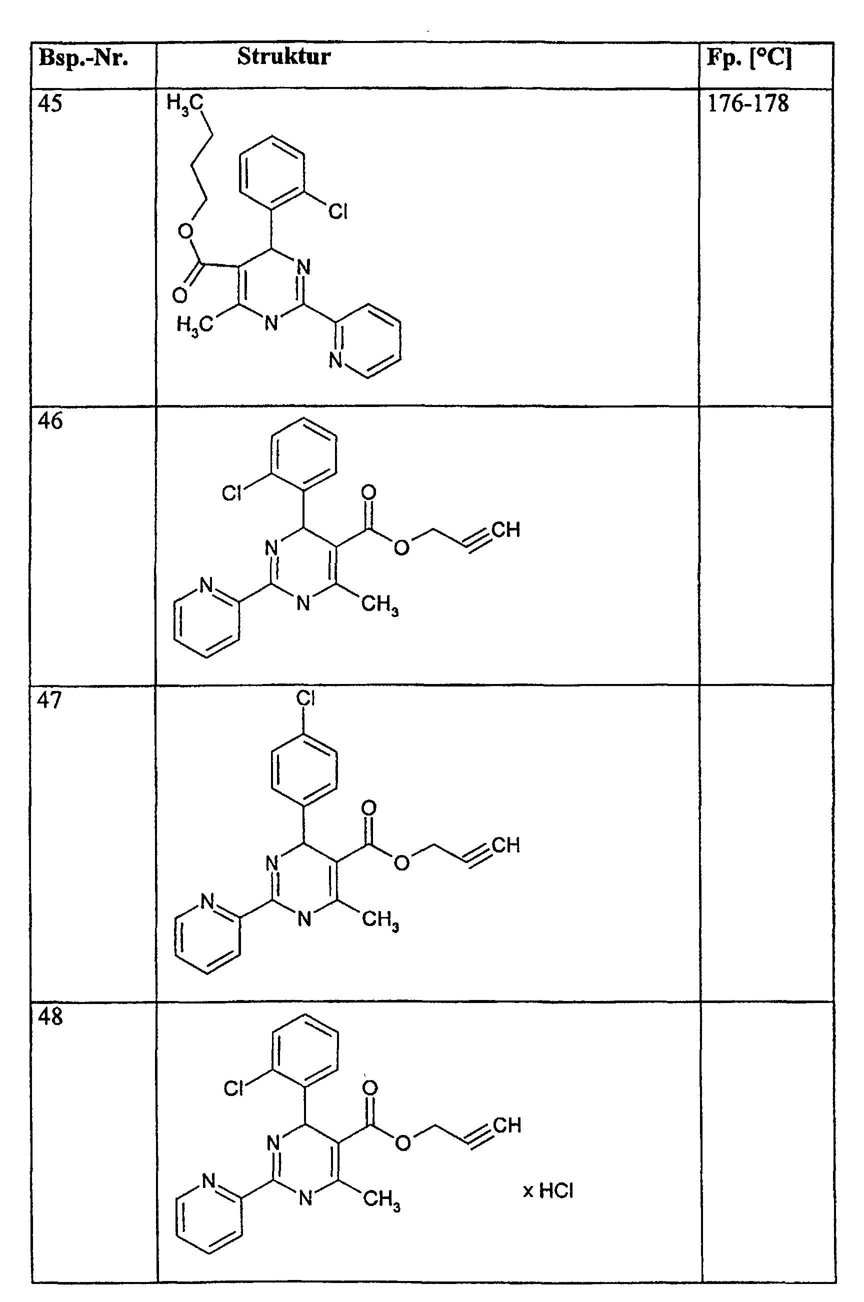

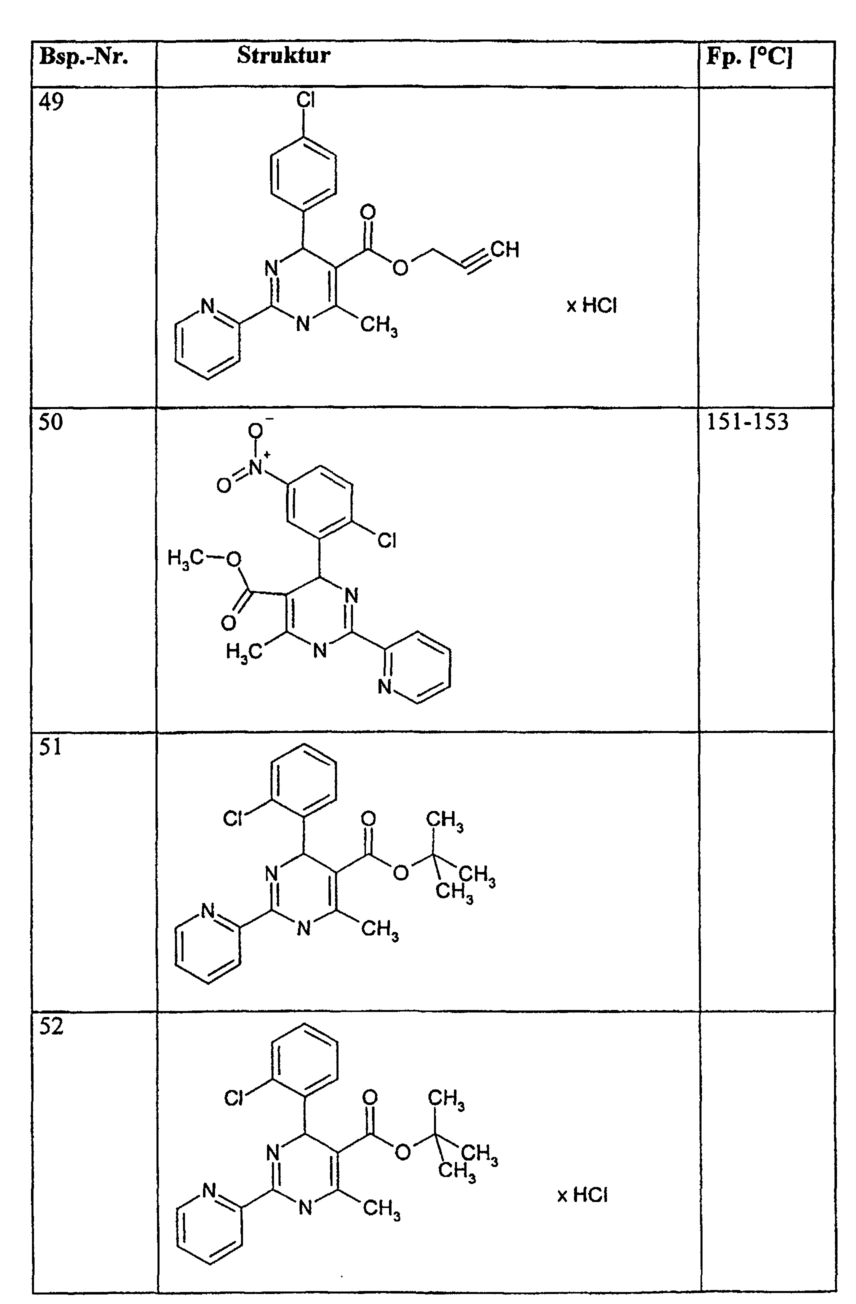

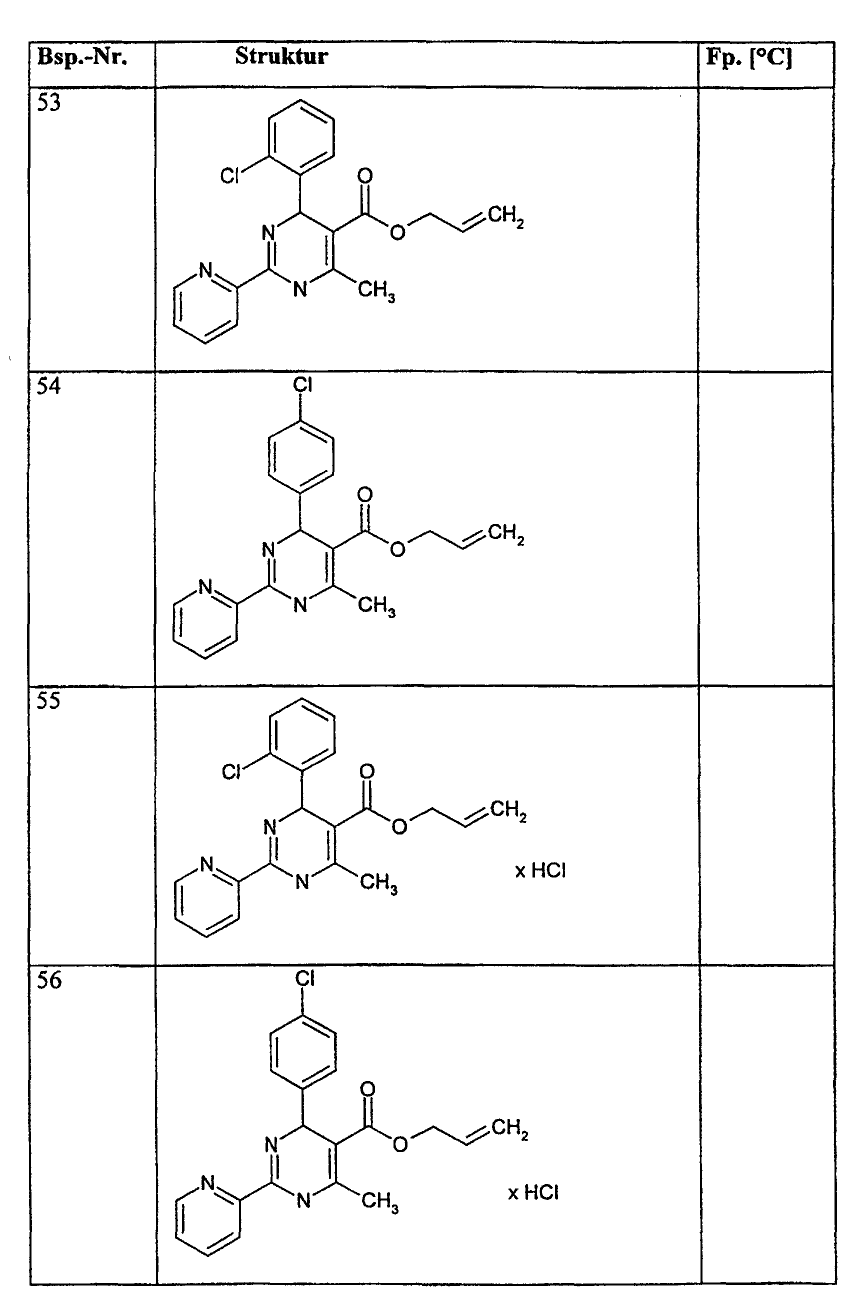

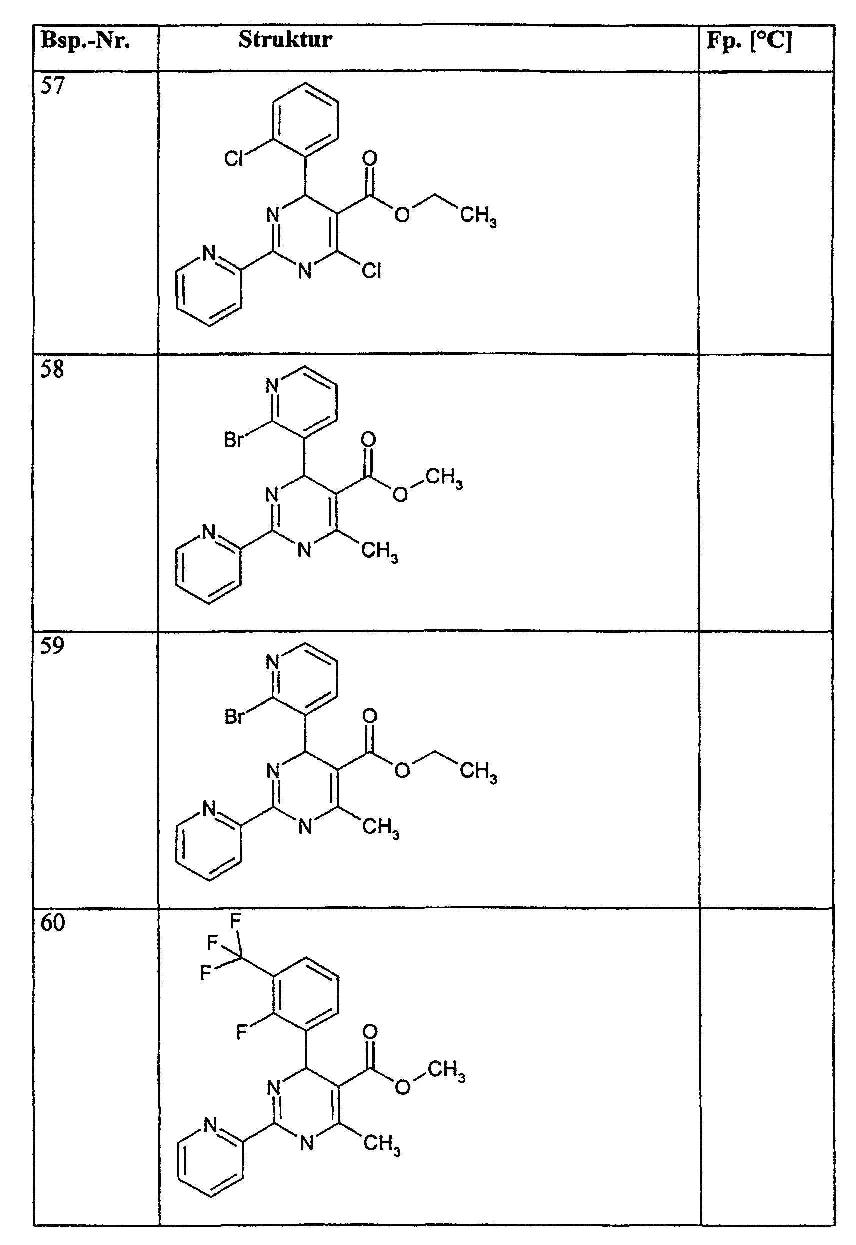

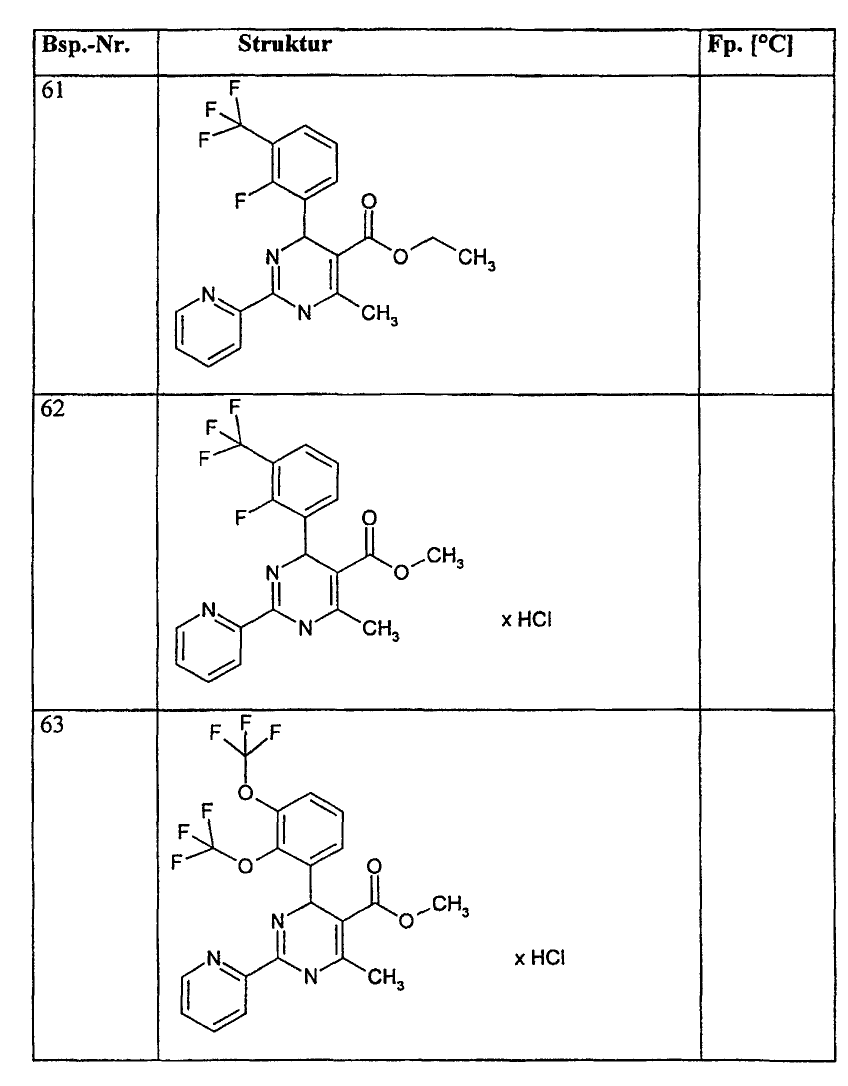

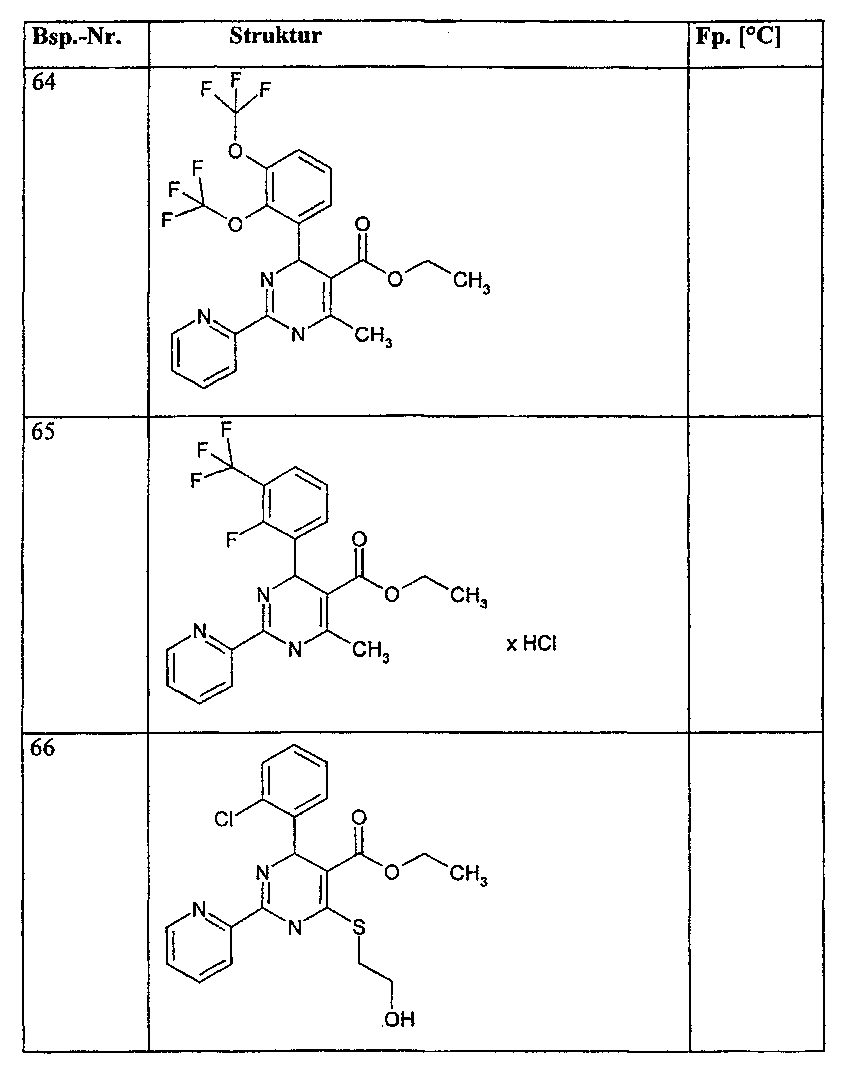

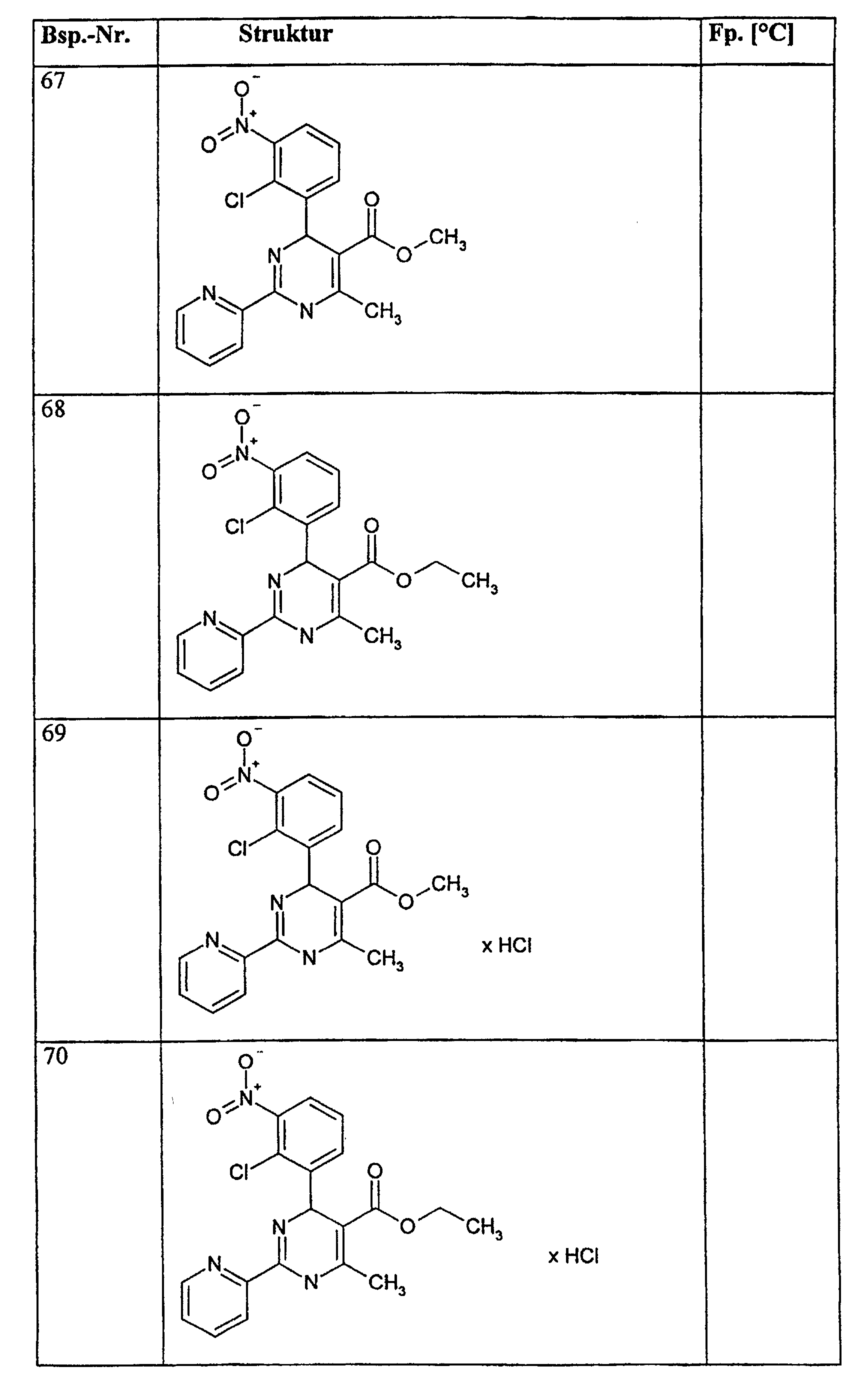

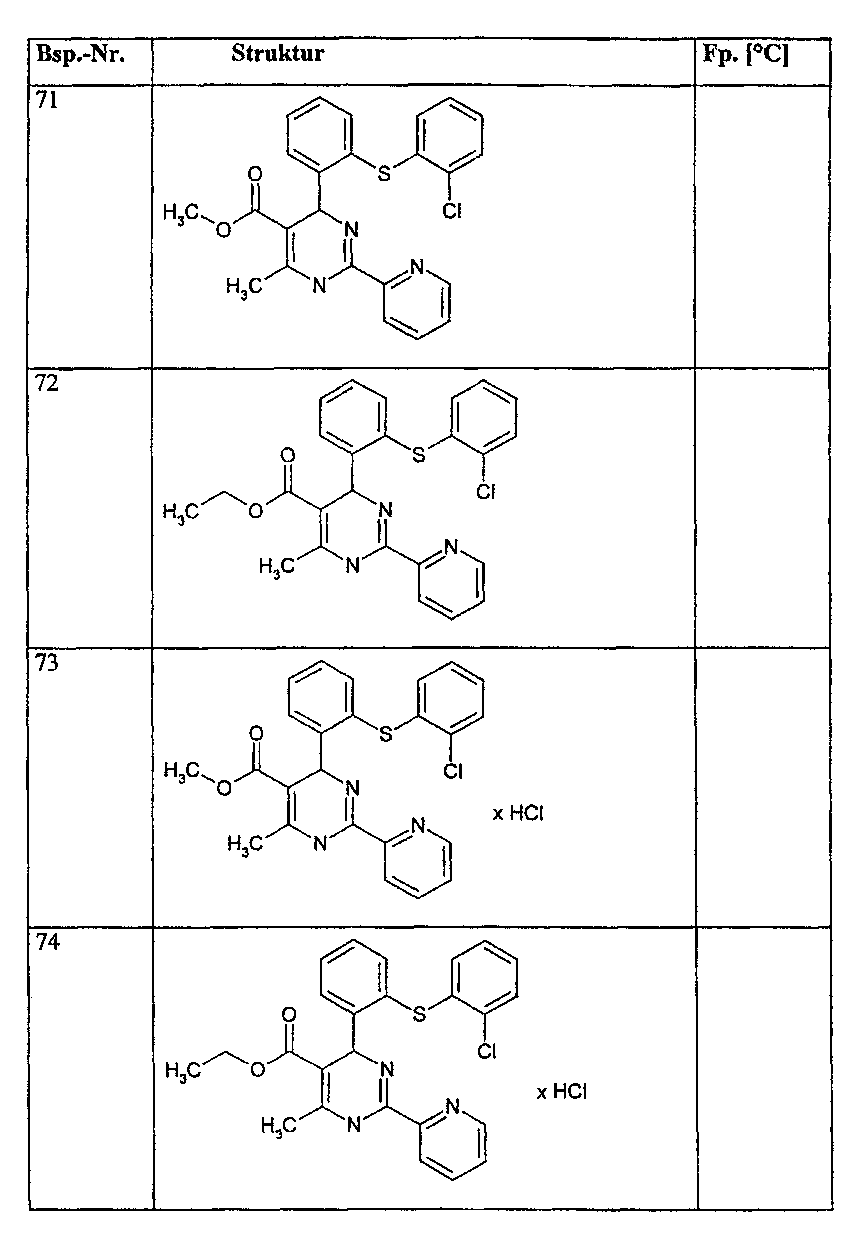

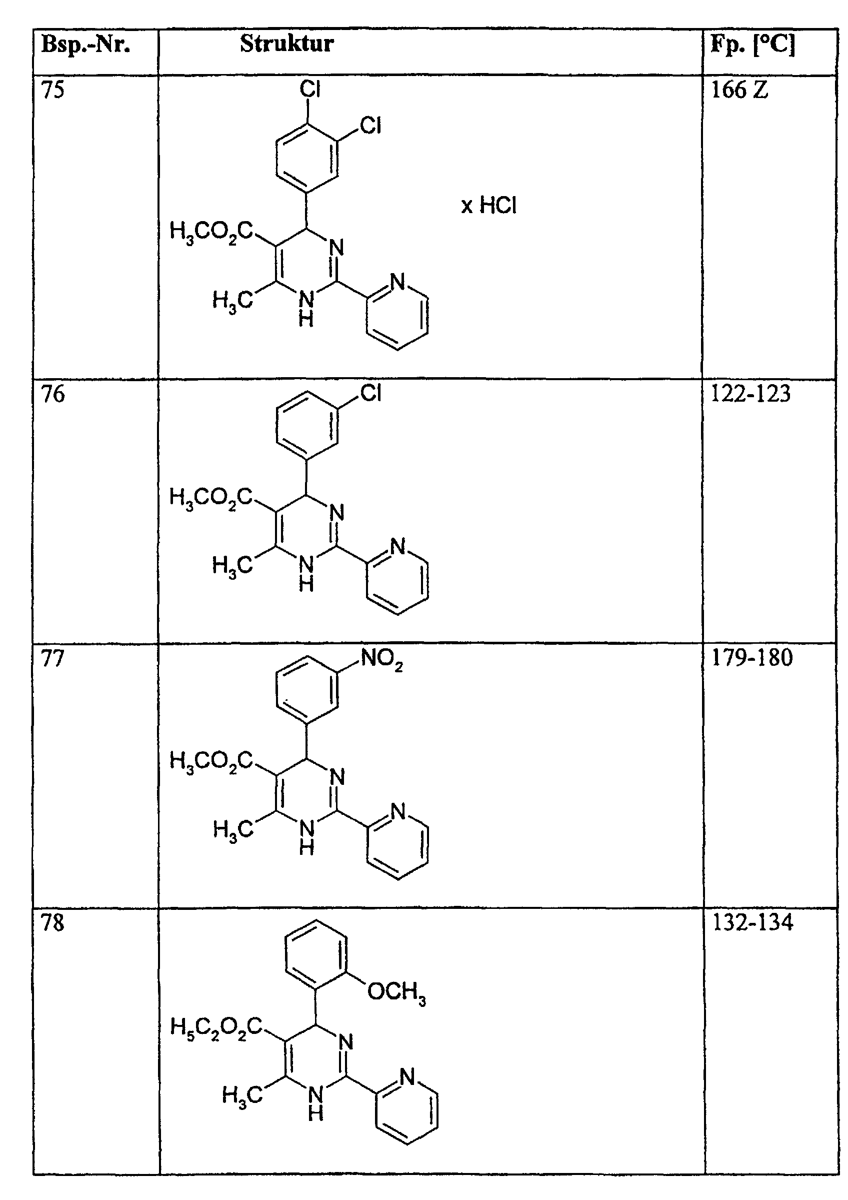

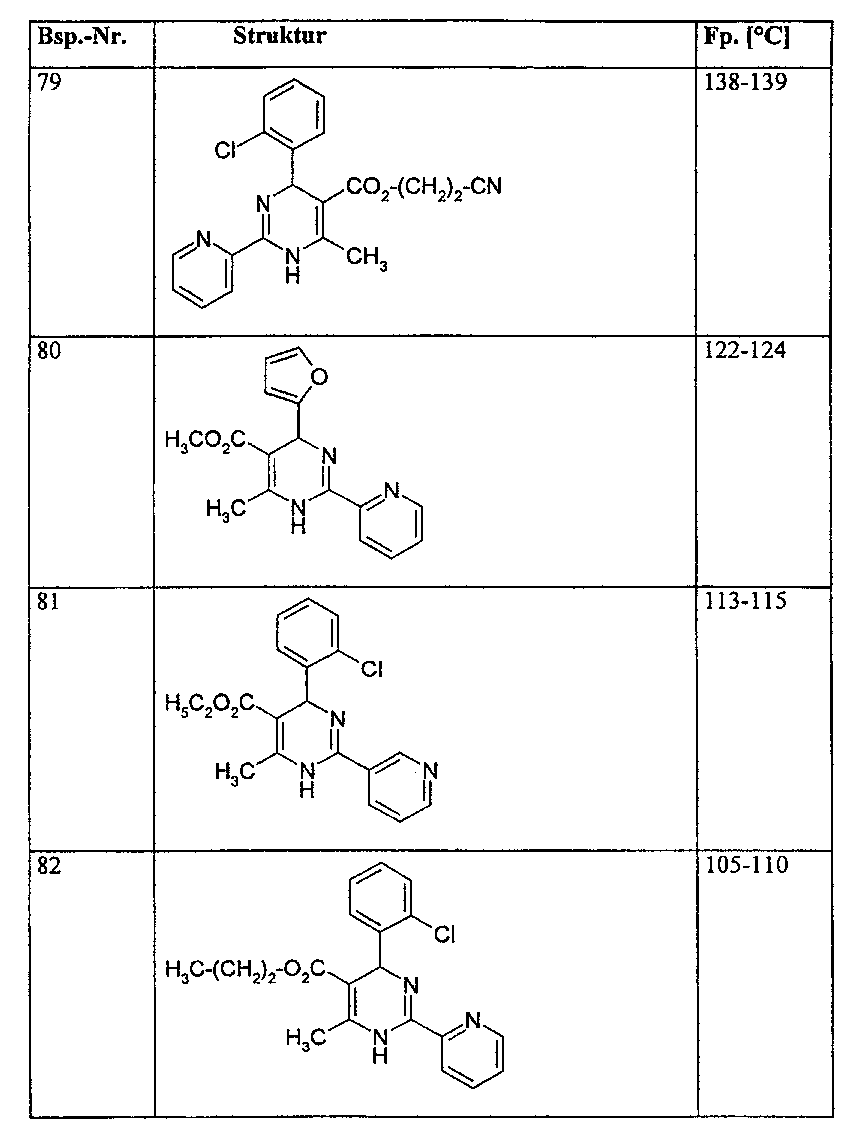

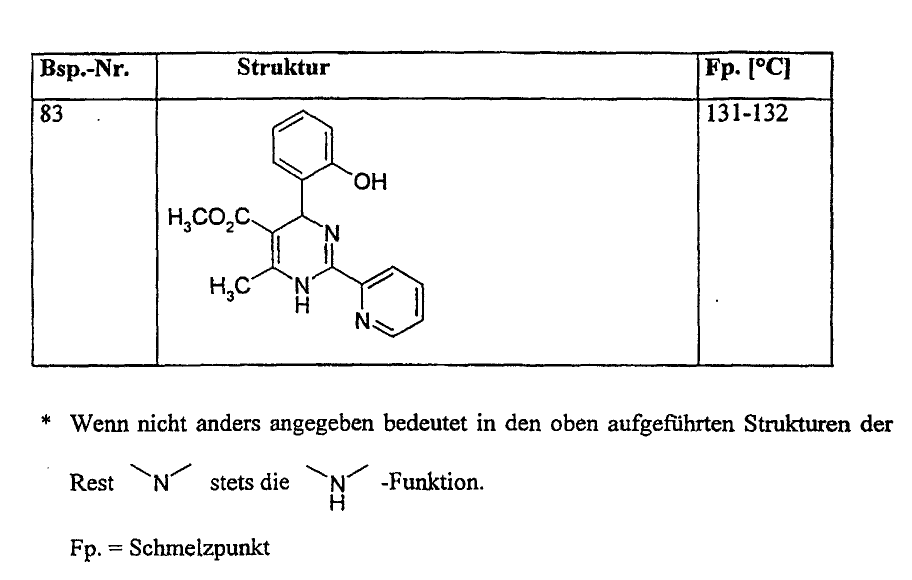

## Patentansprüche

1. Verwendung von Dihydropyrimidinen der allgemeinen Formel (I) 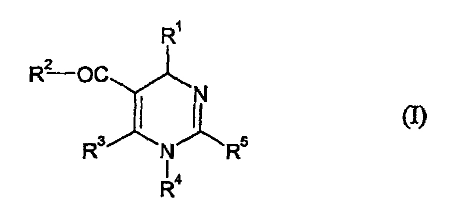 bzw. deren isomeren Form (Ia) 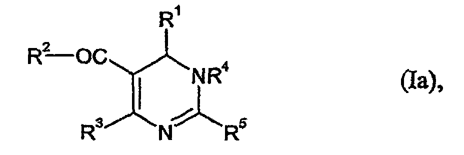 in welcher
R¹ für Phenyl, Furyl, Thienyl, Triazolyl, Pyridyl, Cycloalkyl mit 3 bis 6 Kohlenstoffatomen oder für Reste der Formeln 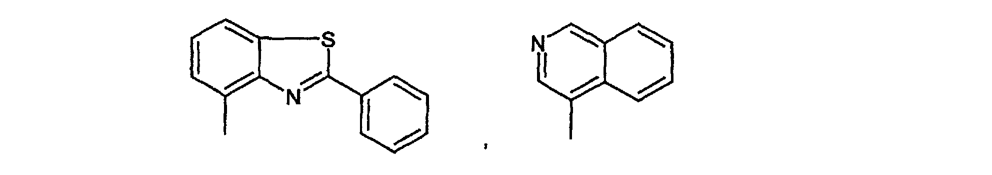 oder 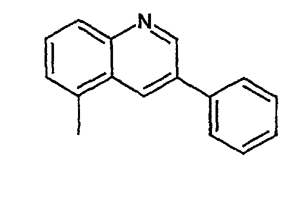 steht,
wobei die oben aufgeführten Ringsysteme gegebenenfalls einfach oder mehrfach, gleich oder verschieden durch Substituenten, ausgewählt aus der Gruppe Halogen, Trifluormethyl, Nitro, Cyano, Trifluormethoxy, Carboxyl, Hydroxyl, (C₁-C₆)-Alkoxy, (C₁-C₆)-Alkoxycarbonyl und (C₁-C₆)-Alkyl substituiert sind, das seinerseits durch Aryl mit 6 bis 10 Kohlenstoffatomen oder Halogen substituiert sein kann,
und/oder die aufgeführten Ringsysteme gegebenenfalls durch Gruppen der Formel -S-R⁶, NR⁷R⁸, CO-NR⁹R¹⁰, SO₂-CF₃ und -A-CH₂-R¹¹ substituiert sind,
worin
R⁶ Phenyl bedeutet, das gegebenenfalls durch Halogen substituiert ist,
R⁷, R⁸, R⁹ und R¹⁰ gleich oder verschieden sind und Wasserstoff, Phenyl, Hydroxy-substituiertes Phenyl, Hydroxy, (C₁-C₆)-Acyl oder (C₁-C₆)-Alkyl bedeuten, das seinerseits durch Hydroxy, (C₁-C₆)-Alkoxycarbonyl, Phenyl oder Hydroxy substituiertes Phenyl substituiert sein kann,
A einen Rest O, S, SO oder SO₂ bedeutet,
R¹¹ Phenyl bedeutet, das gegebenenfalls ein- bis mehrfach, gleich oder verschieden durch Substituenten, ausgewählt aus der Gruppe Halogen, Nitro, Trifluormethyl, (C₁-C₆)-Alkyl und (C₁-C₆)-Alkoxy substituiert ist,
R² für einen Rest der Formel -XR¹² oder -NR¹³R¹⁴ steht,
worin
X eine Bindung oder Sauerstoff bedeutet,
R¹² Wasserstoff, geradkettiges oder verzweigtes (C₁-C₆)-Alkoxycarbonyl oder einen geradkettigen, verzweigten oder cyclischen, gesättigten oder ungesättigten (C₁-C₈)-Kohlenwasserstoffrest bedeutet, der gegebenenfalls eine oder zwei gleiche oder verschiedene Heterokettenglieder aus der Gruppe O, CO, NH, -NH-(C₁-C₄)-Alkyl, -N-((C₁-C₄)-Alkyl)₂, S oder SO₂ enthält und der gegebenenfalls substituiert ist durch Halogen, Nitro, Cyano, Hydroxy, Aryl mit 6 bis 10 Kohlenstoffatomen oder Aralkyl mit 6 bis 10 Kohlenstoffatomen, Heteroaryl oder einer Gruppe der Formel -NR¹⁵R¹⁶,
worin
R¹⁵ und R¹⁶ gleich oder verschieden sind und Wasserstoff, Benzyl oder (C₁-C₆)-Alkyl bedeuten,
R¹³ und R¹⁴ gleich oder verschieden sind und Wasserstoff, (C₁-C₆)-Alkyl oder Cycloalkyl mit 3 bis 6 Kohlenstoffatomen bedeuten,
R³ für Wasserstoff, Amino oder
für einen Rest der Formel 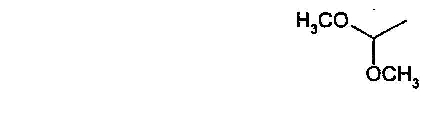 steht, oder
für Formyl, Cyano, hydroxy-substituiertes (C₁-C₆)-Alkylthio, Trifluormethyl oder Pyridyl steht, oder
für einen geradkettigen, verzweigten oder cyclischen, gesättigten oder ungesättigten Kohlenwasserstoffrest mit bis zu 8 Kohlenstoffatomen steht, der gegebenenfalls ein- oder mehrfach, gleich oder verschieden durch Aryloxy mit 6 bis 10 Kohlenstoffatomen, Azido, Halogen, Cyano, Hydroxy, Carboxyl, (C₁-C₆)-Alkoxycarbonyl, einen 5- bis 7-gliedrigen heterocyclischen Ring, (C₁-C₆)-Alkylthio oder (C₁-C₆)-Alkoxy substituiert ist, das seinerseits durch Azido oder Amino substituiert sein kann,
und/oder durch Triazolyl substituiert ist, das seinerseits bis zu 3-fach durch (C₁-C₆)-Alkoxycarbonyl substituiert sein kann,
und/oder durch Gruppen der Formeln -OSO₂-CH₃ oder (CO)ₐ-NR¹⁷R¹⁸ substiutiert sein kann,
worin
a eine Zahl 0 oder 1 bedeutet,
R¹⁷ und R¹⁸ gleich oder verschieden sind und Wasserstoff oder Aryl, Aralkyl mit 6 bis 10 Kohlenstoffatomen bedeuten,
oder (C₁-C₆)-Alkyl bedeuten, das gegebenenfalls durch (C₁-C₆)-Alkoxycarbonyl, Amino, Hydroxyl, Phenyl oder Benzyl substituiert ist, wobei Phenyl oder Benzyl gegebenenfalls ein- oder mehrfach, gleich oder verschieden durch Hydroxy, Carboxyl, (C₁-C₆)-Alkyl oder (C₁-C₆)-Alkoxy substituiert sind, oder (C₁-C₆)-Alkyl gegebenenfalls durch Gruppen der Formeln NH-CO-CH₃ oder NH-CO-CF₃ substituiert ist,
oder
R¹⁷ und R¹⁸ gemeinsam mit dem Stickstoffatom einen Morpholin-, Piperidinyl- oder Pyrrolidinylring bilden,
oder
R³ für Phenyl steht, das gegebenenfalls durch Methoxy substituiert ist,
oder
R² und R³ gemeinsam einen Rest der Formel 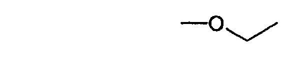 bilden,
R⁴ für Wasserstoff, (C₁-C₄)-Alkyl, (C₂-C₄)-Alkenyl, Benzoyl oder für Acyl mit 2 bis 6 Kohlenstoffatomen steht,
R⁴ für Wasserstoff, Methyl, Benzoyl oder für (C₂-C₆)-Acyl steht,
R⁵ für Pyridyl, Pyrimidyl oder Pyrazinyl steht,
sowie deren Salze zur Herstellung von Arzneimitteln zur Behandlung und Prophylaxe von Hepatitis B.

2. Verwendung nach Anspruch 1, wobei
R¹ für Phenyl, Furyl, Thienyl, Triazolyl, Pyridyl, Cyclopentyl oder Cyclohexyl oder fiir Reste der Formeln 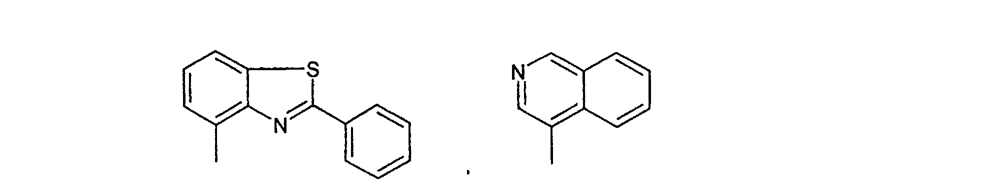 oder 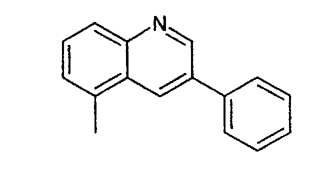 steht,
wobei die oben aufgeführten Ringsysteme gegebenenfalls ein- oder 2-fach gleich oder verschieden durch Substituenten, ausgewählt aus der Gruppe Halogen, Trifluormethyl, Nitro, SO₂-CF₃, Methyl, Cyano, Trifluormethoxy, Hydroxy, Carboxyl, Methoxycarbonyl oder Resten der Formel -CO-NH-CH₂-C(CH₃)₃, -CO-NH(CH₂)₂OH, -CO-NH-CH₂-C₆H₅, -CO-NH-C₆H₅, -CO-NH-(pOH)-C₆H₄, -O-CH₂-C₆H₅ oder -SpCl-C₆H₄ substituiert sind,
R² für einen Rest der Formel -XR¹² oder -NR¹³R¹⁴ steht,
worin
X eine Bindung oder ein Sauerstoffatom bedeutet,
R¹² Wasserstoff, (C₁-C₄)-Alkenyl, (C₁-C₄)-Alkoxycarbonyl oder (C₁-C₄)-Alkyl bedeutet, das gegebenenfalls durch Pyridyl, Cyano, Phenoxy, Benzyl oder durch einen Reste der Formel -NR¹⁵R¹⁶ substituiert sind,
worin
R¹⁵ und R¹⁶ gleich oder verschieden sind und Wasserstoff, Benzyl oder (C₁-C₄)-Alkyl bedeuten,
R¹³ und R¹⁴ gleich oder verschieden sind und Wasserstoff, (C₁-C₄)-Alkyl oder Cyclopropyl bedeuten,
R³ für Wasserstoff, Amino oder einen Rest der Formel 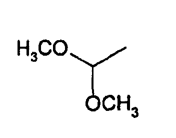 steht,
oder
für Formyl, Cyano, hydroxy-substituiertes (C₁-C₄)-Alkylthio, Trifluormethyl, Cyclopropyl oder Pyridyl steht,
oder für (C₁-C₄)-Alkyl steht, das gegebenenfalls durch Halogen, (C₁-C₄)-Alkoxycarbonyl, Hydroxy oder durch Triazolyl substituiert ist, das seinerseits bis zu 3-fach durch (C₁-C₄)-Alkoxycarbonyl substituiert sein kann,
und/oder Alkyl gegebenenfalls durch Gruppen der Formeln -OSO₂-CH₃ oder (CO)ₐ-NR¹⁷R¹⁸ substituiert ist,
worin
a eine Zahl 0 oder 1 bedeutet,
R¹⁷ und R¹⁸ gleich oder verschieden sind und Wasserstoff, Phenyl oder Benzyl bedeuten, oder
C₁-C₄-Alkyl bedeuten, das gegebenenfalls durch (C₁-C₄)-Alkoxycarbonyl, Amino, Hydroxyl, Phenyl oder Benzyl substituiert ist, wobei Phenyl oder Benzyl gegebenenfalls ein- oder mehrfach gleich oder verschieden durch Hydroxy, Carboxy, (C₁-C₄)-Alkyl oder (C₁-C₄)-Alkoxy substituiert sind, und/oder (C₁-C₄)-Alkyl gegebenenfalls durch Reste der Formeln -NH-CO-CH₃ oder -NH-CO-CF₃ substituiert ist,
oder
R¹⁷ und R¹⁸ gemeinsam mit dem Stickstoffatom einen Morpholin-, Piperidinyl- oder Pyrrolidinylring bilden,
oder
R³ für Phenyl steht, das gegebenenfalls durch Methoxy substiuiert ist,
oder
R² und R³ gemeinsam einen Rest der Formel 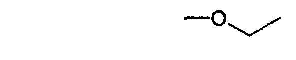 bilden,
R⁴ für Wasserstoff, Methyl, Vinyl oder Acetyl steht,
R⁵ für Pyridyl, Pyrimidyl oder Pyrazinyl steht.

3. Verwendung nach einem der Ansprüche 1 oder 2, wobei
R¹ für Phenyl oder Triazolyl steht, die gegebenenfalls bis zu 2-fach gleich oder verschieden durch Fluor, Chlor, Brom oder Jod substituiert sind,
R² für geradkettiges oder verzweigtes Alkoxycarbonyl mit bis zu 4 Kohlenstoffatomen steht,
R³ für Methyl, Ethyl oder Cyclopropyl steht,
oder
R² und R³ gemeinsam einen Rest der Formel 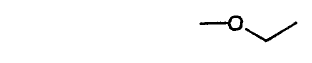 bilden,
R⁴ für Wasserstoff, Vinyl oder Acetyl steht,
und
R⁵ für Pyridyl steht.

4. Dihydropyrimidine der folgenden Strukturen 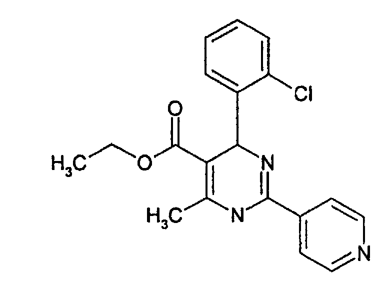 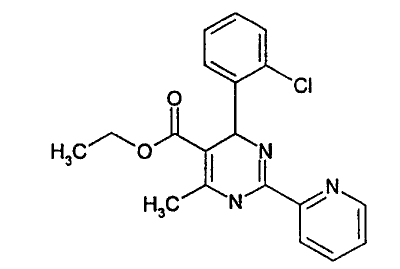 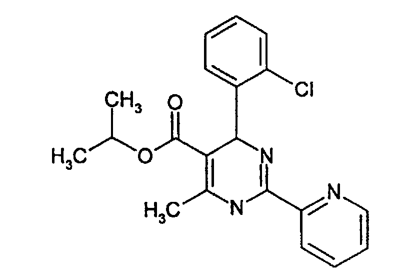 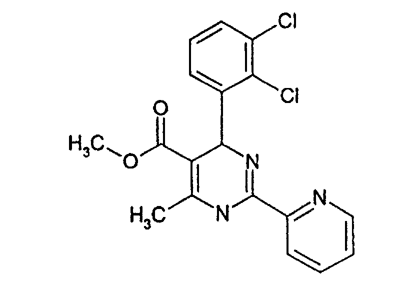 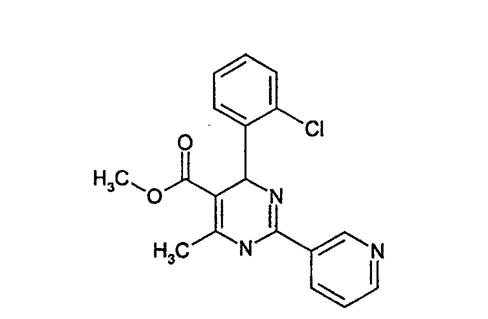 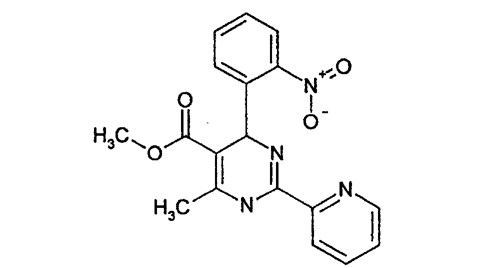 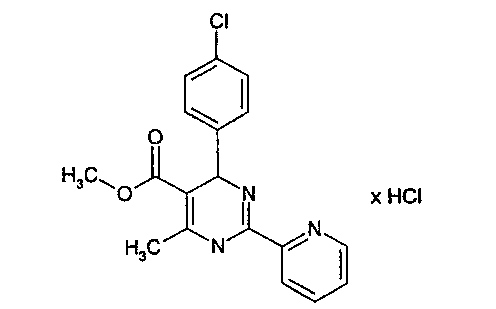 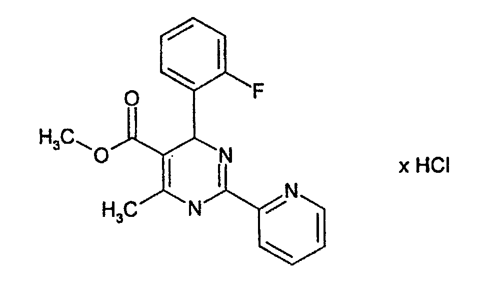 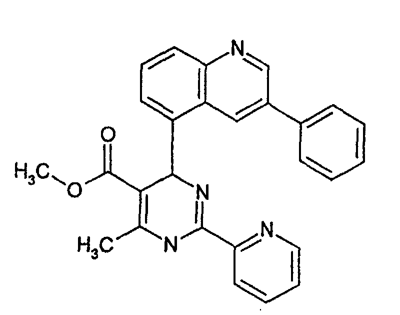 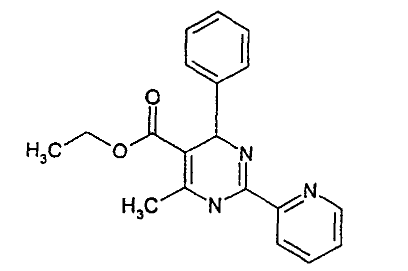 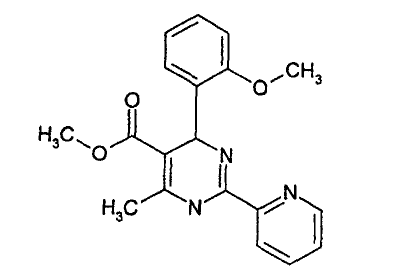 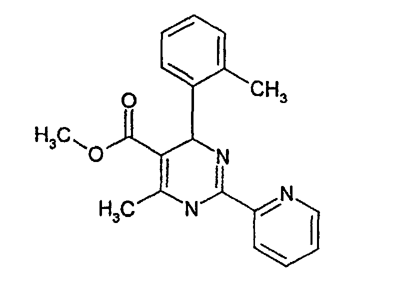 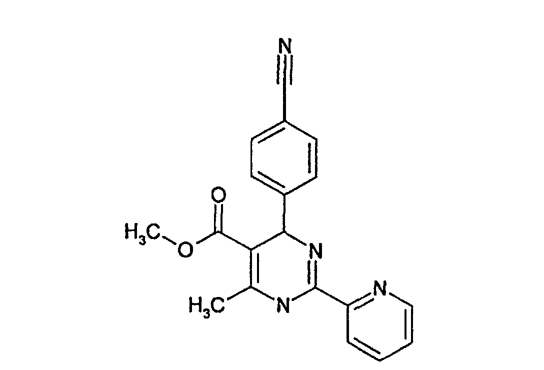 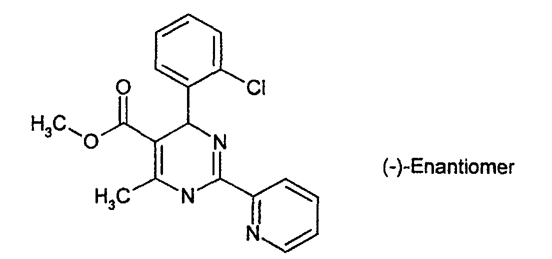 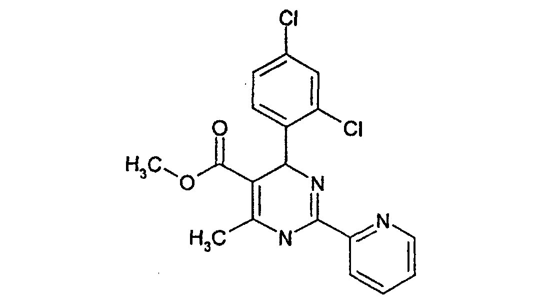 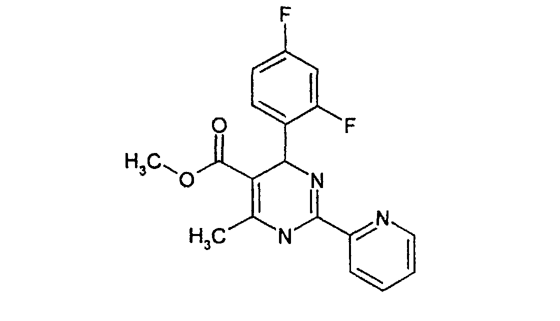 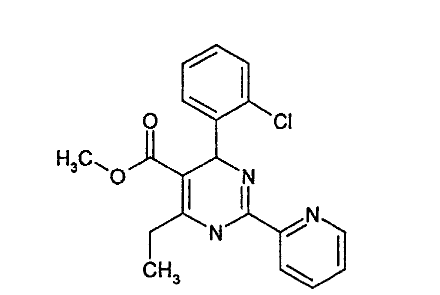 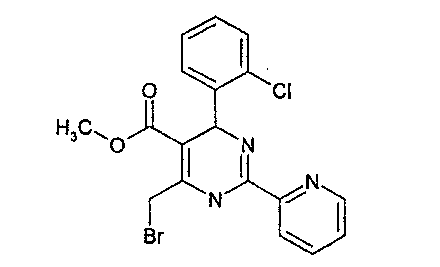 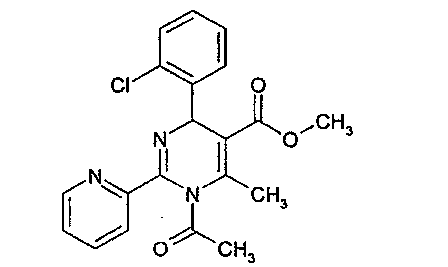 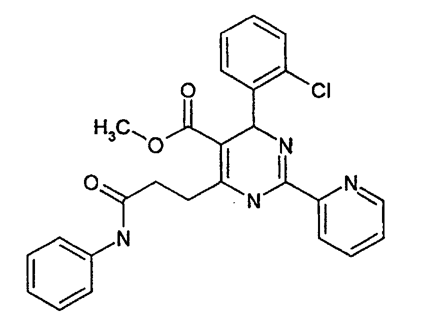 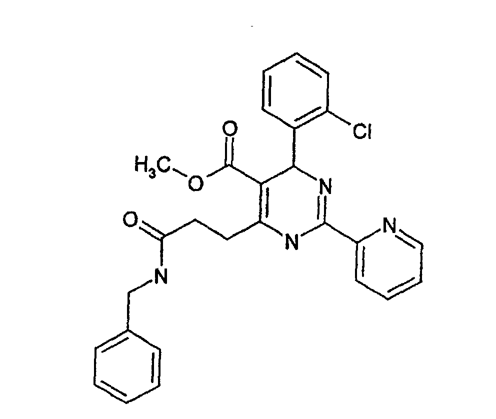 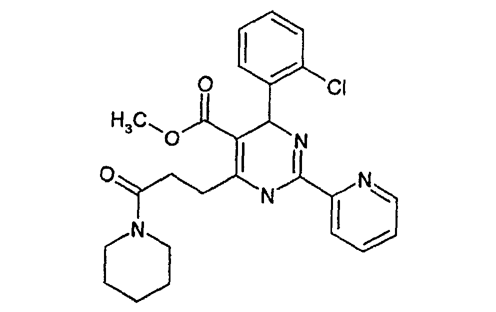 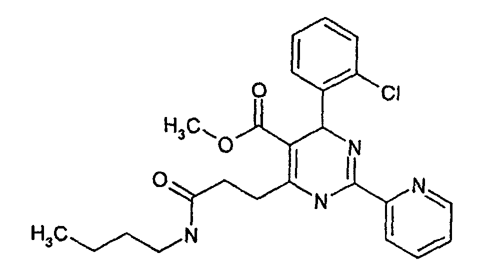 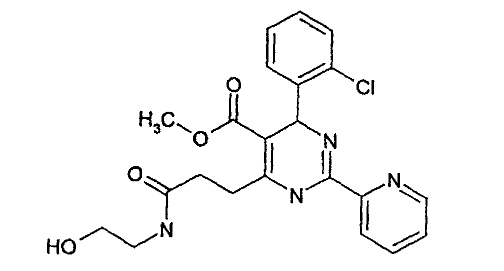 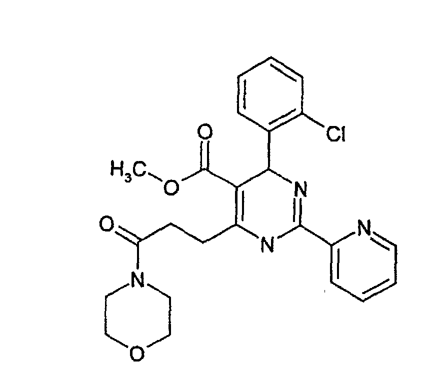 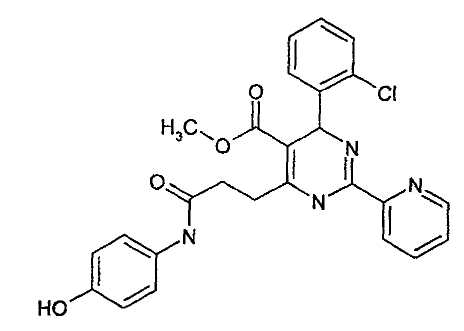 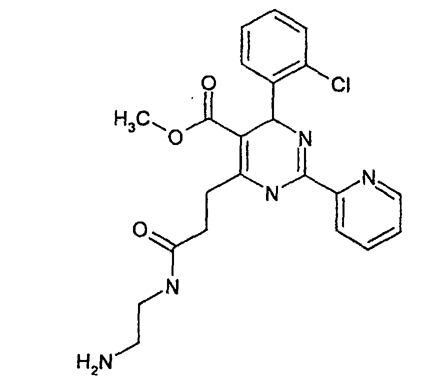 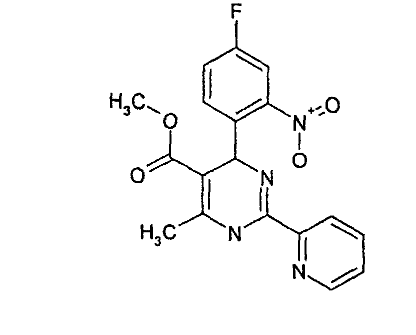 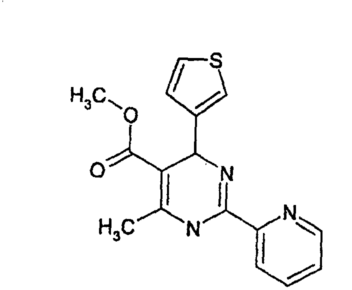 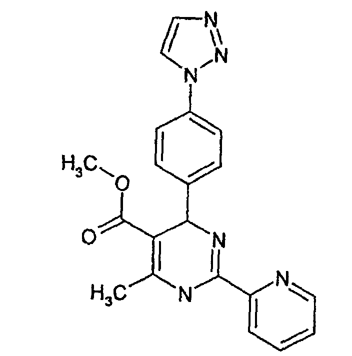 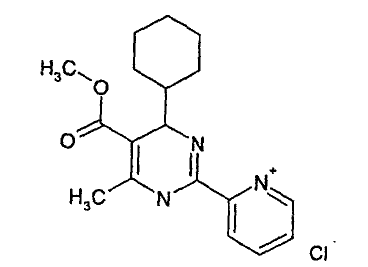 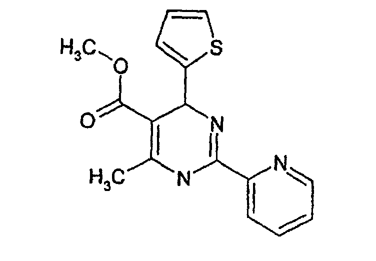 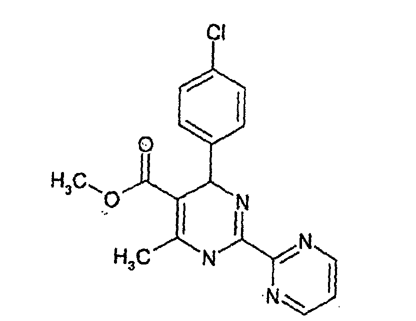 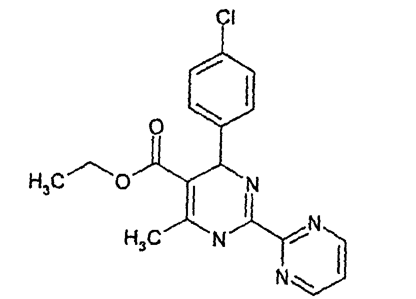 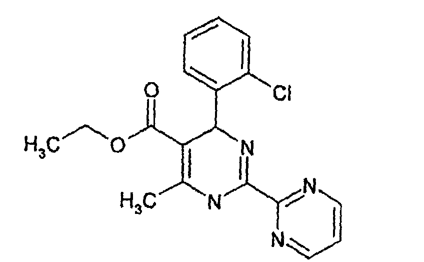 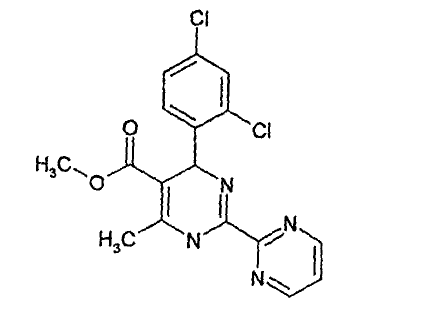 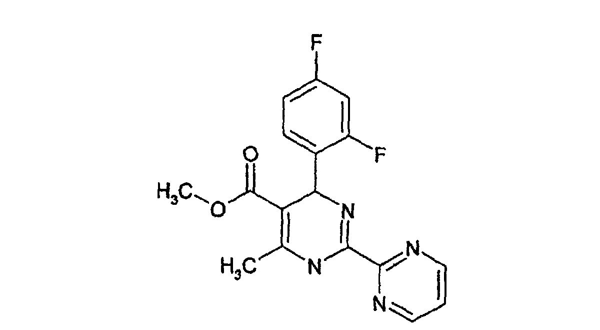 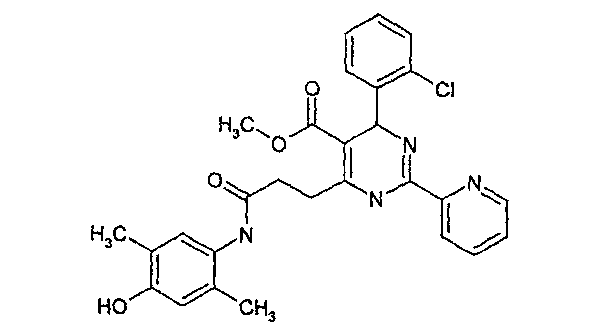 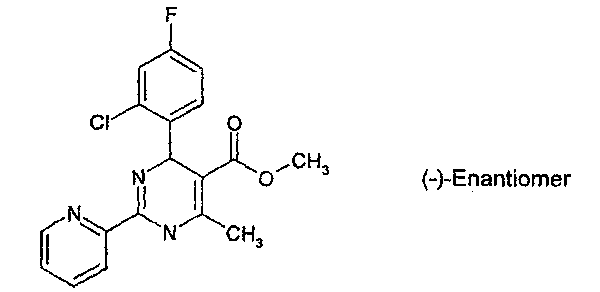 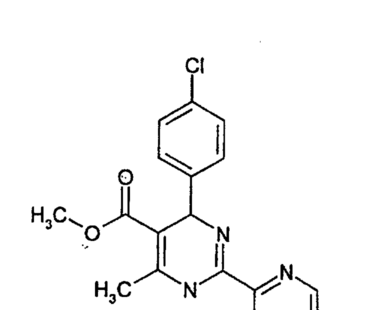 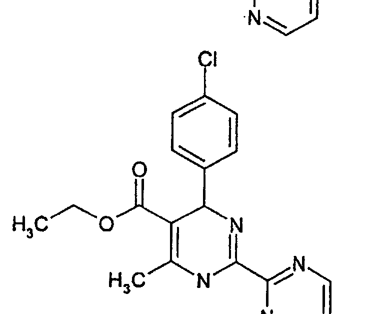 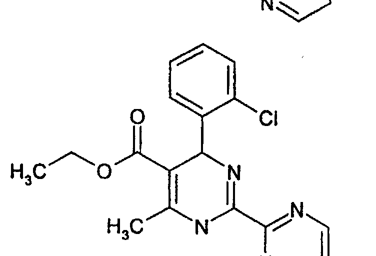 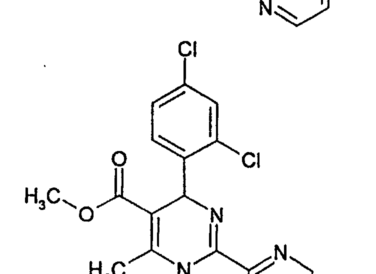 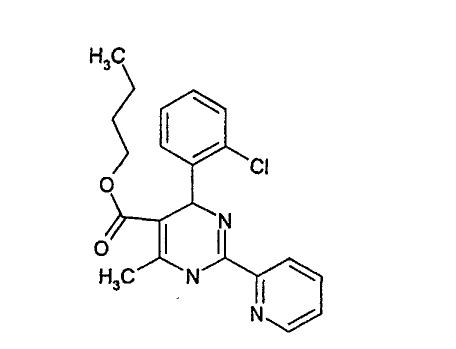 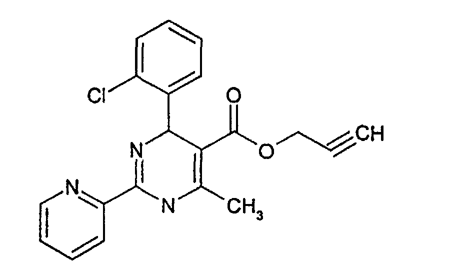 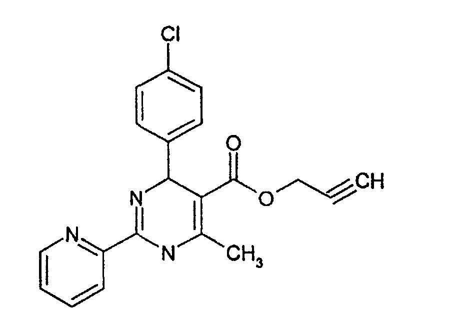 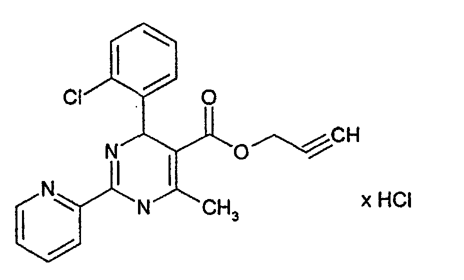 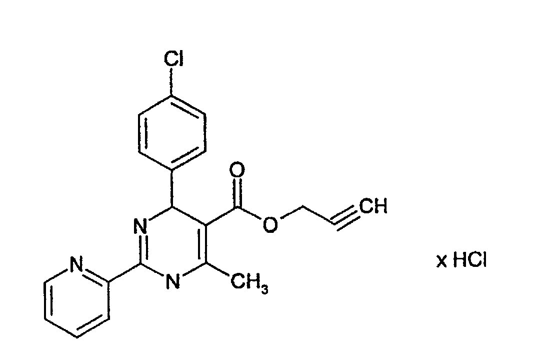 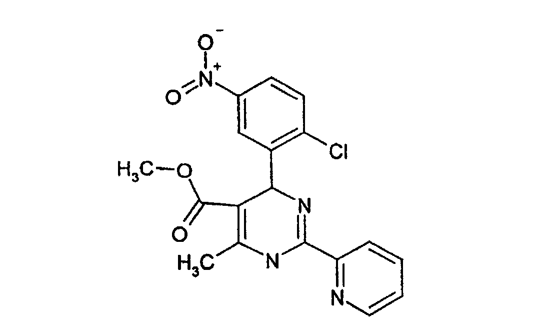 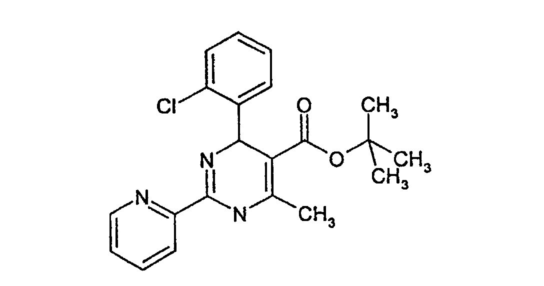 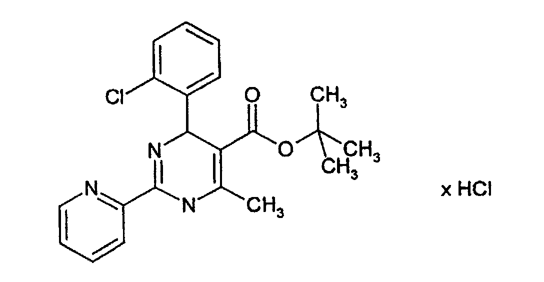 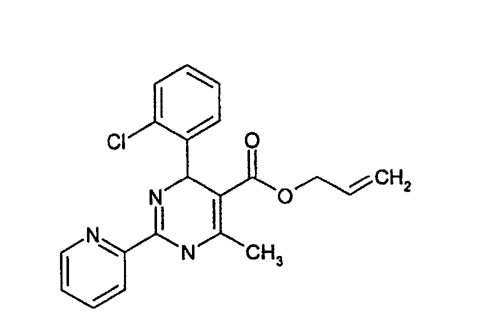 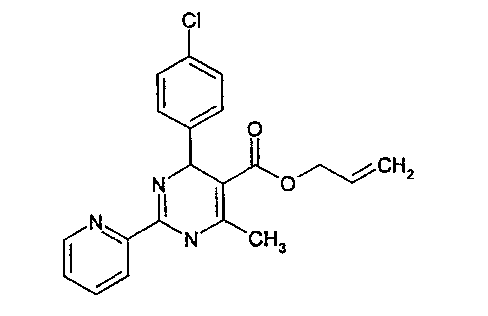 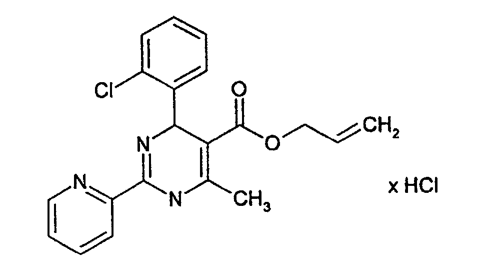 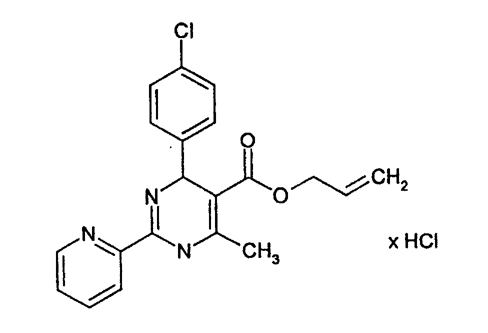 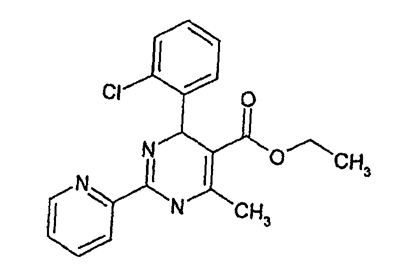 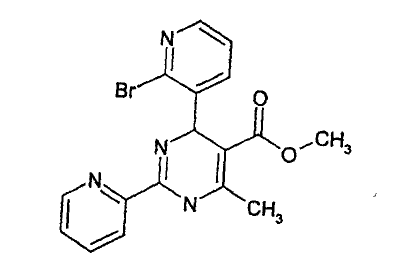 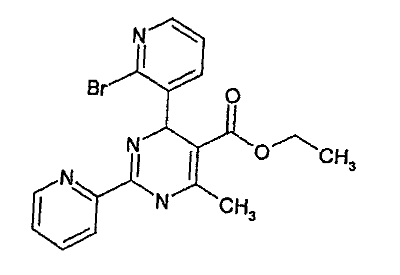 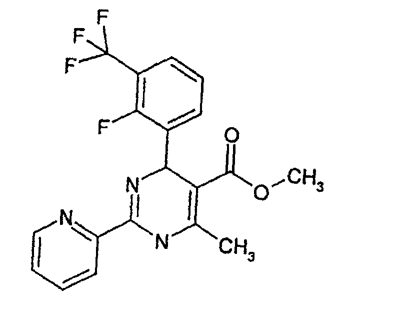 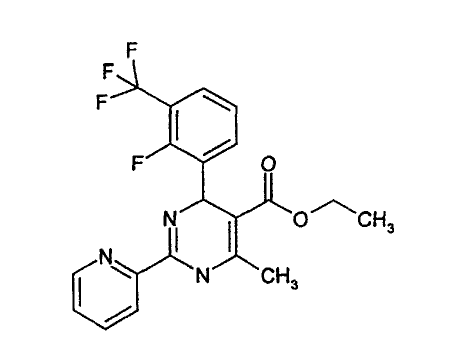 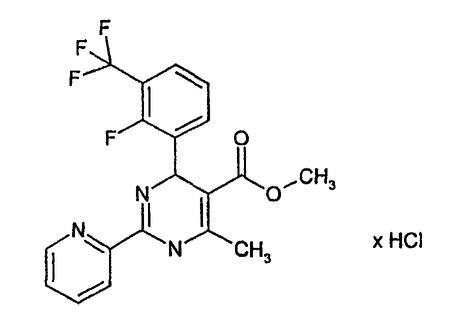 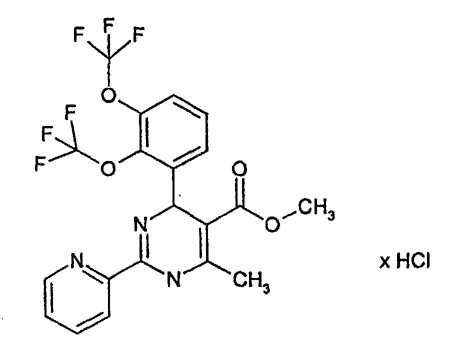 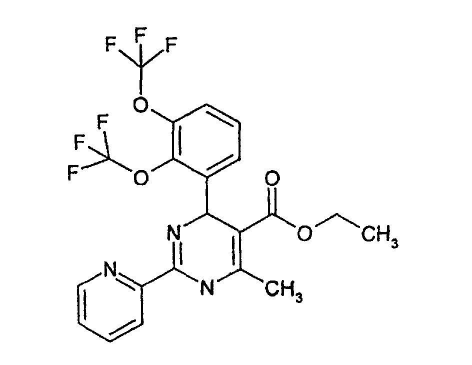 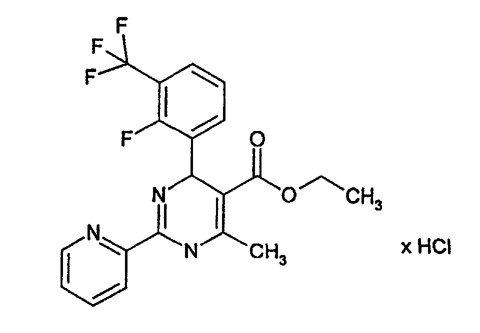 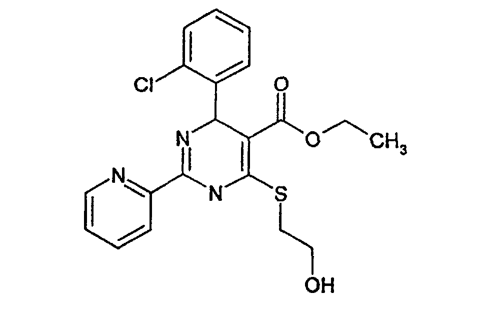 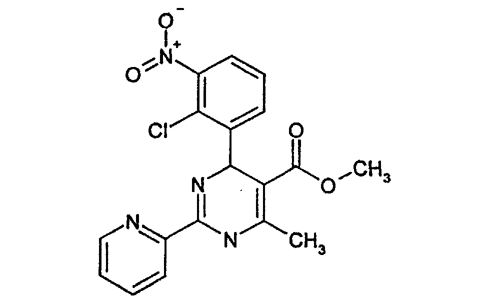 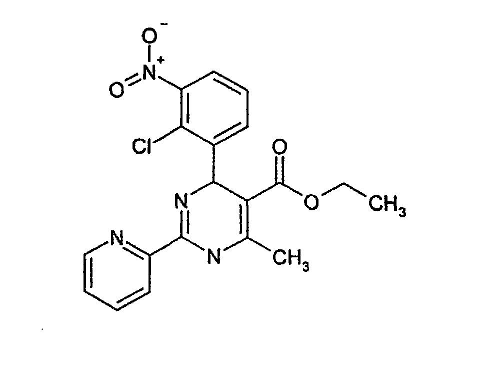 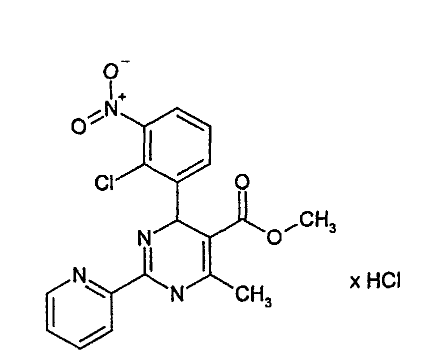 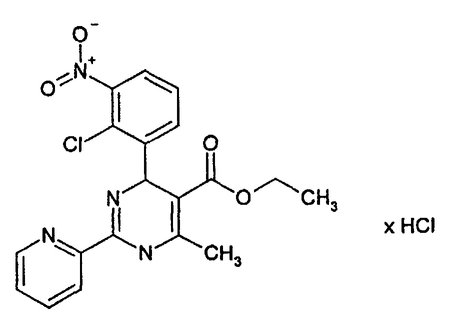 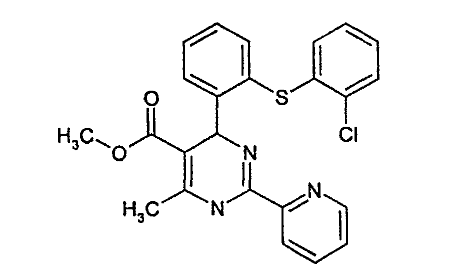 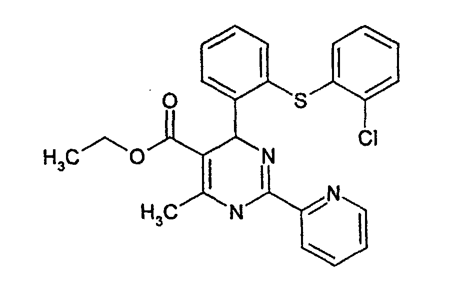 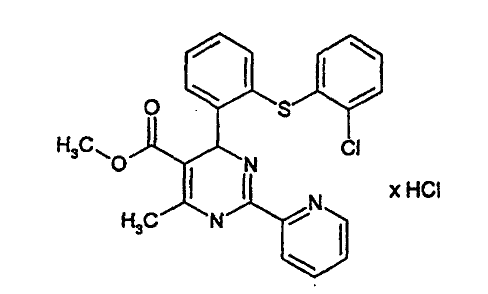 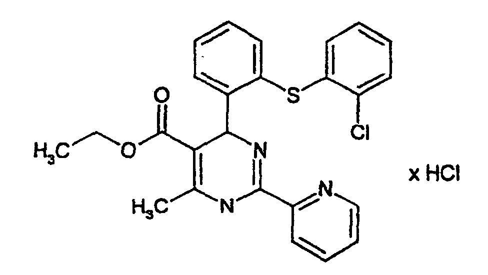 worin 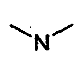 entspricht

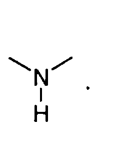

5. Verfahren zur Herstellung von Verbindungen der allgemeinen Formeln (I) und (Ia) der Ansprüche 1 bis 3 und der Verbindungen des Anspruchs 4,
indem man
[A] Aldehyde der allgemeinen Formel (II)
R¹-CHO (II)
in welcher
R¹ die oben angegebene Bedeutung hat,
mit Amidinen der Formel (III) 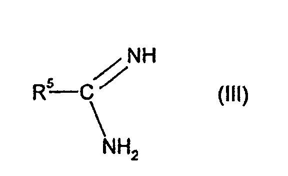 in welcher
R⁵ die oben angegebene Bedeutung hat,
und Verbindungen der allgemeinen Formel (IV)
R³-CO-CH₂-CO-R² (IV)
in welcher
R² und R³ die oben angegebene Bedeutung haben,
gegebenenfalls in Gegenwart inerter organischer Lösemittel mit oder ohne Basen- bzw. Säurezusatz umsetzt,
oder
[B] Verbindungen der allgemeinen Formel (V) 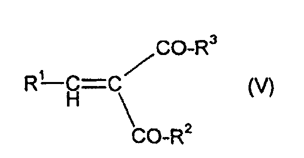 in welcher
R¹, R² und R³ die oben angegebene Bedeutung haben,
mit Amidinen oder deren Hydrochloriden der allgemeinen Formel (III) 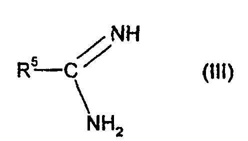 in welcher
R⁵ die oben angegebene Bedeutung hat,
gegebenenfalls in Gegenwart inerter organischer Lösemittel bei Temperaturen zwischen 20°C und 150°C mit oder ohne Basen- oder Säurezusatz umsetzt,
oder
[C] Aldehyde der allgemeinen Formel (II)
R¹-CHO (II)
in welcher
R¹ die oben angegebene Bedeutung hat,
mit Verbindungen der allgemeinen Formel (VI) 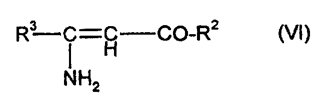 in welcher
R² und R³ die oben angegebene Bedeutung haben,
und Amidinen der allgemeinen Formel (III) wie oben beschrieben umsetzt, oder
[D] Aldehyde der allgemeinen Formel (II) mit Verbindungen der allgemeinen Formel (IV) und Iminoethem der allgemeinen Formel (VII) 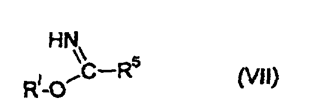 in welcher
R⁵ die oben angegebene Bedeutung hat,
und
R¹ für (C₁-C₄)-Alkyl steht,
in Gegenwart von Ammoniumsalzen umsetzt.

6. Verfahren gemäß Anspruch 5 Variante [A].

7. Verfahren gemäß Anspruch 5 oder 6, **dadurch gekennzeichnet, daß** die Reaktionen in inerten organischen Lösemitteln, bei 20 bis 150°C, vorzugsweise bei Siedetemperatur des Lösemittels, unter Normaldruck, gegebenenfalls in Gegenwart von Säuren oder Basen, vorzugsweise schwachen Säuren, durchgeführt werden.

8. Pharmazeutische Zubereitungen, enthaltend neben nicht-toxischen, inerten pharmazeutisch geeigneten Trägerstoffen eine oder mehrere Verbindungen nach Anspruch 4 oder bestehend aus einer oder mehrerer der besagten Verbindungen.

## Claims

1. Use of dihydropyrimidines of the general formula (I) 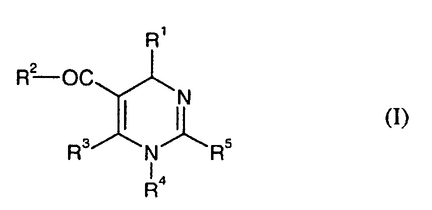 or their isomeric form (Ia) 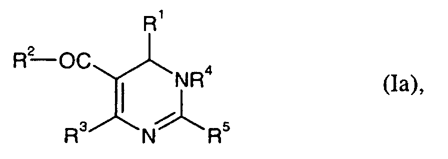 in which
R¹ is phenyl, furyl, thienyl, triazolyl, pyridyl, cycloalkyl having from 3 to 6 carbon atoms or is a radical of the formula 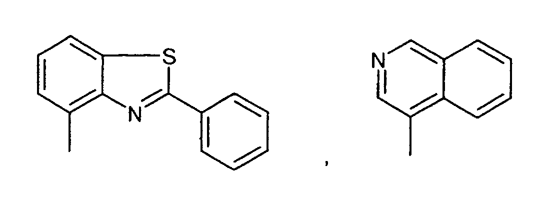 or 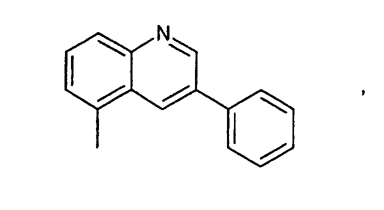 where the abovementioned ring systems are optionally mono- or polysubstituted, identically or differently, by substituents chosen from the group consisting of halogen, trifluoromethyl, nitro, cyano, trifluoromethoxy, carboxyl, hydroxyl, (C₁-C₆)-alkoxy, (C₁-C₆)-alkoxycarbonyl and (C₁-C₆)-alkyl which in turn can be substituted by aryl having from 6 to 10 carbon atoms or halogen,
and/or the above ring systems are optionally substituted by groups of the formulae -S-R⁶, NR⁷R⁸, CO-NR⁹R¹⁰, SO₂-CF₃ and -A-CH₂-R¹¹,
wherein
R⁶ is phenyl, which is optionally substituted by halogen,
R⁷, R⁸, R⁹ and R¹⁰ are identical or different and are hydrogen, phenyl, hydroxy-substituted phenyl, hydroxyl, (C₁-C₆)-acyl or (C₁-C₆)-alkyl, which for its part may be substituted by hydroxyl, (C₁-C₆)-alkoxycarbonyl, phenyl or hydroxy-substituted phenyl,
A is a radical O, S, SO or SO₂,
R¹¹ is phenyl, which is optionally mono- or polysubstituted, identically or differently, by substituents chosen from the group consisting of halogen, nitro, trifluoromethyl, (C₁-C₆)-alkyl and (C₁-C₆)-alkoxy,
R² is a radical of the formula -XR¹² or -NR¹³R¹⁴,
wherein
X is a bond or oxgyen,
R¹² is hydrogen, straight-chain or branched (C₁-C₆)-alkoxycarbonyl or a straight-chain, branched or cyclic, saturated or unsaturated (C₁-C₈)-hydrocarbon radical, which optionally contains one or two identical or different hetero chain members from the group consisting of O, CO, NH, -NH-(C₁-C₄)-alkyl, -N-((C₁-C₄)-alkyl)₂, S and SO₂, and which is optionally substituted by halogen, nitro, cyano, hydroxyl, aryl having from 6 to 10 carbon atoms or aralkyl having from 6 to 10 carbon atoms, heteroaryl or a group of the formula -NR¹⁵R¹⁶,
wherein
R¹⁵ and R¹⁶ are identical or different and are hydrogen, benzyl or (C₁-C₆)-alkyl,
R¹³ and R¹⁴ are identical or different and are hydrogen, (C₁-C₆)-alkyl or cycloalkyl having from 3 to 6 carbon atoms,
R³ is hydrogen, amino or
is a radical of the formula 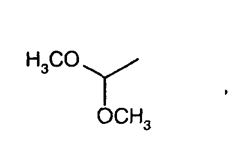 or
is formyl, cyano, hydroxy-substituted (C₁-C₆)-alkylthio, trifluoromethyl or pyridyl, or
is a straight-chain, branched or cyclic, saturated or unsaturated hydrocarbon radical having up to 8 carbon atoms, which is optionally mono- or polysubstituted, identically or differently, by aryloxy having from 6 to 10 carbon atoms, azido, halogen, cyano, hydroxyl, carboxyl, (C₁-C₆)-alkoxycarbonyl, a 5- to 7-membered heterocyclic ring, (C₁-C₆)-alkylthio or (C₁-C₆)-alkoxy, which for its part can be substituted by azido or amino,
and/or is substituted by triazolyl, which for its part can be substituted up to 3 times by (C₁-C₆)-alkoxycarbonyl,
and/or can be substituted by groups of the formula -OSO₂-CH₃ or (CO)ₐ-NR¹⁷R¹⁸,
wherein
a is a number 0 or 1,
R¹⁷ und R¹⁸ are identical or different and are hydrogen or aryl, aralkyl having from 6 to 10 carbon atoms,
or are (C₁-C₆)-alkyl, which is optionally substituted by (C₁-C₆)-alkoxycarbonyl, amino, hydroxyl, phenyl or benzyl, where phenyl or benzyl are optionally mono- or polysubstituted, identically or differently, by hydroxyl, carboxyl, (C₁-C₆)-alkyl or (C₁-C₆)-alkoxy,
or (C₁-C₆)-alkyl is optionally substituted by groups of the formula NH-CO-CH₃ or NH-CO-CF₃,
or
R¹⁷ and R¹⁸ together with the nitrogen atom form a morpholine, piperidinyl or pyrrolidinyl ring,
or
R³ is phenyl, which is optionally substituted by methoxy,
or
R² and R³ together form a radical of the formula 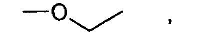
R⁴ is hydrogen, (C₁-C₄)-alkyl, (C₂-C₄)-alkenyl, benzoyl or is acyl having from 2 to 6 carbon atoms,
R⁴ is hydrogen, methyl, benzoyl or is (C₂-C₆)-acyl,
R⁵ is pyridyl, pyrimidyl or pyrazinyl,
and their salts for the preparation of medicaments for the treatment and prophylaxis of hepatitis B.

2. Use according to Claim 1, where
R¹ is phenyl, furyl, thienyl, triazolyl, pyridyl, cyclopentyl or cyclohexyl or is a radical of the formula 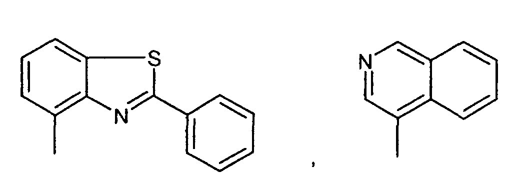 or 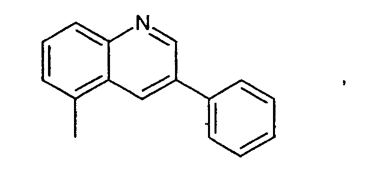 where the abovementioned ring systems are optionally mono- or disubstituted, identically or differently, by substituents chosen from the group consisting of halogen, trifluoromethyl, nitro, SO₂-CF₃, methyl, cyano, trifluoromethoxy, hydroxyl, carboxyl, methoxycarbonyl or radicals of the formula -CO-NH-CH₂-C(CH₃)₃, -CO-NH(CH₂)₂OH, -CO-NH-CH₂-C₆H₅, -CO-NH-C₆H₅, -CO-NH-(pOH)-C₆H₄, -O-CH₂-C₆H₅ or -S-pCl-C₆H₄,
R² is a radical of the formula -XR¹² or -NR¹³R¹⁴,
wherein
X is a bond or an oxygen atom,
R¹² is hydrogen, (C₁-C₄)-alkenyl, (C₁-C₄)-alkoxycarbonyl or (C₁-C₄)-alkyl which are optionally substituted by pyridyl, cyano, phenoxy, benzyl or by a radical of the formula -NR¹⁵R¹⁶,
wherein
R¹⁵ and R¹⁶ are identical or different and are hydrogen, benzyl or (C₁-C₄)-alkyl,
R¹³ and R¹⁴ are identical or different and are hydrogen, (C₁-C₄)-alkyl or cyclopropyl,
R³ is hydrogen, amino or a radical of the formula 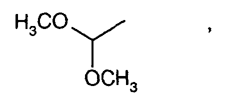 or
is formyl, cyano, hydroxy-substituted (C₁-C₄)-alkylthio, trifluoromethyl, cyclopropyl or pyridyl, or
is (C₁-C₄)-alkyl, which is optionally substituted by halogen, (C₁-C₄)-alkoxycarbonyl, hydroxyl or by triazolyl, which for its part can be substituted up to 3 times by (C₁-C₄)-alkoxycarbonyl,
and/or alkyl is optionally substituted by groups of the formulae -OSO₂-CH₃ or (CO)ₐ-NR¹⁷R¹⁸,
wherein
a is a number 0 or 1,
R¹⁷ and R¹⁸ are identical or different and are hydrogen, phenyl or benzyl, or
are C₁-C₄-alkyl, which is optionally substituted by (C₁-C₄)-alkoxycarbonyl, amino, hydroxyl, phenyl or benzyl, where phenyl or benzyl are optionally mono- or polysubstituted, identically or differently, by hydroxyl, carboxyl, (C₁-C₄)-alkyl or (C₁-C₄)-alkoxy,
and/or (C₁-C₄)-alkyl is optionally substituted by radicals of the formula -NH-CO-CH₃ or -NH-CO-CF₃,
or
R¹⁷ and R¹⁸ together with the nitrogen atom form a morpholine, piperidinyl or pyrrolidinyl ring,
or
R³ is phenyl, which is optionally substituted by methoxy,
or
R² and R³ together form a radical of the formula 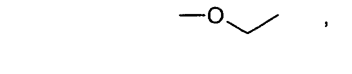
R⁴ is hydrogen, methyl, vinyl or acetyl, and,
R⁵ is pyridyl, pyrimidyl or pyrazinyl.

3. Use according to either of Claims 1 or 2, where
R¹ is phenyl or triazolyl, which are optionally substituted up to twice, identically or differently, by fluorine, chlorine, bromine or iodine,
R² is straight-chain or branched alkoxycarbonyl having up to 4 carbon atoms,
R³ is methyl, ethyl or cyclopropyl,
or
R² and R³ together form a radical of the formula 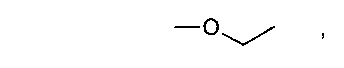
R⁴ is hydrogen, vinyl or acetyl,
and
R⁵ is pyridyl.

4. Dihydropyrimidines having the following structures 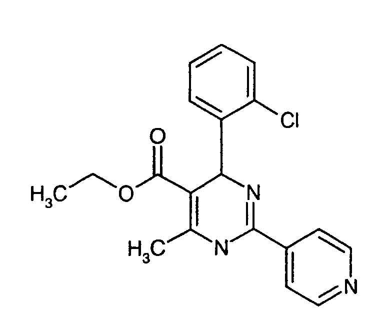 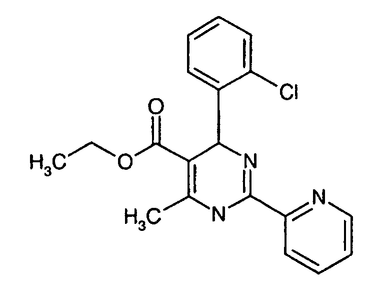 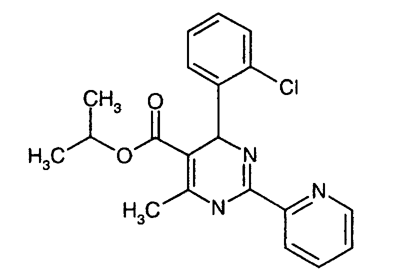 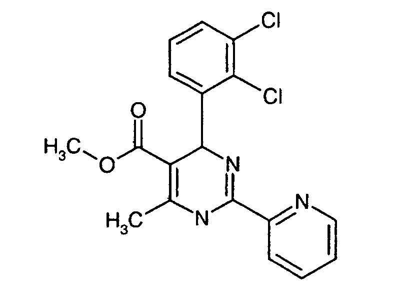 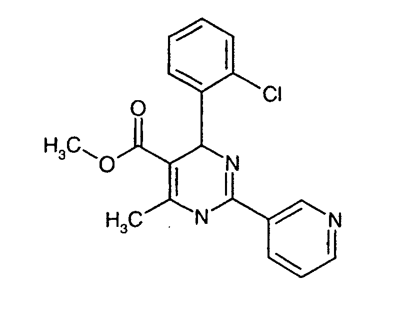 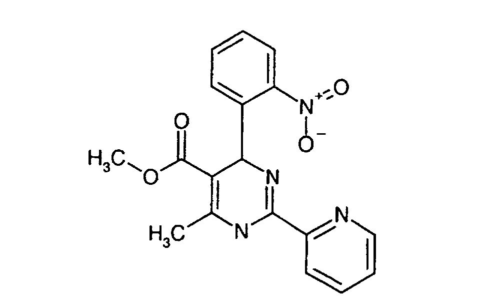 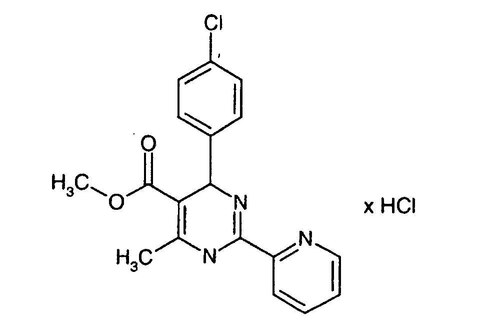 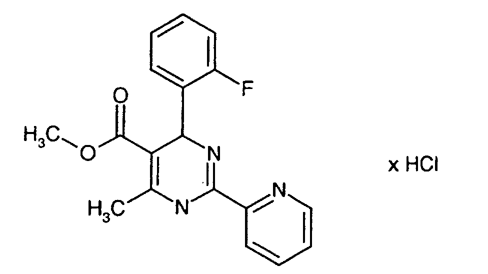 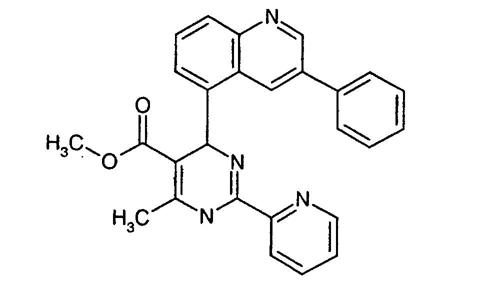 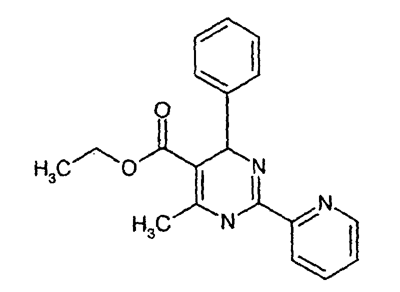 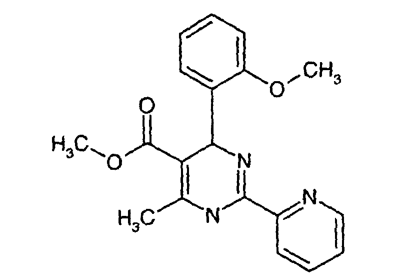 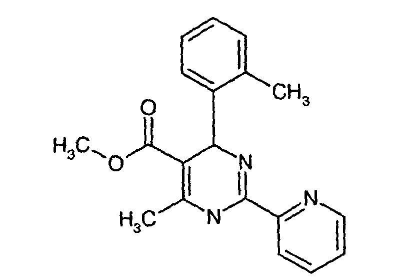 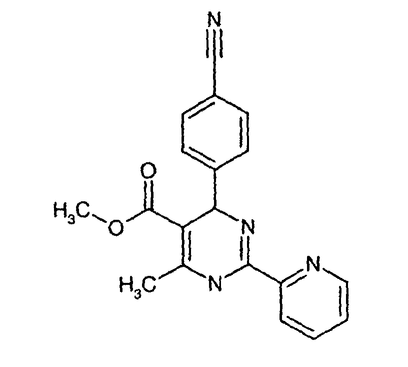 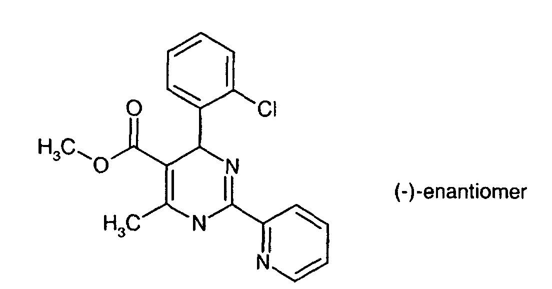 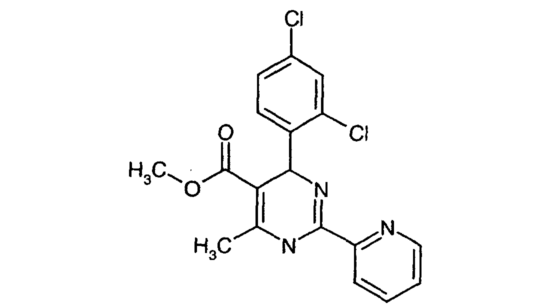 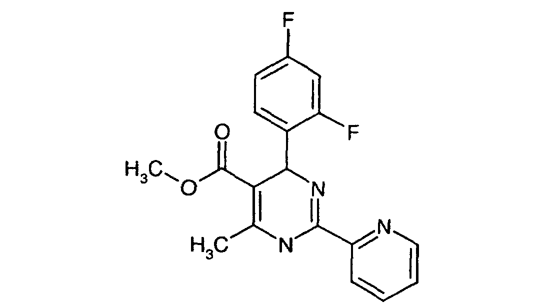 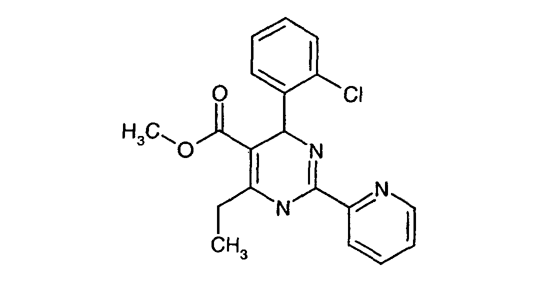 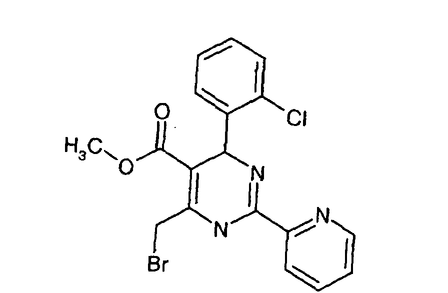 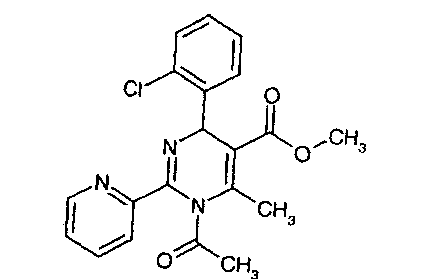 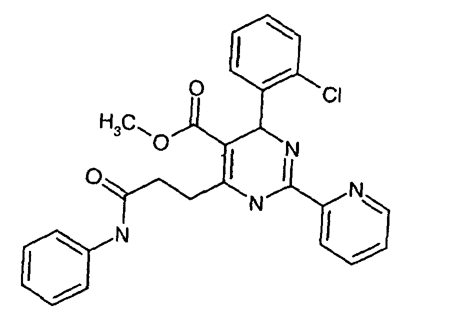 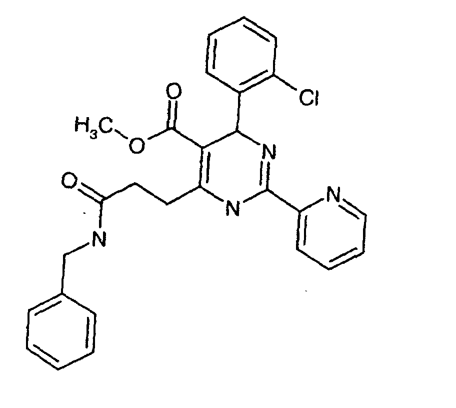 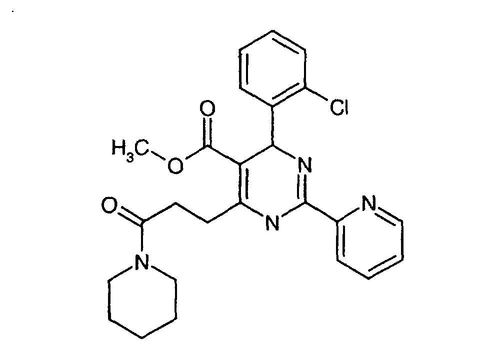 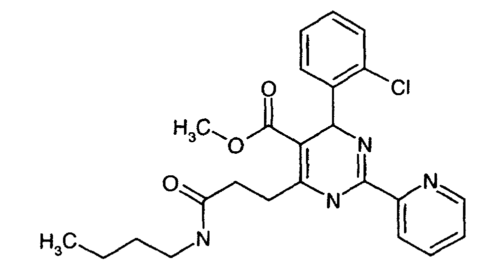 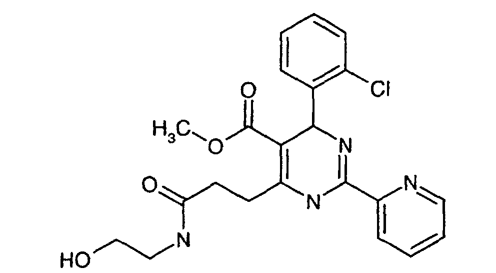 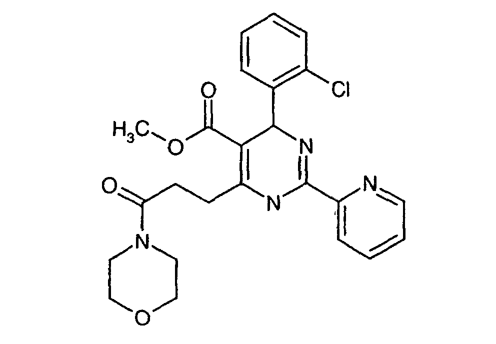 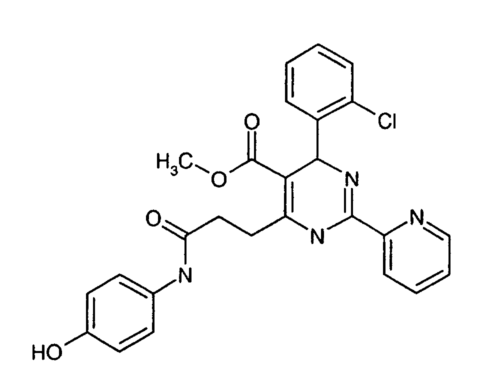 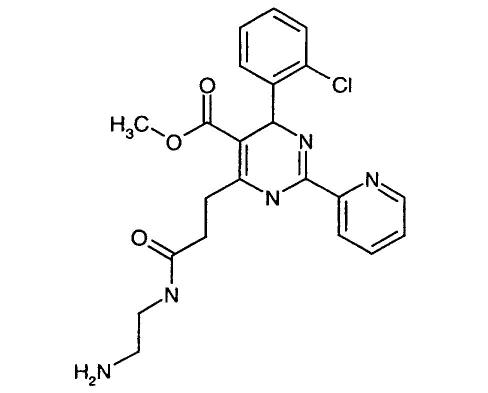 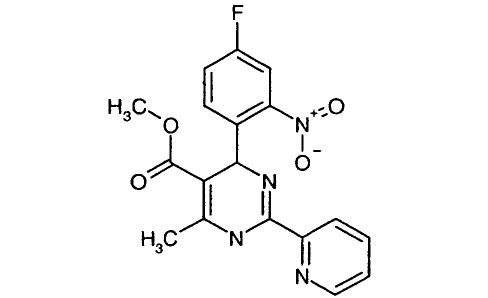 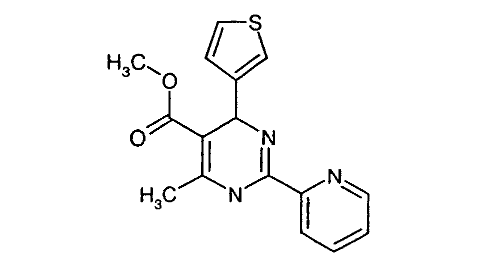 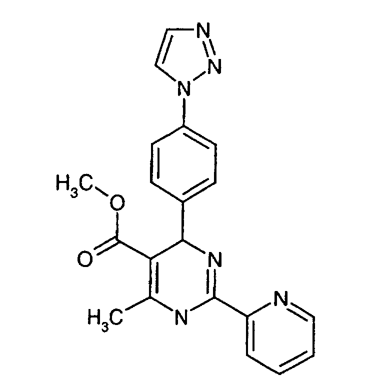 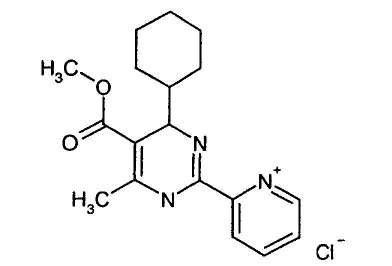 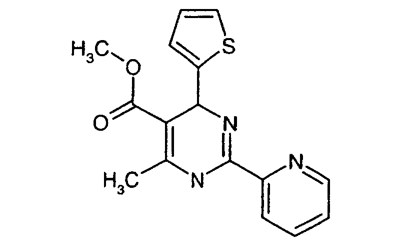 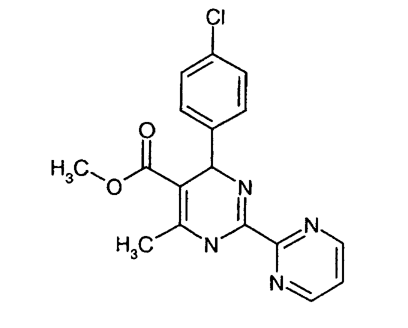 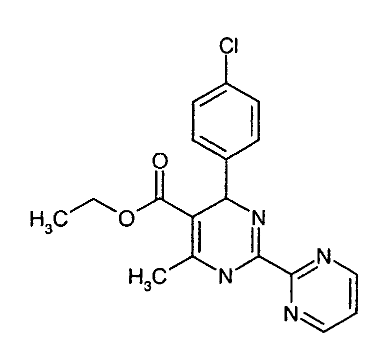 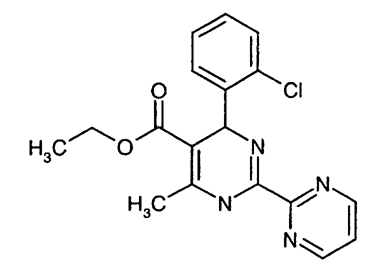 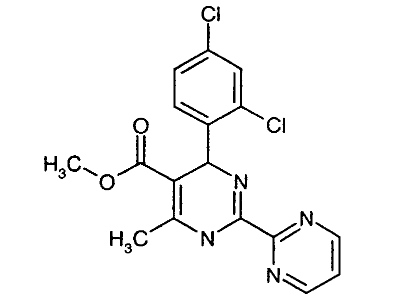 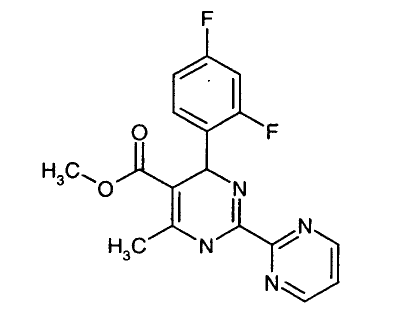 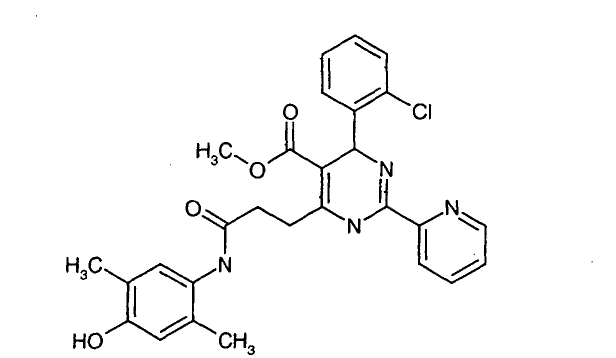 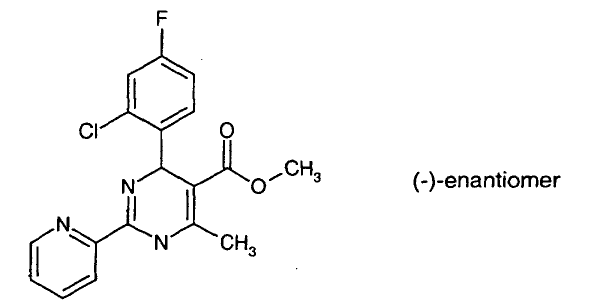 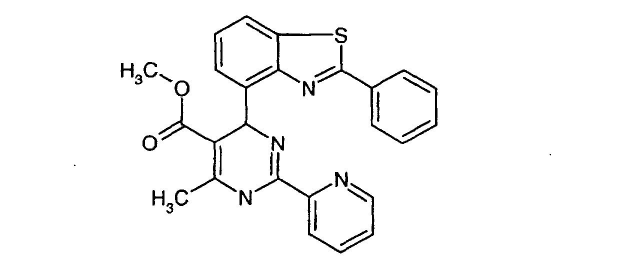 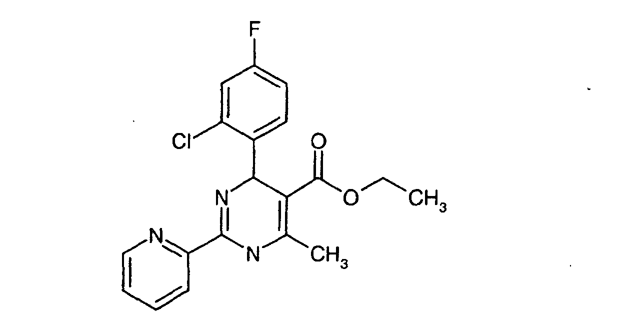 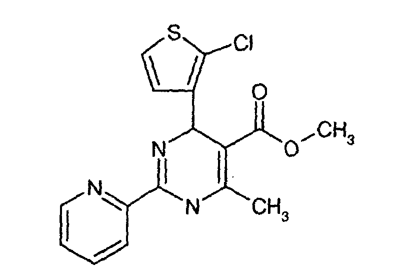 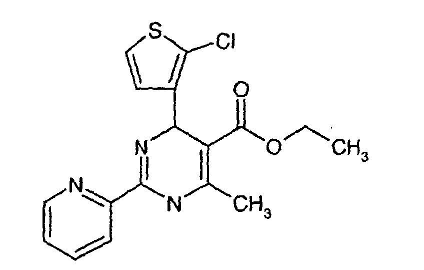 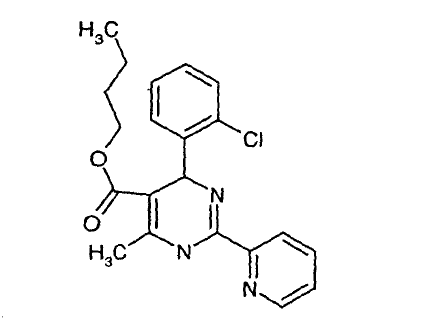 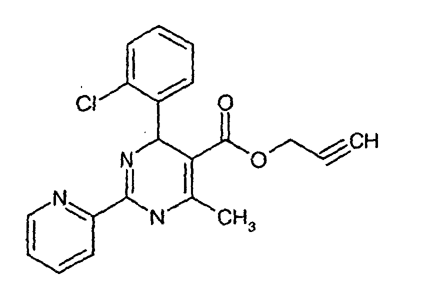 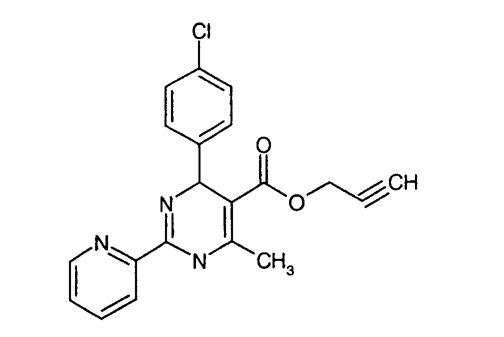 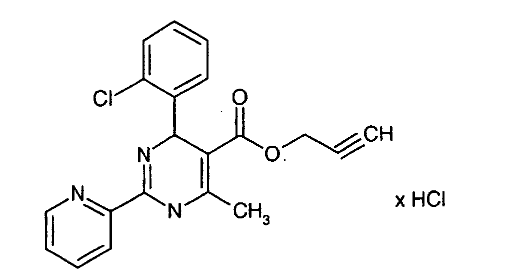 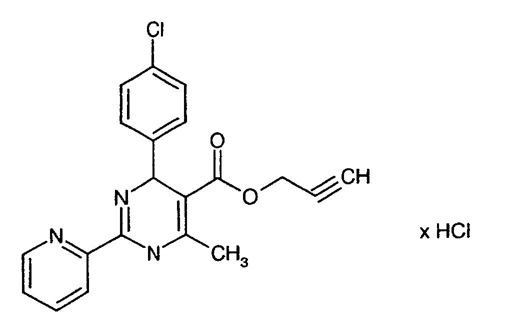 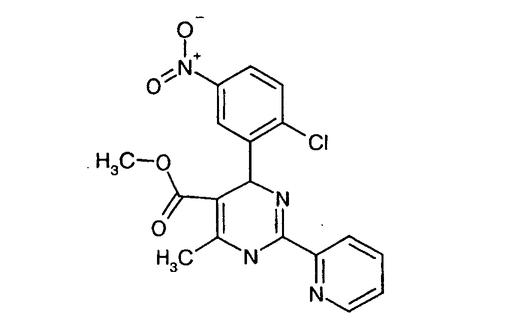 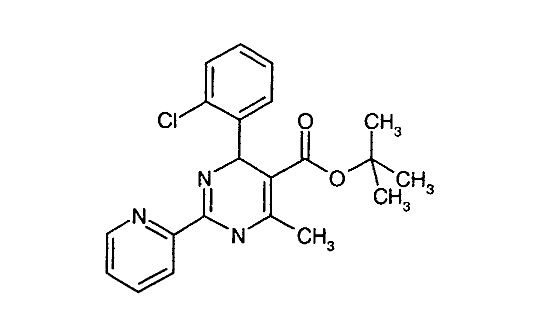 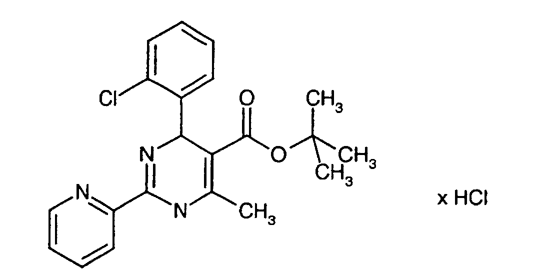 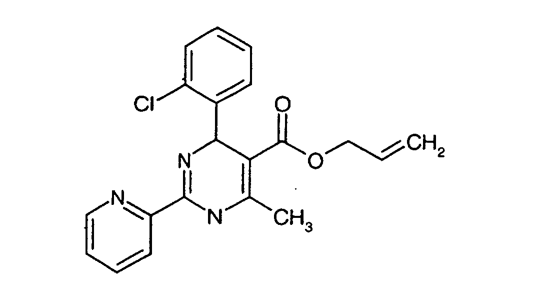 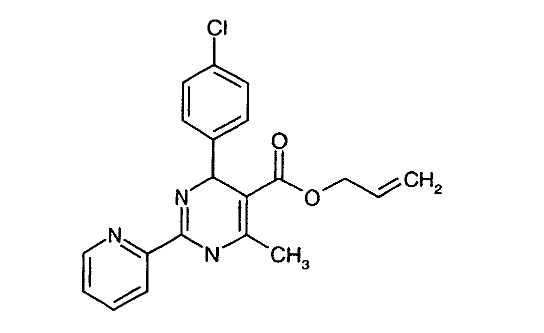 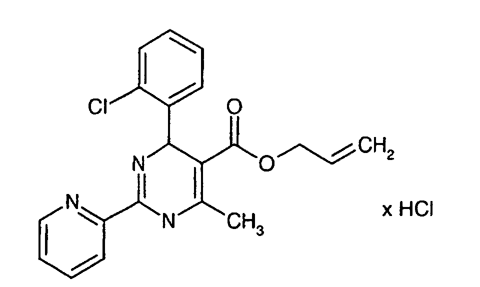 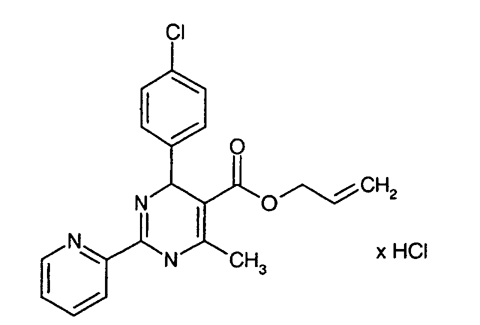 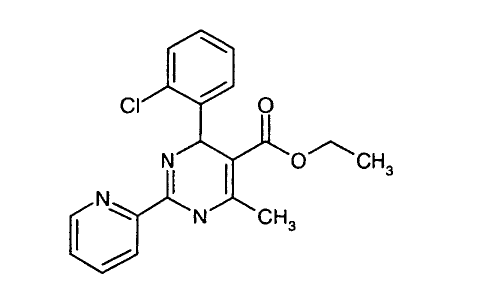 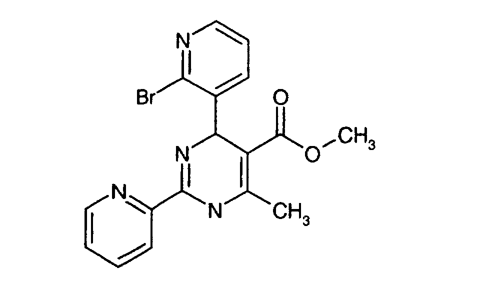 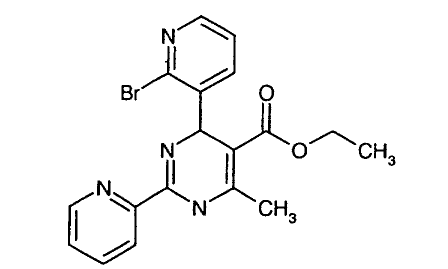 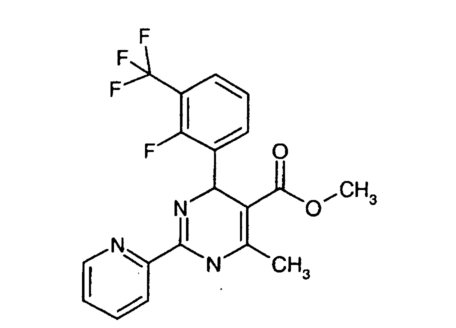 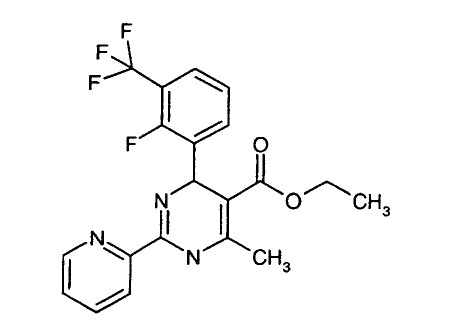 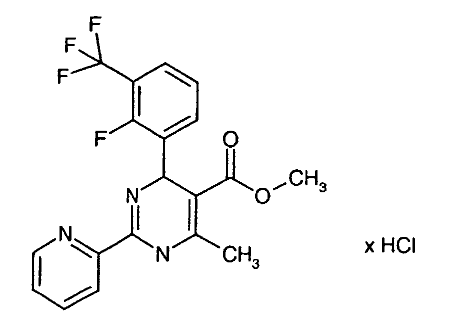 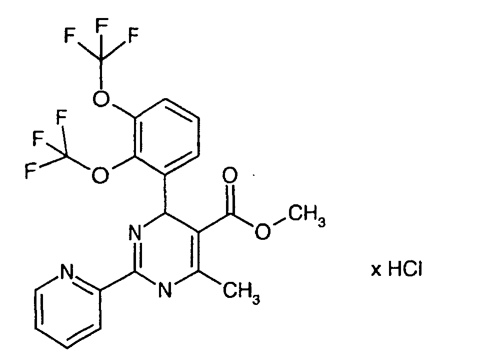 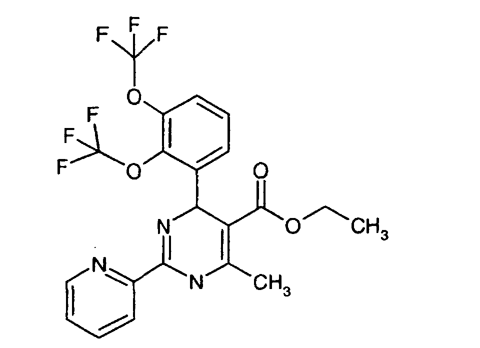 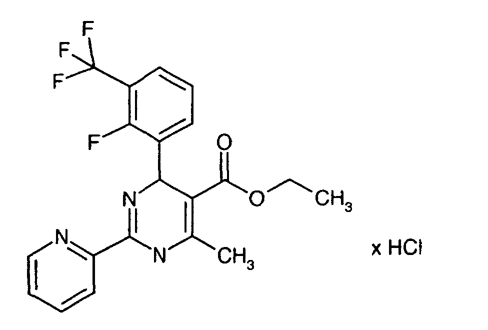 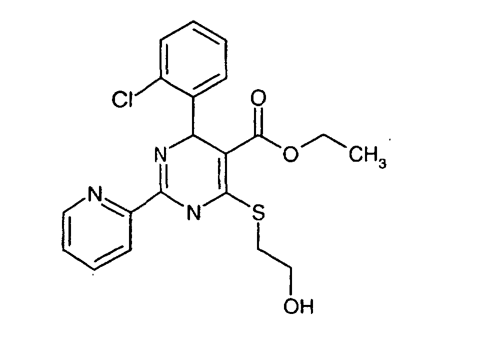 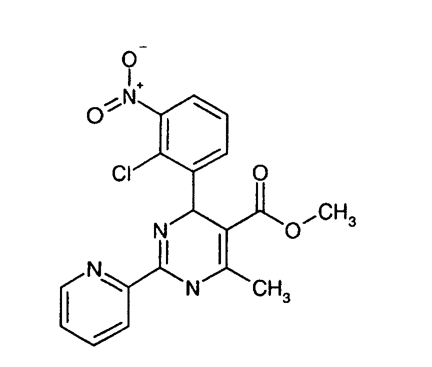 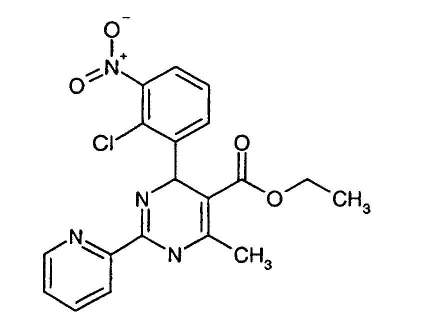 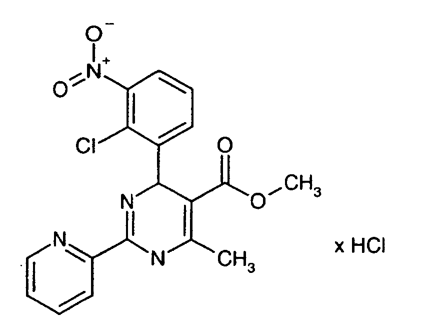 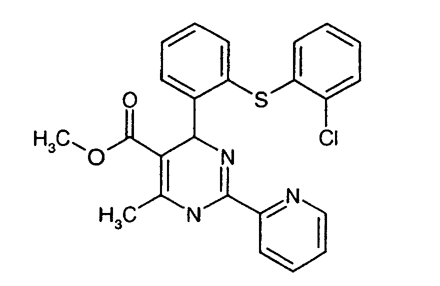 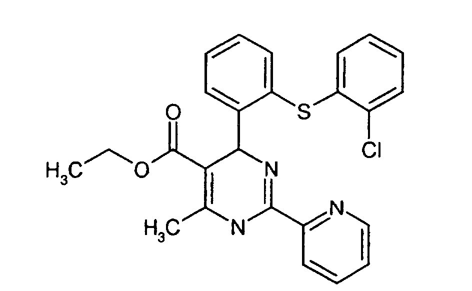 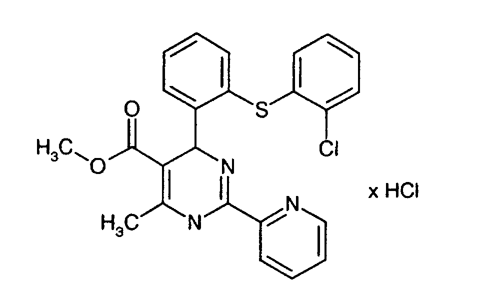 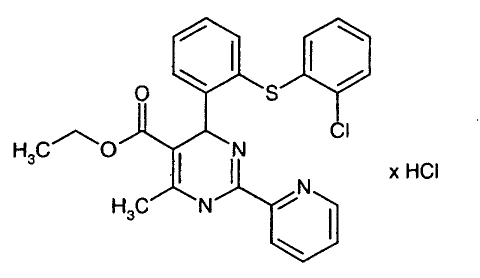 wherein 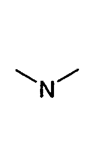 corresponds to

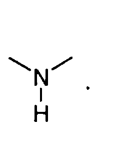

5. Process for the preparation of compounds of the general formulae (I) and (Ia) of Claims 1 to 3 and of the compounds of Claim 4,
by reacting
[A] aldehydes of the general formula (II)
R¹-CHO (II)
in which
R¹ is as defined above,
with amidines of the formula (III) 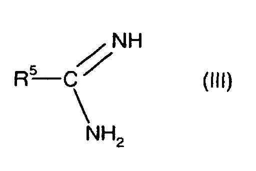 in which
R⁵ is as defined above,
and compounds of the general formula (IV)
R³-CO-CH₂-CO-R² (IV)
in which
R² and R³ are as defined above,
optionally in the presence of inert organic solvents with or without the addition of base or acid,
or reacting
[B] compounds of the general formula (V) 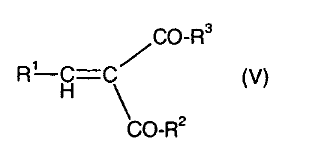 in which
R¹, R² and R³ are as defined above,
with amidines or their hydrochlorides of the general formula (III) 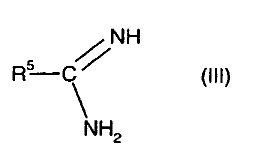 in which
R⁵ is as defined above,
optionally in the presence of inert organic solvents at temperatures between 20°C and 150°C with or without the addition of base or acid,
or reacting
[C] aldehydes of the general formula (II)
R¹-CHO (II)
in which
R¹ is as defined above,
with compounds of the general formula (VI) 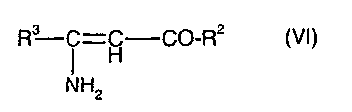 in which
R² and R³ are as defined above,
and amidines of the general formula (III) as described above,
or reacting
[D] aldehydes of the general formula (II) with compounds of the general formula (IV) and imino ethers of the general formula (VII) 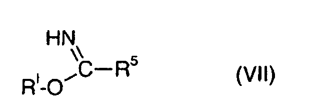 in which
R⁵ is as defined above,
and
R¹ is (C₁-C₄)-alkyl,
in the presence of ammonium salts.

6. Process according to Claim 5 variant [A].

7. Process according to Claim 5 or 6, **characterized in that** the reactions are carried out in inert organic solvents, at 20 to 150°C, preferably at the boiling temperature of the solvent, under atmospheric pressure, optionally in the presence of acids or bases, preferably with weak acids.

8. Pharmaceutical preparations comprising, in addition to non-toxic, inert pharmaceutically suitable carriers, one or more compounds according to Claim 4, or consisting of one or more of said compounds.

## Revendications

1. Utilisation de dihydropyrimidines de formule générale (I) 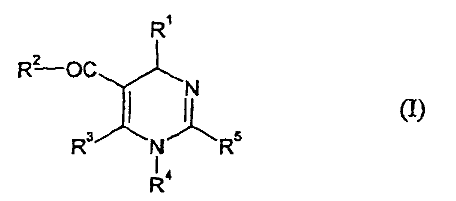 ou de sa forme isomère (Ia) 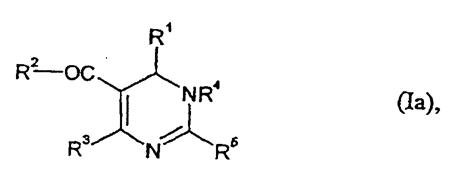 formules dans lesquelles
R¹ est un groupe phényle, furyle, thiényle, triazolyle, pyridyle, cycloalkyle ayant 3 à 6 atomes de carbone ou représente des restes de formules 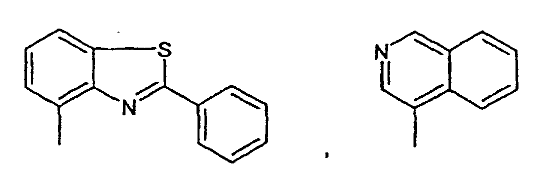 ou 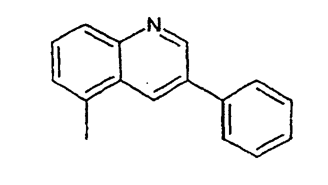 les noyaux indiqués ci-dessus étant éventuellement substitués une ou plusieurs fois identiques ou différentes par des substituants choisis dans le groupe halogène, trifluorométhyle, nitro, cyano, trifluorométhoxy, carboxyle, hydroxyle, alkoxy en C₁ à C₆, (alkoxy en C₁ à C₆)carbonyle et alkyle en C₁ à C₆ qui peut lui-même être substitué par un radical aryle ayant 6 à 10 atomes de carbone ou par un halogène,
et/ou les noyaux indiqués étant éventuellement substitués par des groupes de formules -S-R⁶, NR⁷R⁸, CO-NR⁹R¹⁰, SO₂-CF₃ et -A-CH₂R¹¹,
dans lesquels
R⁶ désigne un groupe phényle qui est éventuellement substitué par un halogène,
R⁷, R⁸, R⁹ et R¹⁰ sont identiques ou différents et représentent l'hydrogène, un groupe phényle, phényle à substituant hydroxy, hydroxy, acyle en C₁ à C₆ ou alkyle en C₁ à C₆ pouvant lui-même être substitué par un radical hydroxy, (alkoxy en C₁ à C₆)carbonyle, phényle ou phényle à substituant hydroxy,
A est un reste O, S, SO ou SO₂,
R¹¹ désigne un groupe phényle qui est éventuellement substitué une ou plusieurs fois identiques ou différentes par des substituants choisis dans le groupe halogène, nitro, trifluorométhyle, alkyle en C₁ à C₆ et alkoxy en C₁ à C₆,
R² est un reste de formule -XR¹² ou -NR¹³R¹⁴,
où
X désigne une liaison ou l'oxygène,
R¹² est l'hydrogène, un groupe (alkoxy en C₁ à C₆)carbonyle linaire ou ramifié ou un reste hydrocarboné en C₁ à C₈ linéaire, ramifié ou cyclique, saturé ou non saturé, qui contient éventuellement un ou deux chaînons hétéroatomiques identiques ou différents du groupe O, CO, NH, -NH-(alkyle en C₁ à C₄), -N-(alkyle en C₁ à C₄)₂, S ou SO₂ et qui est éventuellement substitué par un radical halogéno, nitro, cyano, hydroxy, aryle ayant 6 à 10 atomes de carbone ou aralkyle ayant 6 à 10 atomes de carbone, hétéroaryle ou un groupe de formule -NR¹⁵R¹⁶,
dans laquelle
R¹⁵ et R¹⁶ sont identiques ou différents et représentent l'hydrogène, un reste benzyle ou alkyle en C₁ à C₆,
R¹³ et R¹⁴ sont identiques ou différents et représentent l'hydrogène, un reste alkyle en C₁ à C₆ ou cycloalkyle ayant 3 à 6 atomes de carbone,
R³ est l'hydrogène, un groupe amino ou
un reste de formule générale 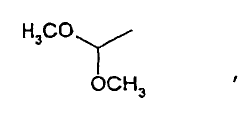 ou représente un groupe formyle, cyano, alkylthio en C₁ à C₆ à substituant hydroxy, trifluorométhyle ou pyridyle ou représente
un reste hydrocarboné linéaire, ramifié ou cyclique, saturé ou non saturé, ayant jusqu'à 8 atomes de carbone, qui est éventuellement substitué une ou plusieurs fois identiques ou différentes par un radical aryloxy ayant 6 à 10 atomes de carbone, azido, halogéno, cyano, hydroxy, carboxyle, (alkoxy en C₁ à C₆)carbonyle, un noyau hétérocyclique pentagonal à heptagonal, un groupe alkylthio en C₁ à C₆ ou alkoxy en C₁ à C₆, qui peut lui-même être substitué par un radical azido ou amino,
et/ou substitué par un reste triazolyle qui peut lui-même être substitué jusqu'à trois fois par un radical (alkoxy en C₁ à C₆)carbonyle,
et/ou peut être substitué par des groupes de formules -OSO₂-CH₃ ou -(CO)ₐ-NR¹⁷R¹⁸,
où
a représente le nombre 0 ou 1,
R¹⁷ et R¹⁸ sont identiques ou différents et représentent l'hydrogène ou un reste aryle, un reste aralkyle ayant 6 à 10 atomes de carbone,
ou désignent un reste alkyle en C₁ à C₆ qui est éventuellement substitué par un groupe (alkoxy en C₁ à C₆)carbonyle, amino, hydroxyle, phényle ou benzyle, les groupes phényle ou benzyle étant éventuellement substitués une ou plusieurs fois identiques ou différentes par des radicaux hydroxy, carboxyle, alkyle en C₁ à C₆ ou alkoxy en C₁ à C₆,
ou bien le reste alkyle en C₁ à C₆ est éventuellement substitué par des groupes de formules NH-CO-CH₃ ou NH-CO-CF₃,
ou bien
R¹⁷ et R¹⁸ forment conjointement avec l'atome d'azote un noyau morpholine, pipéridinyle ou pyrrolidinyle,
ou bien
R³ est un reste phényle qui est éventuellement substitué par un radical méthoxy,
ou bien
R² et R³ forment ensemble un reste de formule générale 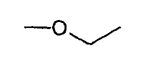
R⁴ est l'hydrogène, un reste alkyle en C₁ à C₄, alcényle en C₂ à C₄, benzoyle ou un reste acyle ayant 2 à 6 atomes de carbone,
R⁴ est l'hydrogène, un groupe méthyle, benzoyle ou un groupe acyle en C₂ à C₆,
R⁵ est un groupe pyridyle, pyrimidyle ou pyrazinyle, ainsi que leurs sels, pour la préparation de médicaments destinés au traitement et à la prophylaxie de l'hépatite B.

2. Utilisation suivant la revendication 1, dans laquelle,
R¹ est un groupe phényle, furyle, thiényle, triazolyle, pyridyle, cyclopentyle ou cyclohexyle ou représente des restes de formules 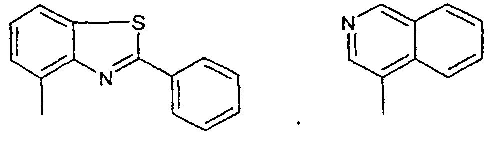 ou 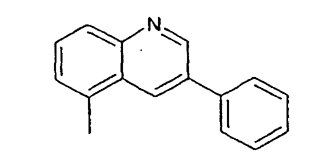 les noyaux énumérés ci-dessus étant éventuellement substitués une ou deux fois identiques ou différentes par des substituants choisis dans le groupe halogène, trifluorométhyle, nitro, SO₂-CF₃, méthyle, cyano, trifluorométhoxy, hydroxy, carboxyle, méthoxycarbonyle ou des restes de formules -CO-NH-CH₂-C(CH₃)₃, -CO-NH(CH₂)₂OH, -CO-NH-CH₂-C₆H₅, -CO-NH-C₆H₅, -CO-NH- (pOH)-C₆H₄, -O-CH₂-C₆H₅ ou -S-pCl-C₆H₄,
R² représente un reste de formule -XR¹² ou -NR¹³R¹⁴
où
X désigne une liaison ou un atome d'oxygène,
R¹² est l'hydrogène, un groupe alcényle en C₁ à C₄, (alkoxy en C₁ à C₄)carbonyle ou alkyle en C₁ à C₄, ces groupes étant éventuellement substitués par un radical pyridyle, cyano, phénoxy, benzyle ou par un reste de formule -NR¹⁵R¹⁶,
où
R¹⁵ et R¹⁶ sont identiques ou différents et représentent l'hydrogène, un radical benzyle ou alkyle en C₁ à C₄,
R¹³ et R¹⁴ sont identiques ou différents et représentent l'hydrogène, un groupe alkyle en C₁ à C₄ ou cyclopropyle,
R³ est l'hydrogène, un groupe amino ou un reste de formule 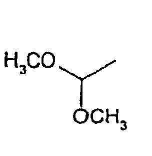 ou bien
un groupe formyle, cyano, alkylthio en C₁ à C₄ à substituant hydroxy, trifluorométhyle, cyclopropyle ou pyridyle,
ou représente un groupe alkyle en C₁ à C₄ qui est éventuellement substitué par un halogène, un radical (alkoxy en C₁ à C₄)carbonyle, hydroxy, ou par un radical triazolyle qui peut lui-même être substitué jusqu'à trois fois par un radical. (alkoxy en C₁ à C₄)carbonyle,
et/ou le groupe alkyle est éventuellement substitué par des groupes de formules -OSO₂-CH₃ ou (CO)ₐ-NR¹⁷R¹⁸,
où
a représente le nombre 0 ou 1,
R¹⁷ et R¹⁸ sont identiques ou différents et représentent l'hydrogène, un groupe phényle ou benzyle, ou représentent
un groupe alkyle en C₁ à C₄ qui est éventuellement substitué par un radical (alkoxy en C₁ à C₄)carbonyle, amino, hydroxyle, phényle ou benzyle, les radicaux phényle ou benzyle étant éventuellement substitués une ou plusieurs fois identiques ou différentes par des groupes hydroxy, carboxy, alkyle en C₁ à C₄ ou alkoxy en C₁ à C₄,
et/ou le groupe alkyle en C₁ à C₄ est éventuellement substitué par des restes de formules -NH-CO-CH₃ ou -NH-CO-CF₃,
ou bien
R¹⁷ et R¹⁸ forment conjointement avec l'atome d'azote un noyau morpholine, pipéridinyle ou pyrrolidinyle,
ou bien
R³ est un reste phényle qui est éventuellement substitué par un radical méthoxy,
ou bien
R² et R³ forment ensemble un reste de formule 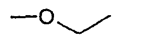
R⁴ est l'hydrogène, un groupe méthyle, vinyle ou acétyle,
R⁵ est un groupe pyridyle, pyrimidyle ou pyrazinyle.

3. Utilisation suivant l'une des revendications 1 ou 2, dans laquelle
R¹ est un groupe phényle ou un groupe triazolyle qui sont éventuellement substitués jusqu'à deux fois identiques ou différentes par du fluor, du chlore, du brome ou de l'iode,
R² est un groupe alkoxycarbonyle linéaire ou ramifié ayant jusqu'à 4 atomes de carbone,
R³ est un groupe méthyle, éthyle ou cyclopropyle, ou bien
R² et R³ forment ensemble un reste de formule 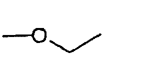
R⁴ est l'hydrogène, un groupe vinyle ou acétyle, et
R⁵ est un groupe pyridyle.

4. Dihydropyrimidines de formules suivantes : 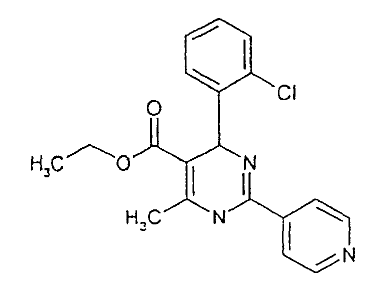 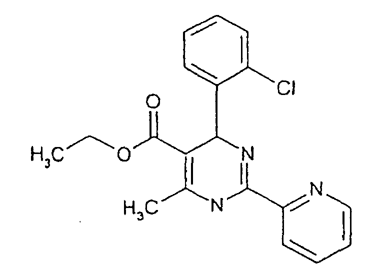 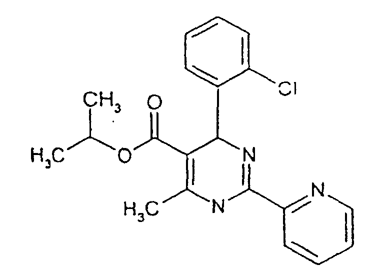 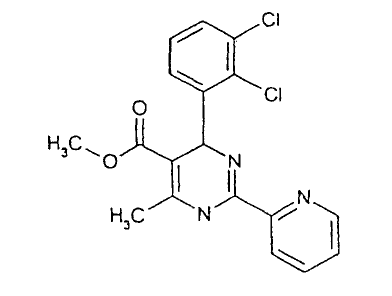 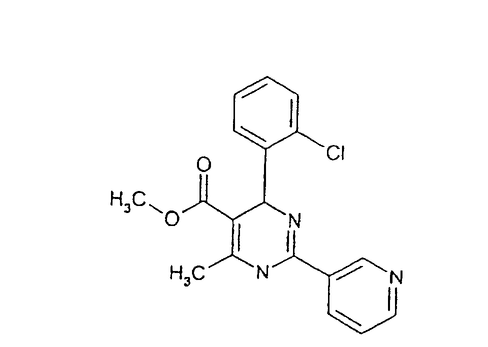 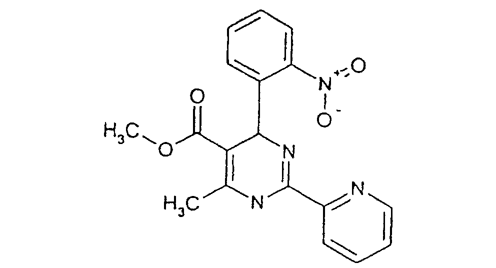 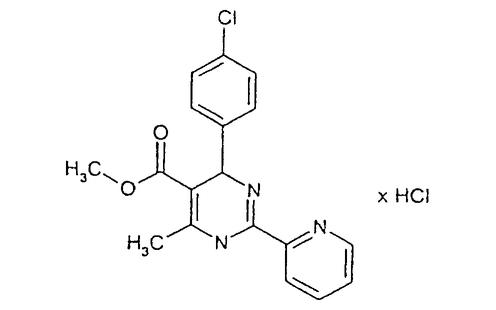 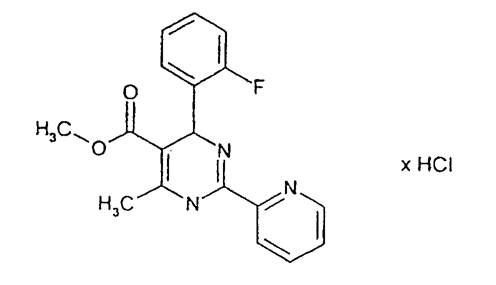 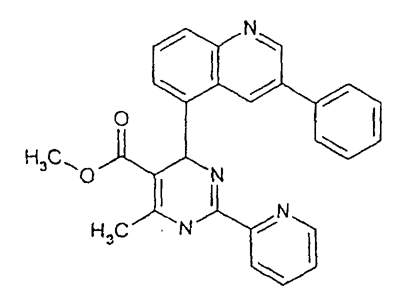 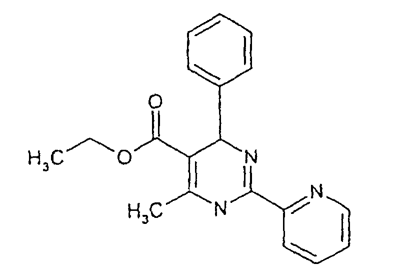 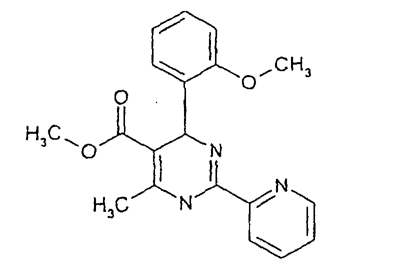 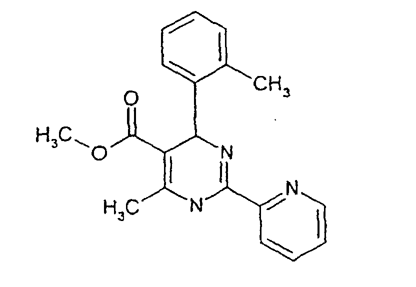 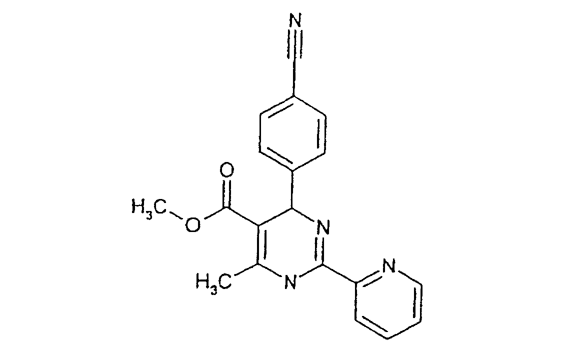 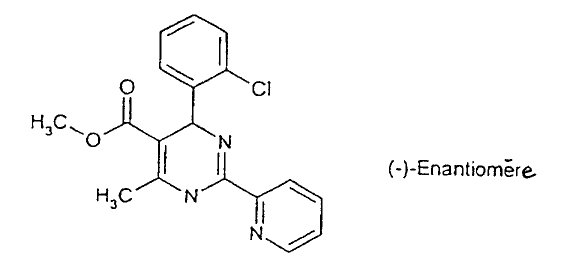 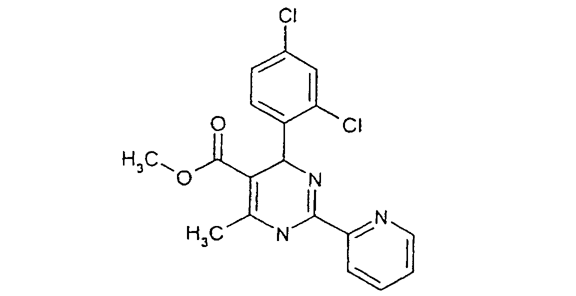 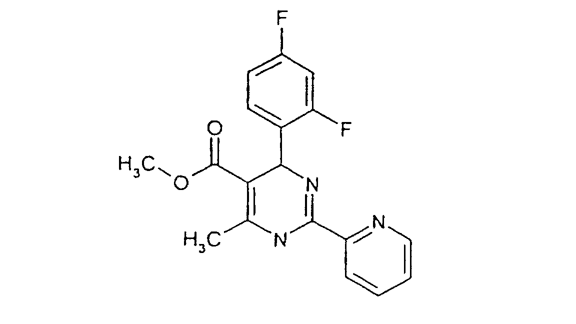 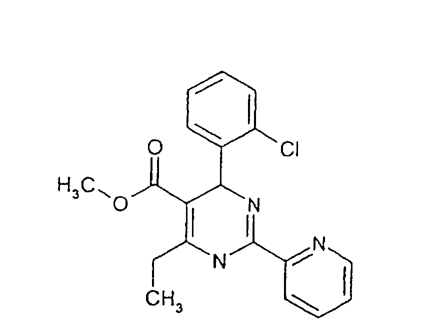 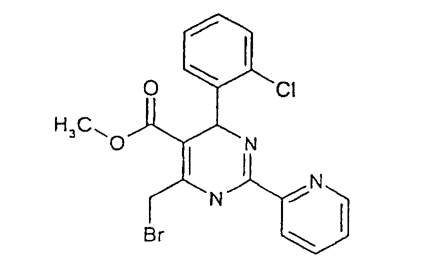 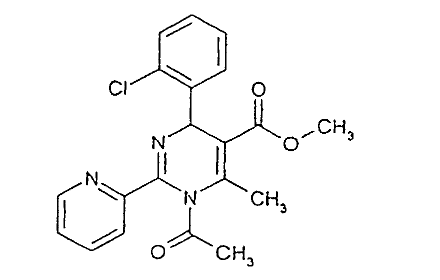 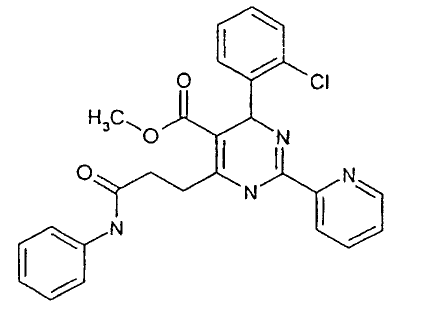 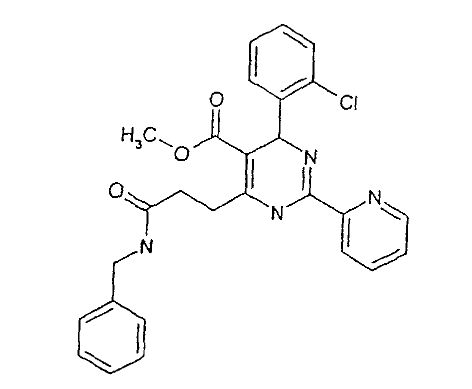 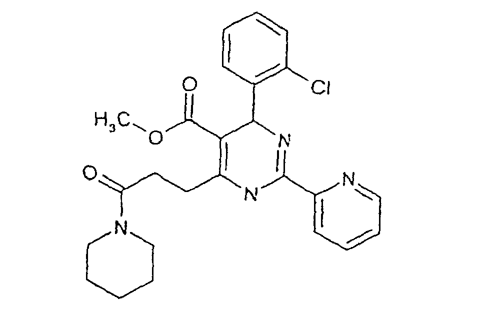 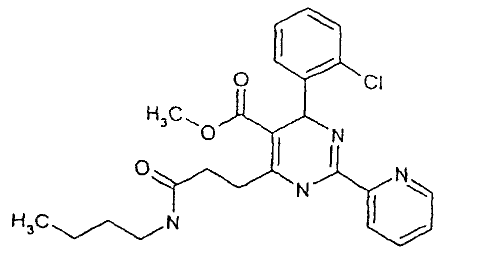 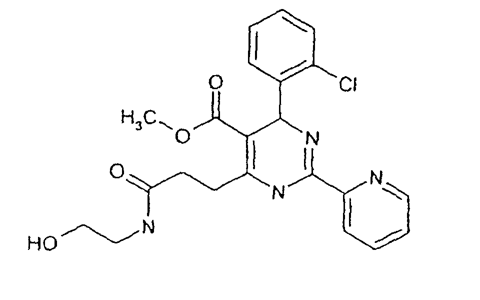 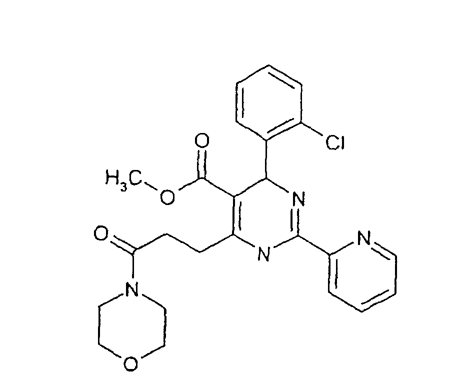 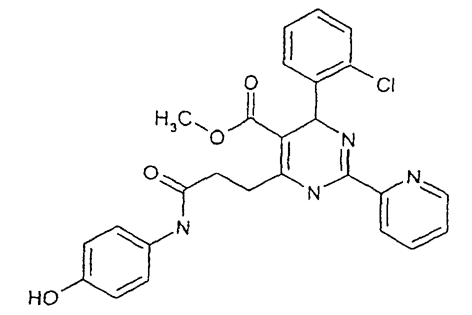 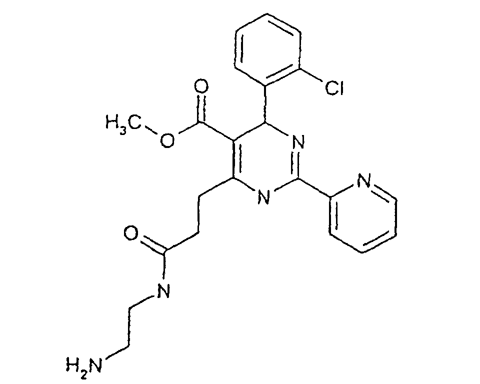 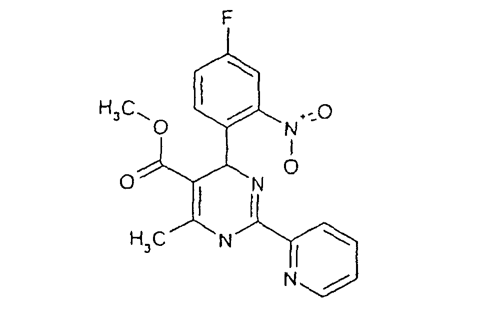 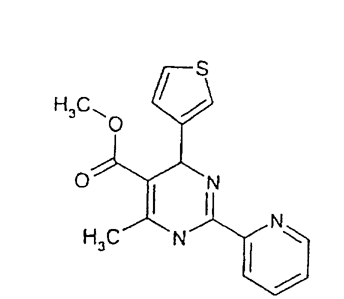 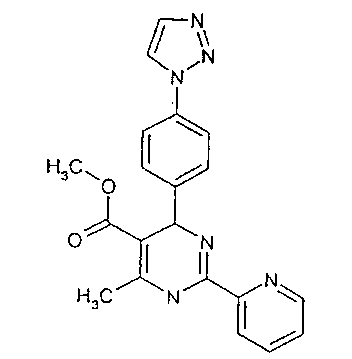 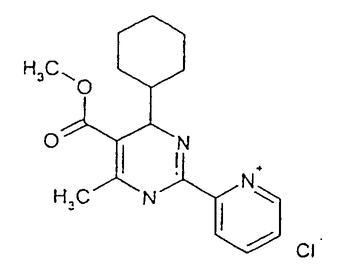 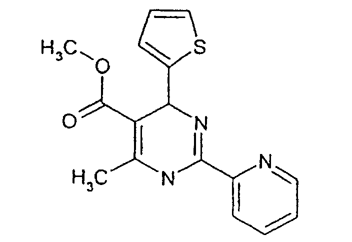 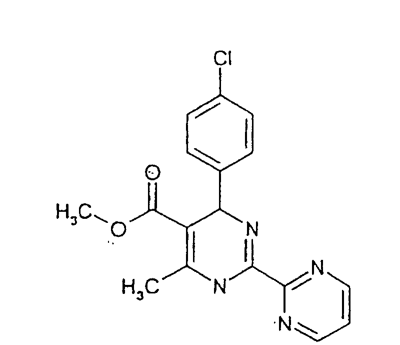 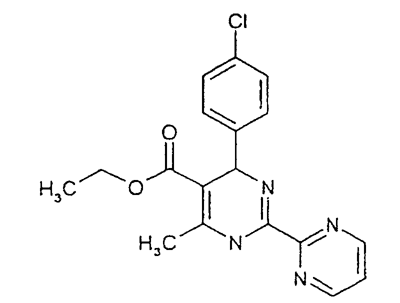 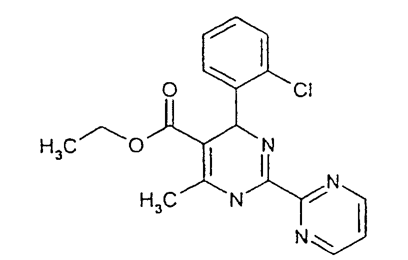 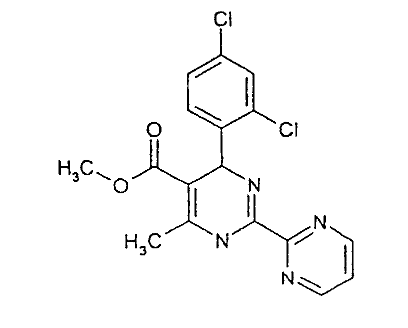 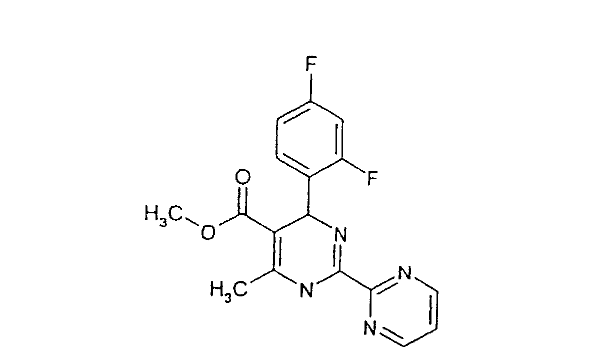 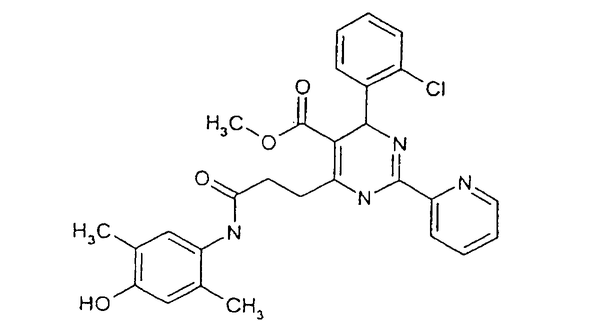 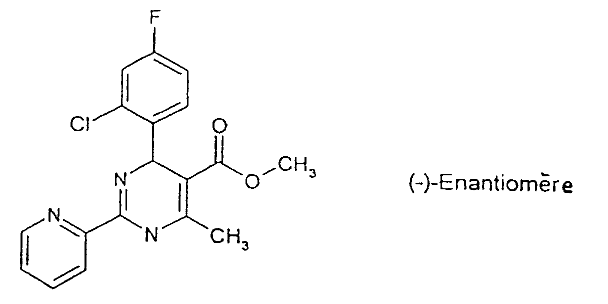 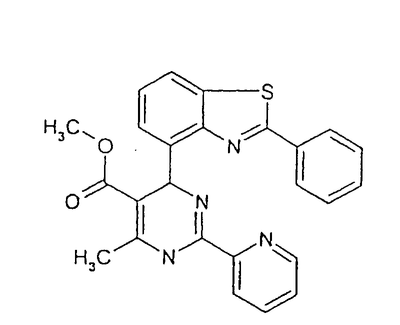 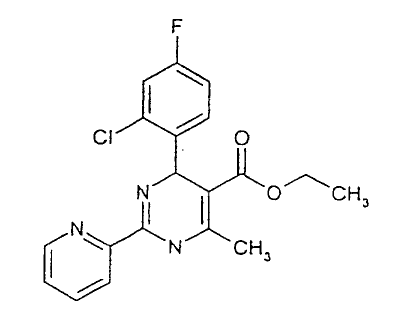 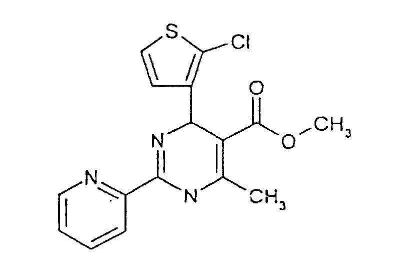 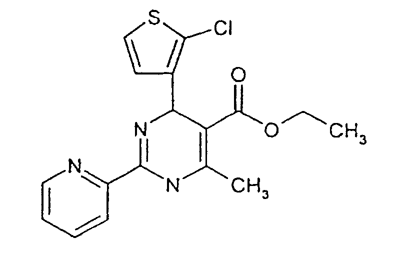 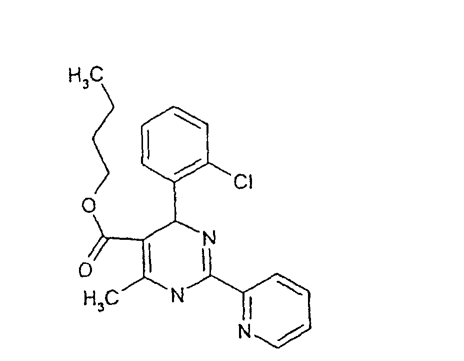 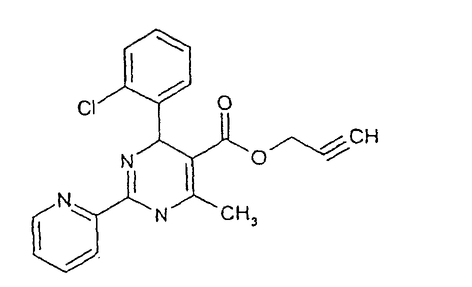 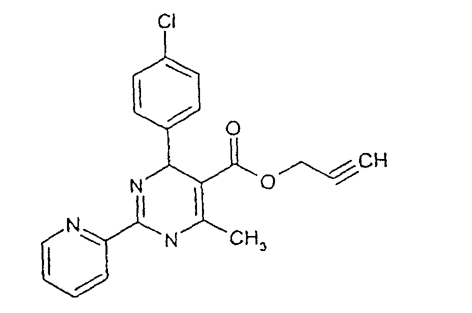 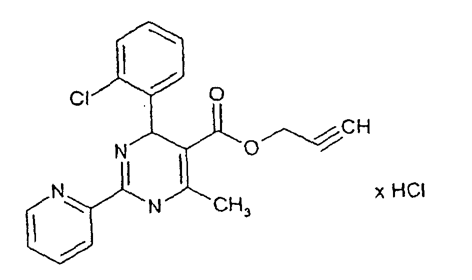 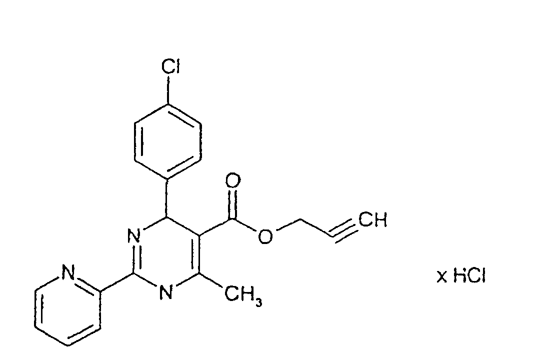 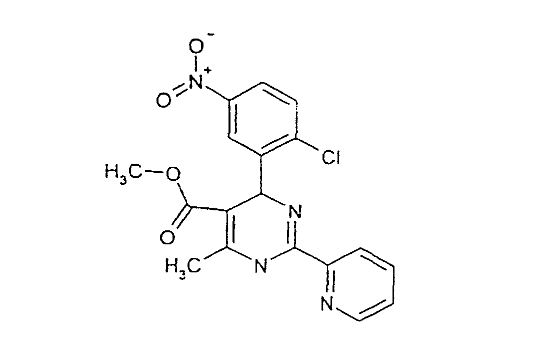 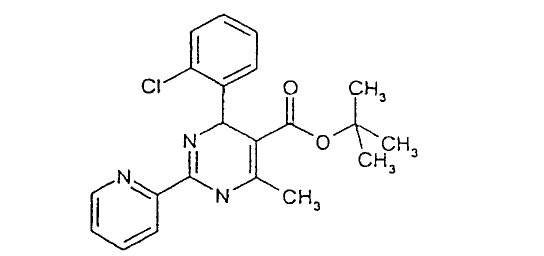 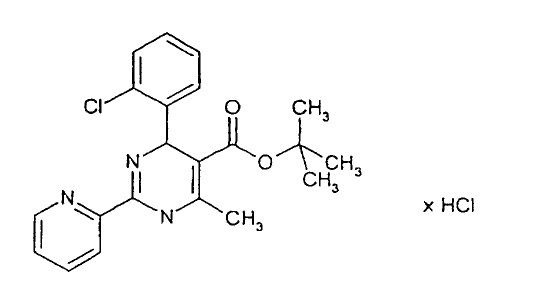 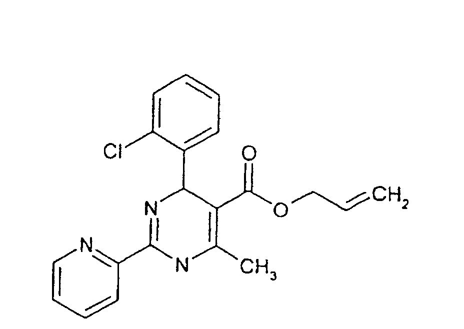 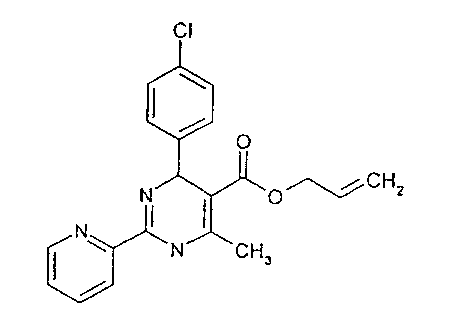 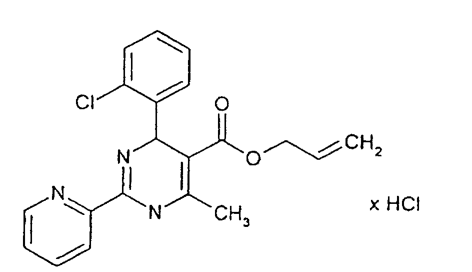 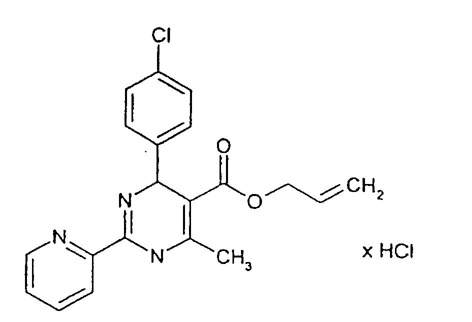 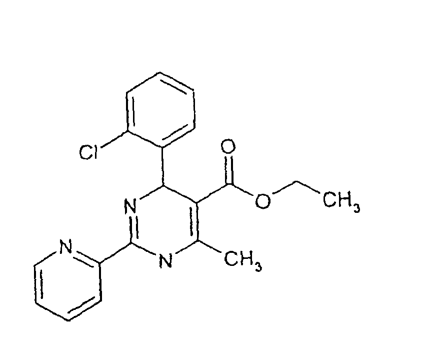 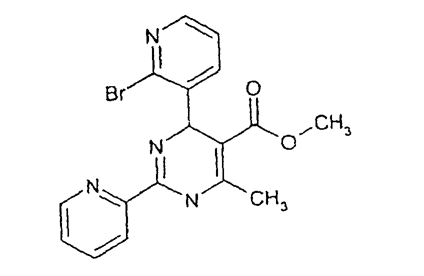 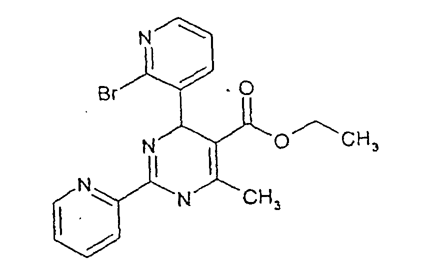 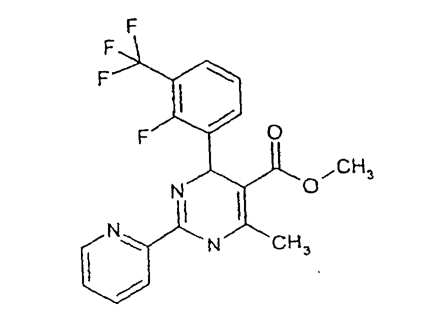 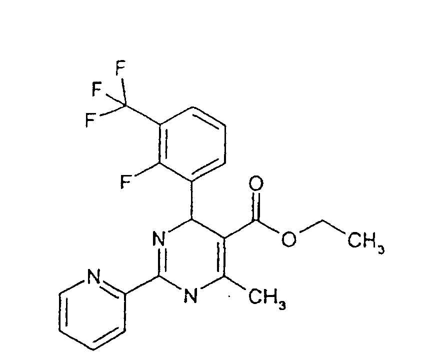 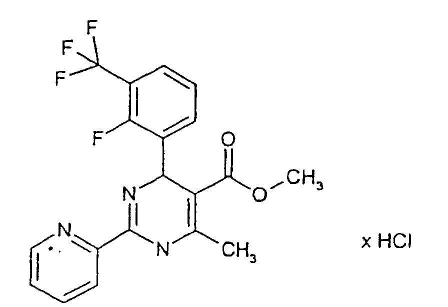 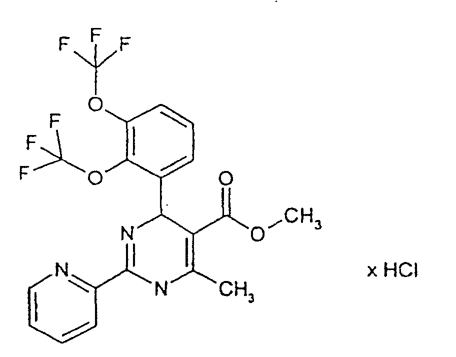 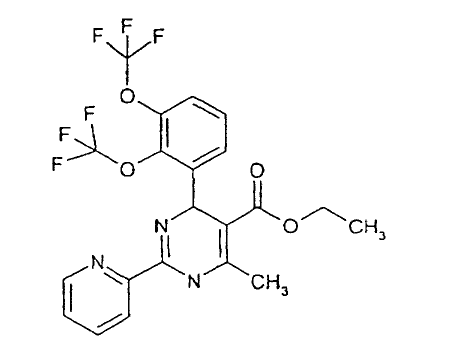 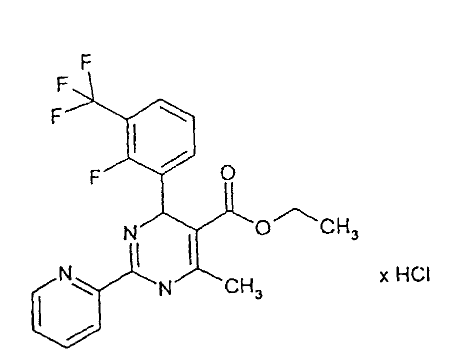 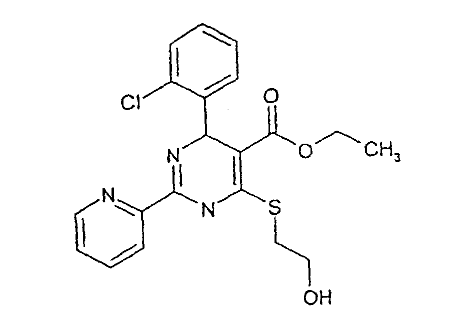 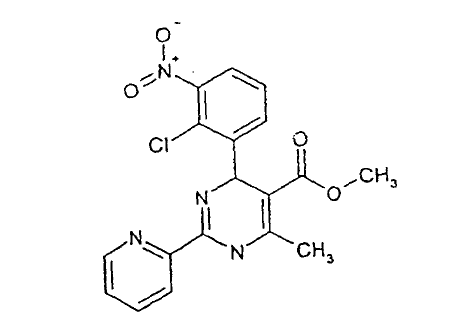 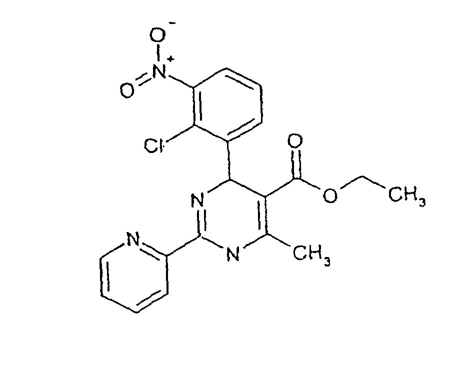 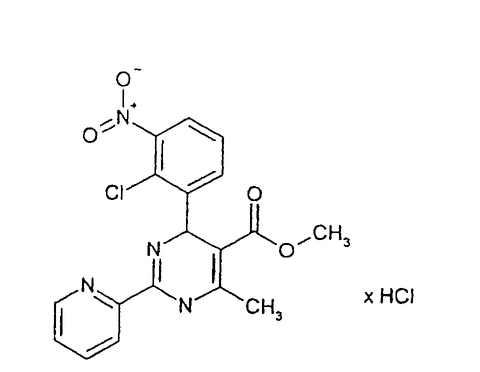 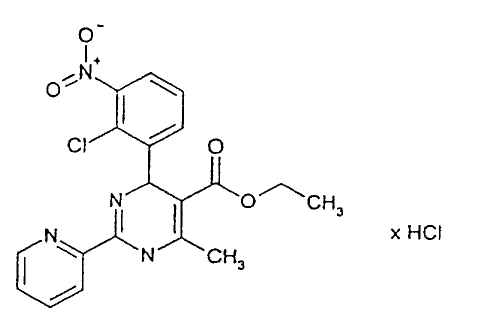 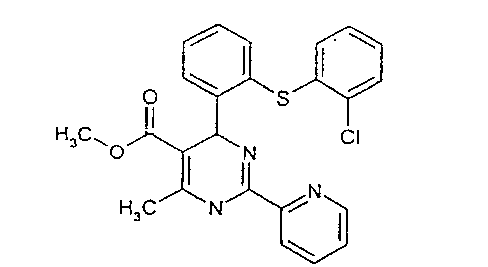 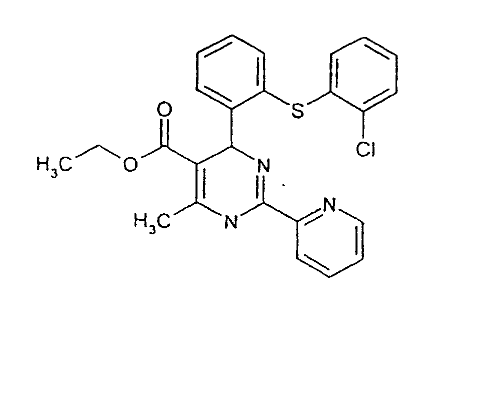 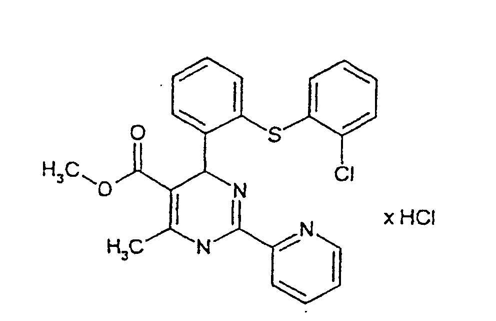 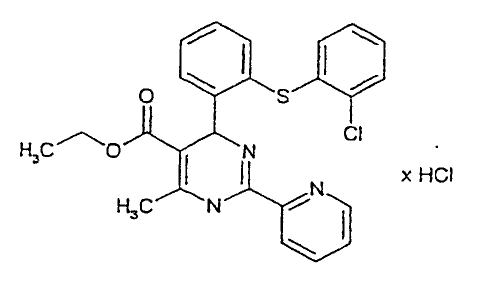 où 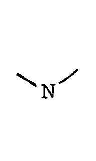 correspond à

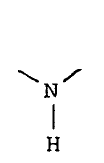

5. Procédé de production de composés de formules générales (I) et (Ia) suivant les revendications 1 à 3 et des composés suivant la revendication 4, dans lequel
[A] on fait réagir des aldéhydes de formule générale(II)
R¹-CHO (II)
dans laquelle
R¹ a la définition indiquée ci-dessus,
avec des amidines de formule (III) 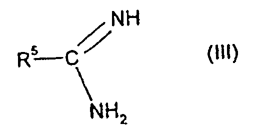 dans laquelle
R⁵ a la définition indiquée ci-dessus,
et des composés de formule générale (IV)
R³-CO-CH₂-CO-R² (IV)
dans laquelle
R² et R³ ont la définition indiquée ci-dessus,
éventuellement en présence de solvants organiques inertes, avec ou sans addition de bases ou d'acides,
ou bien
[B] on fait réagir des composés de formule générale (V) 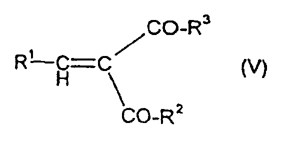 dans laquelle
R¹, R² et R³ ont la définition indiquée ci-dessus,
avec des amidines ou leurs chlorhydrates de formule générale (III) 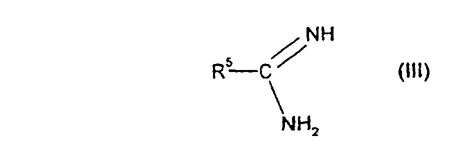 dans laquelle
R⁵ a la définition indiquée ci-dessus,
éventuellement en présence de solvants organiques inertes, à des températures comprises entre 20°C et 150°C avec ou sans addition de bases ou d'acides,
ou bien
[C) on fait réagir des aldéhydes de formule générale (II)
R¹-CHO (II)
dans laquelle
R¹ a la définition indiquée ci-dessus
avec des composés de formule générale (VI) 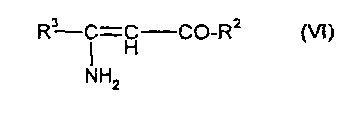 dans laquelle
R² et R³ ont la définition indiquée ci-dessus,
et des amidines de formule générale (III) comme décrit ci-dessus,
ou bien
[D) on fait réagir des aldéhydes de formule générale (II) avec des composés de formule générale (IV) et des iminoéthers de formule générale (VII) 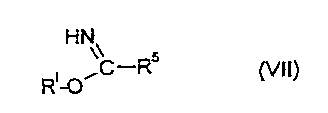 dans laquelle
R⁵ a la définition indiquée ci-dessus
et
R¹ est un groupe alkyle en C₁ à C₄,
en présence de sels d'ammonium.

6. Procédé suivant la variante [A] de la revendication 5.

7. Procédé suivant la revendication 5 ou 6, **caractérisé en ce que** les réactions sont conduites dans des solvants organiques inertes, à une température de 20 à 150°C, de préférence à la température d'ébullition du solvant, sous pression normale, éventuellement en présence d'acides ou de bases, de préférence d'acides faibles.

8. Préparations pharmaceutiques, qui contiennent, à côté de supports inertes non toxiques acceptables du point de vue pharmaceutique, un ou plusieurs composés suivant la revendication 4, ou constituées d'un ou plusieurs des composés en question.
